# EUROPEAN PATENT SPECIFICATION

(11) **EP 1 439 174 B1**
(45) Date of publication and mention of the grant of the patent: **21.03.2012**
(21) Application number: 02802049.3
(22) Date of filing: 22.10.2002
(51) Int. Cl.: C07D 401/04, C07D 401/14, C07D 403/04, C07D 403/14, C07D 405/14, C07D 409/14, A61K 31/4439, A61K 31/444, A61K 31/4545, A61K 31/496, A61K 31/506, A61P 1/00, A61P 1/04, A61P 1/16, A61P 3/10, A61P 9/00, A61P 9/10, A61P 11/00, A61P 11/02, A61P 11/06, A61P 13/12, A61P 17/00, A61P 17/02, A61P 17/04, A61P 17/06

(54) **4-IMIDAZOLIN-2-ONE COMPOUNDS AS p38 MAP KINASE INHIBITORS**
4-IMIDAZOLIN-2-ONVERBINDUNGEN ALS p38-MAP-KINASEINHIBITORS
COMPOSES DE 4-IMIDAZOLIN-2-ONE EN TANT QU'INHIBITEURS DE p38 MAP KINASE

(30) Priority: 22.10.2001 JP 2001324029; 10.09.2002 JP 2002263680
(43) Date of publication of application: 21.07.2004
(73) Proprietor: Mitsubishi Tanabe Pharma Corporation, Osaka 541-8505 (JP)
(72) Inventor: KUBO, Akira, Miyakojima-ku, Osaka-shi, Osaka 534-0025 (JP); IMASHIRO, Ritsuo, Takatsuki-shi, Osaka 569-1146 (JP); SAKURAI, Hiroaki, Toyama-shi, Toyama 930-0884 (JP); MIYOSHI, Hidetaka, Yodogawa-ku, Osaka-shi, Osaka 532-0034 (JP); OGASAWARA, Akihito, Toda-shi, Saitama 335-0015 (JP); HIRAMATSU, Hajime, Ibaraki-shi, Osaka 567-0012 (JP)
(74) Representative: Benson, John Everett
(86) International application number: PCT/JP2002/010937
(87) International publication number: WO 2003/035638

(56) References cited:
- WO-A-02/076974
- WO-A1-00/64894
- WO-A1-85/02402
- WO-A1-96/21452
- WO-A1-96/21654
- WO-A1-96/40143
- WO-A1-99/01449
- WO-A1-99/03837
- US-A- 3 538 104
- J. MED. CHEM. vol. 42, no. 14, 1999, pages 2706 - 2715, XP001093697
- PROCEEDINGS NOBCCHE vol. 21, 1994, pages 101 - 115, XP002966831

## Description

### Technical Field

The present invention relates to a novel 4-imidazolin-2-one compound which has an excellent p38MAP kinase inhibitory action and is useful for a medicament.

### Background Art

Mitogen-activated protein (MAP) kinase is a member of serine-threonine kinases which transfers a γ -phosphate group of adenosine triphosphate (ATP) to a hydroxy of specific serine or threonine which constitutes a protein, and is involved in various cellular responses against extracellular signals. p38 MAP kinase is an about 38 kDa protein and cloned as a homologue of MAP kinases.

p38MAP kinase is activated by inflammatory cytokines such as tumor necrosis factor α (TNF-α) and interleukin 1 (IL-1), and by stimulation caused by stress such as ultraviolet irradiation. p38 MAP kinase recognizes various transcription factors and protein kinases as a substrate. It has been clearly shown that, being activated by p38 MAP kinase, these transcription factors and protein kinases become involved in promoting transcription, post-transcriptional regulation (e.g. stabilizing mRNA and promoting protein translation) or stabilizing proteins, etc. of various proteins including inflammatory cytokines, which are involved in inflammatory reactions. From these findings, it is thought that p38 MAP kinase is critically involved in the various inflammatory reactions by regulating the production and the signal transduction of inflammatory cytokines, and an inhibitor of p38 MAP kinase can highly expected to serve as a therapeutic agent for various diseases including inflammatory diseases.

As the inhibitors for p38 MAP kinase, there have been disclosed imidazole derivatives in PCT Japanese Provisional Patent Publication No.2000-503304, 1,3-thiazole derivatives in Japanese Provisional Patent Publication No. 2001-14690, 1,3-thiazole derivatives and 1,3-oxazole derivatives, in Japanese Provisional Patent Publication No. 2001-114779, imidazole derivatives, pyrrole derivatives, furan derivatives, 3-pyrazolin-5-one derivatives, pyrazole derivatives and thiophene derivative, etc. in Expert Opinion on Therapeutic Patents (2000) 10(1) : 25-37, respectively. However, there has been no description on 4-imidazolin-2-one derivatives in any of these.

An object of the present invention is to provide a novel compound having an excellent p38 MAP kinase inhibitory action and is useful as a pharmaceutical.

### Disclosure of Invention

The present inventions are as disclosed as follows.
[1] A compound having the formula [Ia]: 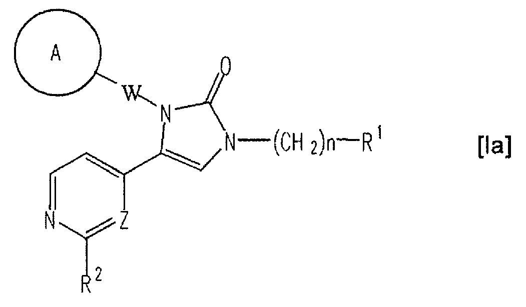 wherein
   - ring A is benzene ring or thiophene ring, and the benzene ring and thiophene ring may be substituted by 1 to 3 substituent(s), which is(are) the same or different, and selected from the group consisting of
      - a halogen atom;
      - nitro;
      - a C₁-C₆ alkyl which may be substituted by 1 to 3 group(s) selected from a halogen atom(s), hydroxy(s) and amino(s);
      - a C₁-C₆ alkoxy which may be substituted by 1 to 3 group(s) selected from hydroxy(s) and amino(s);
      - an amino which may be substituted by 1 or 2 group(s) selected from (a) a C₁-C₆ alkyl which may be substituted by 1 to 3 group(s) selected from the group consisting of C₁-C₆ alkoxy(s), amino(s) and carboxy(s), and (b) a C₂-C₇ alkanoyl;
      - a carbamoyl which may be substituted by 1 or 2 C₁-C₆ alkyl(s); and
      - cyano,
   - W is a single bond, or a C₁ - C₄ alkylene which may be substituted by 1 or 2 C₁-C₆ alkyl(s),
   - n is 0, 1, 2, 3 or 4,
   - R¹ is - hydrogen atom;
      - a C₁-C₆ alkyl which may be substituted by 1 to 3 group(s) selected from (a) a C₂-C₇ alkynyl, (b) cyano, (c) a C₁-C₆ alkoxy, (d) hydroxy, (e) amino which may be substituted with 1 or 2 substituents selected from the group consisting of a C₁-C₆ alkyl, a C₂-C₇ alkanoyl and a C₁-C₆ alkyl-sulfonyl, (f) carboxy, (g) a C₁-C₆ alkoxy-carbonyl, (h) carbamoyl which may be substituted with 1 or 2 C₁-C₆ alkyl(s), (i) phenyl and (j) naphthyl;
      - a C₃-C₈ cycloalkyl which may be substituted by 1 to 3 group(s) selected from (a) hydroxy, (b) a C₁-C₆ alkoxy which may be substituted by 1 to 3 C₁-C₆ alkoxy(s), (c) amino which may be substituted by 1 or 2 group(s) selected from the group consisting of the following (1) to (5): (1) a C₁-C₆ alkyl, (2) a C₂-C₇ alkanoyl, (3) a C₁-C₆ alkoxy-carbonyl, (4) carbamoyl which may be substituted by 1 or 2 C₁-C₆ alkyl(s), and(5) a C₁-C₆ alkyl-sulfonyl, (d) carboxy, (e) a C₁-C₆ alkyl which may be substituted by a group selected from the group consisting of hydroxy, a C₁-C₆ alkoxy and amino, (f) a carbamoyl which may be substituted by C₁-C₆ alkyl(s);
      - a phenyl which may be substituted by 1 to 3 group(s) selected from (a) a halogen atom, (b) nitro, (c) a C₁-C₆ alkyl which may be substituted by 1 to 3 group(s) selected from the group consisting of a halogen atom, hydroxy, amino, carboxy, and phenylsulfonyl, (d) a C₂-C₇ alkenyl, (e) cyano, (f) hydroxy, (g) a C₁-C₆ alkoxy which may be substituted by 1 to 3 group(s) selected from the group consisting of a halogen atom, carboxy, a C₁-C₆ alkoxy-carbonyl, carbamoyl, phenyl and morpholinylcarbonyl, (h) amino which may be substituted with 1 or 2 group(s) selected from the group consisting of the following (1) to (4) : (1) a C₁-C₆ alkyl, (2) a C₂-C₇ alkanoyl, (3) carbamoyl which may be substituted by 1 or 2 group(s) selected from the group consisting of a C₁-C₆ alkyl and a C₃-C₈ cycloalkyl, and (4) a C₁-C₆ alkyl-sulfonyl, (i) a C₂-C₇ alkanoyl, (j) carboxy, (k) a C₁-C₆ alkoxy-carbonyl, (l) carbamoyl which may be substituted by 1 or 2 group(s) selected from the group consisting of the following (1) and (2): (1) a C₁-C₆ alkyl which may be substituted by 1 to 3 hydroxy(s) and (2) a C₃-C₈ cycloalkyl, (m) a C₁-C₆ alkyl-thio, (n) a C₁-C₆ alkyl-sulfinyl, (o) a C₁-C₆ alkyl-sulfonyl, (p) phenyl, (q) tetrazolyl, and (r) a carbonyl substituted by heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of a C₁-C₆ alkyl and a C₁-C₆ alkoxy-carbonyl; or
      - a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from (a) a halogen atom, (b) nitro, (c) a C₁-C₆ alkyl which may be substituted by a group selected from the group consisting of hydroxy, a C₁-C₆ alkoxy, a carbamoyl which may be substituted by C₁-C₆ alkyl(s) and carboxy(s), (d) cyano, (e) hydroxy, (f) amino, (g) a C₂-C₇ alkanoyl, (h) carboxy, (i) a C₁-C₆ alkoxy-carbonyl, (j) carbamoyl which may be substituted by 1 or 2 C₁-C₆ alkyl(s), (k) a C₁-C₆ alkyl-sulfonyl, and (l) phenyl,
   - Z is CH or N,
   - R² is hydrogen atom, -NR³R⁴, -OR⁵, -COR⁶ or -CHR⁷R⁸,
      where R³ to R⁸, each independently is
      - hydrogen atom,
      - a C₁-C₆ alkyl which may be substituted by 1 to 3 group(s) selected from (a) hydroxy, (b) a C₁-C₆ alkoxy, (c) amino which may be substituted by 1 or 2 group(s) selected from the group consisting of a C₁-C₆ alkyl, a C₂-C₇alkanoyl and a C₁-C₆ alkyl-sulfonyl, (d) a C₁-C₆ alkoxy-carbonyl, (e) a C₃-C₈ cycloalkyl which may be substituted by 1 to 3 group(s) selected from the group consisting of (1) hydroxy, (2) an amino which may be substituted by C₁-C₆ alkyl(s), (3) a C₂-C₇ alkanoyl-amino, (4) a C₁-C₆ alkyl-sulfonylamino, (5) a C₁-C₆ alkyl which may be substituted by a group selected from hydroxy, a C₁-C₆ alkoxy, amino and a carbamoyl which may be substituted by C₁-C₆ alkyl(s), (6) carboxy and (7) a carbamoyl which may be substituted by C₁-C₆ alkyl(s),(f) phenyl which may be substituted by 1 to 3 group(s) selected from the group consisting of the following (1) to (6): (1) a halogen atom, (2) a C₁-C₆ alkoxy, (3) amino which may be substituted by 1 or 2 group(s) selected from the group consisting of a C₁-C₆ alkyl and a C₁-C₆ alkoxy-carbonyl, (4) a C₁-C₆ alkoxy-carbonyl, (5) carbamoyl, and (6) morpholinylcarbonyl, and (g) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of the following (1) to (5): (1) a C₁-C₆ alkyl which may be substituted by 1 to 3 hydroxy(s), (2) hydroxy, (3) amino, (4) a C₁-C₆ alkoxy-carbonyl, and (5) carbamoyl;
      - a C₂-C₇ alkenyl;
      - a C₂-C₇ alkynyl;
      - hydroxy;
      - a C₁-C₆ alkoxy;
      - an amino which may be substituted by 1 or 2 group(s) selected from (a) a C₁-C₆ alkyl which may be substituted by 1 to 3 group(s) selected from the group consisting of (1) hydroxy, (2) a C₁-C₆ alkoxy and (3) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom, (b) a C₃-C₈ cycloalkyl which may be substituted by 1 to 3 hydroxy(s), and (c) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
      - a C₂-C₇ alkanoyl which may be substituted by 1 to 3 group(s) selected from (a) hydroxy, (b) a C₁-C₆ alkoxy, (c) amino which may be substituted by 1 or 2 group(s) selected from the group consisting of a C₁-C₆ alkyl and a C₂-C₇ alkanoyl, and (d) a C₁-C₆ alkoxy-carbonyl;
      - a carbamoyl which may be substituted by 1 or 2 group(s) selected from (a) a C₁-C₆ alkyl, (b) a C₃-C₈ cycloalkyl, and (c) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
      - a C₁-C₆ alkoxy-oxalyl;
      - a C₁-C₆ alkyl-sulfonyl;
      - a C₃-C₈ cycloalkyl which may be substituted by 1 to 3 group(s) selected from (a) a halogen atom, (b) a C₁-C₆ alkyl which may be substituted by 1 to 3 group(s) selected from the group consisting of (1) hydroxy, (2) a C₁-C₆ alkoxy, (3) amino and (4) a carbamoyl which may be substituted by a C₁-C₆ alkyl, (c) hydroxy, (d) a C₁-C₆ alkoxy, (e) amino which may be substituted by 1 or 2 group(s) selected from the group consisting of (1) a C₁-C₆ alkyl, (2) a C₂-C₇ alkanoyl, (3) a C₁-C₆ alkoxy-carbonyl and (4) a C₁-C₆ alkyl-sulfonyl, (f) carboxy, (g) a C₂-C₇ alkanoyl-oxy, (h) a C₁-C₆ alkoxy-carbonyl, and (i) carbamoyl which may be substituted by 1 or 2 group(s) selected from the group consisting of (1) a C₁-C₆ alkyl, (2) a C₃-C₈ cycloalkyl and (3) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
      - a phenyl which may be substituted by 1 to 3 group(s) selected from (a) a C₁-C₆ alkyl, (b) hydroxy, (c) a C₁-C₆ alkoxy, (d) a halogen atom, and (e) amino which may be substituted by 1 or 2 C₁-C₆ alkyl(s);
      - a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from (a) a C₁-C₆ alkyl which may be substituted by 1 to 3 group(s) selected from the group consisting of (1) phenyl, (2) hydroxy, (3) a C₁-C₆ alkoxy, (4) amino and (5) a carbamoyl which may be substituted by a C₁-C₆ alkyl, (b) carboxy, (c) a C₁-C₆ alkoxy-carbonyl, (d) a C₂-C₇ alkanoyl, (e) a C₁-C₆ alkyl-sulfonyl, and (f) oxo;
      - a carbonyl substituted by a C₃-C₈ cycloalkyl which may be substituted by 1 to 3 group(s) selected from (a) hydroxy, (b) a C₁-C₆ alkoxy, (c) amino which may be substituted by 1 or 2 group(s) selected from the group consisting of (1) a C₁-C₆ alkyl and (2) a C₂-C₇ alkanoyl, and (d) a C₁-C₆ alkoxy-carbonyl;
      - a carbonyl substituted by phenyl, which may be substituted by 1 to 3 group(s) selected from (a) a halogen atom, (b) hydroxy, (c) a C₁-C₆ alkoxy, and (d) amino which may be substituted by 1 or 2 group(s) selected from the group consisting of a C₁-C₆ alkyl and a C₂-C₇ alkanoyl; or
      - a carbonyl substituted by a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 groups selected from (a) a halogen atom, (b) a C₁-C₆ alkyl, (c) hydroxy, (d) amino which may be substituted by 1 or 2 C₁-C₆ alkyl(s), (e) a C₂-C₇ alkanoyl, and (f) oxo,
   or a pharmaceutically acceptable salt thereof.
[2] The compound according to [1], wherein the ring A is a benzene ring which may be substituted by 1 to 3 substituent(s), which is(are) the same or different, and selected from the group consisting of
   - a halogen atom;
   - nitro;
   - a C₁-C₆ alkyl which may be substituted by 1 to 3 group(s) selected from a halogen atom(s), hydroxy(s) and amino(s);
   - a C₁-C₆ alkoxy which may be substituted by 1 to 3 group(s) selected from hydroxy(s) and amino(s);
   - an amino which may be substituted by 1 or 2 group(s) selected from (a) a C₁-C₆ alkyl which may be substituted by 1 to 3 group(s) selected from the group consisting of C₁-C₆ alkoxy(s), amino(s) and carboxy(s), and (b) a C₂-C₇ alkanoyl, and
   - cyano,
   and W is a single bond,
   or a pharmaceutically acceptable salt thereof.
[3] The compound according to [1] or [2], wherein n is 0 or 1, or a pharmaceutically acceptable salt thereof.
[4] The compound according to any one of [1] to [3], wherein
   - n is 0 and R¹ is a C₁-C₆ alkyl which may be substituted by 1 to 3 group(s) selected from (a) a C₂-C₇ alkynyl, (b) cyano, (c) a C₁-C₆ alkoxy, (d) hydroxy, (e) amino which may be substituted with 1 or 2 substituents selected from the group consisting of a C₁-C₆ alkyl, a C₂-C₇ alkanoyl and a C₁-C₆ alkyl-sulfonyl, (f) carboxy, (g) a C₁-C₆ alkoxy-carbonyl, (h) carbamoyl which may be substituted with 1 or 2 C₁-C₆alkyl(s), (i) phenyl and (j) naphthyl, or
   - n is 1 and R¹ is
      (A) a C₃-C₈ cycloalkyl which may be substituted by 1 to 3 group(s) selected from (a) hydroxy, (b) a C₁-C₆ alkoxy which may be substituted by 1 to 3 C₁-C₆ alkoxy(s), (c) amino which may be substituted by 1 or 2 group(s) selected from the group consisting of the following (1) to (5): (1) a C₁-C₆ alkyl, (2) a C₂-C₇ alkanoyl, (3) a C₁-C₆ alkoxy-carbonyl, (4) carbamoyl which may be substituted by 1 or 2 C₁-C₆ alkyl(s), and (5) a C₁-C₆ alkyl-sulfonyl, (d) carboxy, (e) a C₁-C₆ alkyl which may be substituted by a group selected from the group consisting of hydroxy, a C₁-C₆ alkoxy and amino, (f) a carbamoyl which may be substituted by C₁-C₆ alkyl(s), or
      (B) a phenyl which may be substituted by 1 to 3 group(s) selected from (a) a halogen atom, (b) nitro, (c) a C₁-C₆ alkyl which may be substituted by 1 to 3 group(s) selected from the group consisting of a halogen atom, hydroxy, amino, carboxy, and phenylsulfonyl, (d) a C₂-C₇ alkenyl, (e) cyano, (f) hydroxy, (g) a C₁-C₆ alkoxy which may be substituted by 1 to 3 group(s) selected from the group consisting of a halogen atom, carboxy, a C₁-C₆ alkoxy-carbonyl, carbamoyl, phenyl and morpholinylcarbonyl, (h) amino which may be substituted with 1 or 2 group(s) selected from the group consisting of the following (1) to (4): (1) a C₁-C₆ alkyl, (2) a C₂-C₇ alkanoyl, (3) carbamoyl which may be substituted by 1 or 2 group(s) selected from the group consisting of a C₁-C₆ alkyl and a C₃-C₈ cycloalkyl, and (4) a C₁-C₆ alkyl-sulfonyl, (i) a C₂-C₇ alkanoyl, (j) carboxy, (k) a C₁-C₆ alkoxy-carbonyl, (l) carbamoyl which may be substituted by 1 or 2 group(s) selected from the group consisting of the following (1) and (2): (1) a C₁-C₆ alkyl which may be substituted by 1 to 3 hydroxy(s) and (2) a C₃-C₈ cycloalkyl, (m) a C₁-C₆ alkyl-thio, (n) a C₁-C₆ alkyl-sulfinyl, (o) a C₁-C₆ alkyl-sulfonyl, (p) phenyl, (q) tetrazolyl, and (r) a carbonyl substituted by heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of a C₁-C₆ alkyl and a C₁-C₆ alkoxy-carbonyl,
   or a pharmaceutically acceptable salt thereof.
[5] The compound according to any one of [1] to [4], wherein R² is -NR³R⁴ or -OR⁵, or a pharmaceutically acceptable salt thereof.
[6] The compound according to any one of [1] to [4], wherein R² is -NHR⁴, and R⁴ is
   - a C₁-C₆ alkyl which may be substituted by 1 to 3 group(s) selected from
      (a) hydroxy,
      (b) a C₁-C₆ alkoxy,
      (c) amino which may be substituted by 1 or 2 group(s) selected from the group consisting of a C₁-C₆ alkyl, a C₂-C₇ alkanoyl and a C₁-C₆ alkyl-sulfonyl,
      (d) a C₁-C₆ alkoxy-carbonyl,
      (e) a C₃-C₈ cycloalkyl which may be substituted by 1 to 3 group(s) selected from the group consisting of (1) hydroxy, (2) an amino which may be substituted by C₁-C₆ alkyl(s), (3) a C₂-C₇ alkanoyl-amino, (4) a C₁-C₆ alkyl-sulfonylamino, (5) a C₁-C₆ alkyl which may be substituted by a group selected from hydroxy, a C₁-C₆ alkoxy, amino and a carbamoyl which may be substituted by C₁-C₆ alkyl(s), (6) carboxy and (7) a carbamoyl which may be substituted by C₁-C₆ alkyl(s),
      (f) phenyl which may be substituted by 1 to 3 group(s) selected from the group consisting of the following (1) to (6): (1) a halogen atom, (2) a C₁-C₆ alkoxy, (3) amino which may be substituted by 1 or 2 group(s) selected from the group consisting of a C₁-C₆ alkyl and a C₁-C₆ alkoxy-carbonyl, (4) a C₁-C₆ alkoxy-carbonyl, (5) carbamoyl, and (6) morpholinylcarbonyl, and
      (g) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of the following (1) to (5): (1) a C₁-C₆ alkyl which may be substituted by 1 to 3 hydroxy(s), (2) hydroxy, (3) amino, (4) a C₁-C₆ alkoxy-carbonyl, and(5) carbamoyl;
   - a C₂-C₇ alkenyl;
   - a C₂-C₇ alkanoyl which may be substituted by 1 to 3 group(s) selected from (a) hydroxy, (b) a C₁-C₆ alkoxy, (c) amino which may be substituted by 1 or 2 group(s) selected from the group consisting of a C₁-C₆ alkyl and a C₂-C₇ alkanoyl, and (d) a C₃-C₆ alkoxy-carbonyl;
   - a carbamoyl which may be substituted by 1 or 2 group(s) selected from (a) a C₁-C₆ alkyl, (b) a C₃-C₈ cycloalkyl, and (c) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
   - a C₃-C₈ cycloalkyl which may be substituted by 1 to 3 group(s) selected from (a) a halogen atom, (b) a C₁-C₆ alkyl which may be substituted by 1 to 3 group(s) selected from the group consisting of (1) hydroxy, (2) a C₁-C₆ alkoxy, (3) amino and (4) a carbamoyl which may be substituted by a C₁-C₆ alkyl, (c) hydroxy, (d) a C₁-C₆ alkoxy, (e) amino which may be substituted by 1 or 2 group(s) selected from the group consisting of (1) a C₁-C₆ alkyl, (2) a C₂-C₇ alkanoyl, (3) a C₁-C₆alkoxy-carbonyl and (4) a C₁-C₆ alkyl-sulfonyl, (f) carboxy, (g) a C₂-C₇ alkanoyl-oxy, (h) a C₁-C₆alkoxy-carbonyl, and (i) carbamoyl which may be substituted by 1 or 2 group(s) selected from the group consisting of (1) a C₁-C₆ alkyl, (2) a C₃-C₈ cycloalkyl and (3) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
   - a phenyl which may be substituted by 1 to 3 group(s) selected from (a) a C₁-C₆ alkyl, (b) hydroxy, (c) a C₁-C₆ alkoxy, (d) a halogen atom, and (e) amino which may be substituted by 1 or 2 C₁-C₆ alkyl(s);
   - a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from (a) a C₁-C₆ alkyl which may be substituted by 1 to 3 group(s) selected from the group consisting of (1) phenyl, (2) hydroxy, (3) a C₁-C₆ alkoxy, (4) amino and (5) a carbamoyl which may be substituted by a C₁-C₆ alkyl, (b) carboxy, (c) a C₁-C₆ alkoxy-carbonyl, (d) a C₂-C₇ alkanoyl, (e) a C₁-C₆ alkyl-sulfonyl, and (f) oxo;
   - a carbonyl substituted by a C₃-C₈ cycloalkyl which may be substituted by 1 to 3 group(s) selected from (a) hydroxy, (b) a C₁-C₆ alkoxy, (c) amino which may be substituted by 1 or 2 group(s) selected from the group consisting of (1) a C₁-C₆ alkyl and (2) a C₂-C₇ alkanoyl, and (d) a C₁-C₆ alkoxy-carbonyl; or
   - a carbonyl substituted by a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 selected from (a) a halogen atom, (b) a C₁-C₆ alkyl, (c) hydroxy, (d) amino which may be substituted by 1 or 2 C₁-C₆ alkyl(s), (e) a C₂-C₇ alkanoyl, and (f) oxo,
   or a pharmaceutically acceptable salt thereof.
[7] The compound according to [1], wherein
   - the ring A is a benzene ring which may be substituted by 1 or 2 substituent(s), which is(are) the same or different, and selected from the group consisting of a halogen atom, a C₁-C₆ alkyl, a C₁-C₆ alkoxy, an amino optionally substituted by C₁-C₆ alkyl(s) and cyano,
   - W is a single bond,
   - n is 0 or 1,
   - R¹ is
      (1) hydrogen atom,
      (2) a C₁-C₆ alkyl optionally substituted by group(s) selected from the group consisting of phenyl, a C₁-C₆ alkoxy, a C₁-C₆ alkyl-amino, a di C₁-C₆ alkyl-amino, a C₂-C₇ alkanoyl-amino, a C₁-C₆ alkyl-sulfonylamino, a carbamoyl optionally substituted by C₁-C₆ alkyl(s), hydroxy, carboxy and cyano,
      (3) a C₃-C₈ cycloalkyl optionally substituted by group(s) selected from the group consisting of the following (i) to (v): (i) hydroxy, (ii) a C₁-C₆ alkoxy optionally substituted by C₁-C₆ alkoxy(s), (iii) an amino optionally substituted by group(s) selected from the group consisting of a C₁-C₆ alkyl, a C₂-C₇ alkanoyl and a C₁-C₆ alkyl-sulfonyl, (iv) a carbamoyl optionally substituted by C₁-C₆ alkyl(s), and (v) a C₁-C₆ alkyl optionally substituted by group(s) selected from the group consisting of hydroxy, a C₁-C₆ alkoxy and amino,
      (4) a phenyl optionally substituted by group(s) selected from the group consisting of the following (i) to (vi): (i) a halogen atom, (ii) a C₁-C₆ alkyl optionally substituted by group(s) selected from the group consisting of a halogen atom, hydroxy and phenylsulfonyl, (iii) cyano, (iv) a C₁-C₆ alkoxy, (v) an amino optionally substituted by group(s) selected from the group consisting of a C₁-C₆ alkyl and a C₁-C₆ alkyl-sulfonyl, (vi) a carbonyl substituted by a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, or
      (5) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by group(s) selected from the group consisting of the following (i) to (iv): (i) a C₁-C₆ alkoxy-carbonyl, (ii) a C₁-C₆ alkyl optionally substituted by group(s) selected from the group consisting of hydroxy, a C₁-C₆ alkoxy and a carbamoyl optionally substituted by C₁-C₆ alkyl(s), (iii) a C₂-C₇ alkanoyl and (iv) a C₁-C₆ alkyl-sulfonyl,
   - Z is CH or N,
   - R² is hydrogen atom, -NR³R⁴, -OR⁵, -COR⁶ or -CHR⁷R⁸,
      where R³ to R⁸ each independently is:
      (1) hydrogen atom,
      (2) a C₁-C₆ alkyl optionally substituted by group(s) selected from the group consisting of the following (i) to (vii):
         (i) hydroxy,
         (ii) a C₁-C₆ alkoxy,
         (iii) an amino optionally substituted by group(s) selected from the group consisting of a C₁-C₆ alkyl, a C₂-C₇ alkanoyl and a C₁-C₆ alkyl-sulfonyl,
         (iv) a C₁-C₆ alkoxy-carbonyl,
         (v) a C₃-C₈ cycloalkyl optionally substituted by group(s) selected from the group consisting of the following a) to g):
            a) hydroxy,
            b) an amino optionally substituted by C₁-C₆ alkyl(s),
            c) a C₂-C₇ alkanoyl-amino,
            d) a C₁-C₆ alkyl-sulfonylamino,
            e) a C₁-C₆ alkyl optionally substituted by group(s) selected from the group consisting of hydroxy, a C₁-C₆ alkoxy, amino, a carbamoyl optionally substituted by C₁-C₆ alkyl(s),
            f) carboxy and
            g) a carbamoyl optionally substituted by C₁-C₆ alkyl(s),
         (vi) a phenyl optionally substituted by group(s) selected from the group consisting of a halogen atom, a C₁-C₆ alkoxy and morpholinylcarbonyl, and
         (vii) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by C₁-C₆ alkyl(s),
      (3) a C₂-C₇ alkenyl,
      (4) a C₁-C₆ alkoxy,
      (5) a C₂-C₇ alkanoyl optionally substituted by group(s) selected from the group consisting of the following (i) to (iv):
         (i) hydroxy,
         (ii) a C₁-C₆ alkoxy,
         (iii) an amino optionally substituted by group(s) selected from the group consisting of a C₁-C₆ alkyl and a C₂-C₇ alkanoyl,
         (iv) a C₁-C₆ alkoxy-carbonyl,
      (6) a carbamoyl optionally substituted by C₁-C₆ alkyl(s),
      (7) a C₁-C₆ alkoxy-oxalyl,
      (8) a C₃-C₈ cycloalkyl optionally substituted by group(s) selected from the group consisting of the following (i) to (vii):
         (i) a halogen atom,
         (ii) hydroxy,
         (iii) a C₁-C₆ alkoxy,
         (iv) an amino optionally substituted by group(s) selected from the group consisting of a C₁-C₆ alkyl, a C₂-C₇ alkanoyl, a C₁-C₆ alkoxy-carbonyl and a C₁-C₆ alkyl-sulfonyl,
         (v) a C₁-C₆ alkyl optionally substituted by group(s) selected from the group consisting of hydroxy, a C₁-C₆ alkoxy, amino, a carbamoyl optionally substituted by C₁-C₆ alkyl(s),
         (vi) a C₂-C₇ alkanoyl-oxy and
         (vii) a carbamoyl optionally substituted by C₁-C₆ alkyl(s),
      (9) a phenyl optionally substituted by group(s) selected from the group consisting of a halogen atom and a C₁-C₆ alkoxy,
      (10) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by group(s) selected from the group consisting of the following (i) to (v):
         (i) a C₁-C₆ alkyl optionally substituted by group(s) selected from the group consisting of phenyl, hydroxy, a C₁-C₆ alkoxy, amino and a carbamoyl optionally substituted by C₁-C₆ alkyl(s),
         (ii) a C₁-C₆ alkoxy-carbonyl,
         (iii) a C₂-C₇ alkanoyl,
         (iv) a C₁-C₆ alkyl-sulfonyl,
         (v) oxo
      (11) a carbonyl substituted by a C₃-C₈ cycloalkyl optionally substituted by group(s) selected from the group consisting of hydroxy, amino and a C₂-C₇ alkanoyl-amino, or
      (12) a carbonyl substituted by heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom,
   or a pharmaceutically acceptable salt thereof.
[8] A compound according to [1] and having the formula [Ic]: 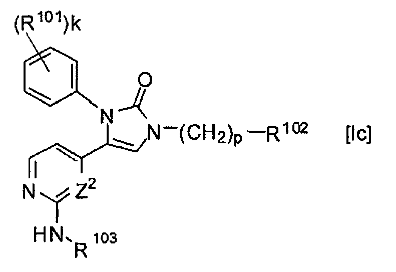 wherein each R¹⁰¹ is the same or different, and selected from the group consisting of
   (1) a halogen atom;
   (2) nitro;
   (3) a C₁-C₆ alkyl which may be substituted by 1 to 3 group(s) selected from a halogen atom(s), hydroxy(s) and amino(s);
   (4) an C₁-C₆ alkoxy which may be substituted by 1 to 3 group(s) selected from hydroxy(s) and amino(s);
   (5) an amino which may be substituted by 1 or 2 group(s) selected from (a) a C₁-C₆ alkyl which may be substituted by 1 to 3 group(s) selected from the group consisting of C₁-C₆ alkoxy(s), amino(s) and carboxy(s), and (b) a C₂-C₇ alkanoyl; and
   (6) cyano,
      k is 0 to 3,
      p is 0 or 1;
      R¹⁰² is
   (1) a C₁-C₆ alkyl which may be substituted by 1 to 3 group(s) selected from (a) a C₂-C₇ alkynyl, (b) cyano, (c) a C₁-C₆ alkoxy, (d) hydroxy, (e) amino which may be substituted with 1 or 2 substituents selected from the group consisting of a C₁-C₆ alkyl, a C₂-C₇ alkanoyl and a C₁-C₆ alkyl-sulfonyl, (f) carboxy, (g) a C₁-C₆ alkoxy-carbonyl, (h) carbamoyl which may be substituted with 1 or 2 C₁-C₆ alkyl(s), (i) phenyl and (j) naphthyl; or
   (2) a heterocyclic group, which is an unsaturated, or a partially or completely saturated monocyclic, bicyclic or tricyclic heterocyclic group containing I to 3 heteroatoms selected from nitrogen atom, oxygen atom and sulfur atom, and which may be substituted by 1 to 3 group(s) selected from (a) a halogen atom, (b) nitro, (c) a C₁-C₆ alkyl which may be substituted by a group selected from the group consisting of hydroxy, a C₁-C₆ alkoxy, a carbamoyl which may be substituted by C₁-C₆ alkyl(s) and carboxy(s), (d) cyano, (e) hydroxy, (f) amino, (g) a C₂-C₇ alkanoyl, (h) carboxy, (i) a C₁-C₆ alkoxy-carbonyl, (j) carbamoyl which may be substituted by 1 or 2 C₁-C₆ alkyl(s), (k) a C₁-C₆ alkyl-sulfonyl, and (l) phenyl,
      Z² is CH or N, and
      R¹⁰³ is
   (1) a C₁-C₆ alkyl which may be substituted by 1 to 3 group(s) selected from
      (a) hydroxy,
      (b) a C₁-C₆ alkoxy,
      (c) amino which may be substituted by 1 or 2 group(s) selected from the group consisting of a C₁-C₆ alkyl, a C₂-C₇ alkanoyl and a C₁-C₆ alkyl-sulfonyl,
      (d) a C₁-C₆ alkoxy-carbonyl,
      (e) a C₃-C₈ cycloalkyl which may be substituted by 1 to 3 group(s) selected from the group consisting of (i) hydroxy, (ii) an amino which may be substituted by C₁-C₆ alkyl(s), (iii) a C₂-C₇ alkanoyl-amino, (iv) a C₁-C₆ alkyl-sulfonylamino, (v) a C₁-C₆ alkyl which may be substituted by a group selected from hydroxy, a C₁-C₆ alkoxy, amino and a carbamoyl which may be substituted by C₁-C₆ alkyl(s), (vi) carboxy and (vii) a carbamoyl which may be substituted by C₁-C₆ alkyl(s),
      (f) phenyl which may be substituted by I to 3 group(s) selected from the group consisting of the following (i) to (vi): (i) a halogen atom, (ii) a C₁-C₆ alkoxy, (iii) amino which may be substituted by 1 or 2 group(s) selected from the group consisting of a C₁-C₆ alkyl and a C₁-C₆ alkoxy-carbonyl, (iv) a C₁-C₆ alkoxy-carbonyl, (v) carbamoyl, and (vi) morpholinylcarbonyl, and
      (g) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of the following (i) to (v): (i) a C₁-C₆ alkyl which may be substituted by 1 to 3 hydroxy(s), (ii) hydroxy, (iii) amino, (iv) a C₁-C₆ alkoxy-carbonyl, and (v) carbamoyl; or
   (2) a C₃-C₈ cycloalkyl which may be substituted by 1 to 3 group(s) selected from
      (a) a halogen atom,
      (b) a C₁-C₆ alkyl which may be substituted by 1 to 3 group(s) selected from the group consisting of (i) hydroxy, (ii) a C₁-C₆ alkoxy, (iii) amino and (iv) a carbamoyl which may be substituted by a C₁-C₆ alkyl,
      (c) hydroxy,
      (d) a C₁-C₆ alkoxy,
      (e) amino which may be substituted by 1 or 2 group(s) selected from the group consisting of (i) a C₁-C₆ alkyl, (ii) a C₂-C₇ alkanoyl, (iii) a C₁-C₆ alkoxy-carbonyl and (iv) a C₁-C₆ alkyl-sulfonyl,
      (f) carboxy,
      (g) a C₂-C₇ alkanoyl-oxy,
      (h) a C₁-C₆ alkoxy-carbonyl, and
      (i) carbamoyl which may be substituted by 1 or 2 group(s) selected from the group consisting of (i) a C₁-C₆ alkyl, (ii) a C₃-C₈ cycloalkyl and (iii) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom,
   or a pharmaceutically acceptable salt thereof.
[9] The compound according to [8], wherein p is 0, or a pharmaceutically acceptable salt thereof.
[10] The compound according to [8] or [9], wherein R¹⁰¹ is a halogen atom, a C₁ - C₄ alkyl or a C₁ - C₄ alkoxy, or a pharmaceutically acceptable salt thereof.
[11] The compound according to [8] or [9], wherein R¹⁰² is
   (1) 5- or 6-membered monocyclic heterocyclic group containing 1 to 3 heteroatoms selected from nitrogen atom, oxygen atom, and sulfur atom or
   (2) a C₁-C₆ alkyl optionally substituted by 1 to 3 substituent(s), which may be the same or different from each other, and selected from the group consisting of cyano, a C₁-C₆ alkoxy, hydroxy, amino, carboxy, a carbamoyl optionally substituted by a C₁-C₆ alkyl, and phenyl,
   or a pharmaceutically acceptable salt thereof.
[12] The compound according to any one of [8] to [11], wherein R¹⁰³ is
   (1) a C₁-C₆ alkyl optionally substituted by 1 to 3 substituent(s) which may be the same or different from each other, and selected from (a) hydroxy, (b) a C₁-C₆ alkoxy, (c) amino, (d) a C₁-C₆ alkoxy-carbonyl, (e) a C₃-C₈ cycloalkyl, (f) phenyl and (g) a heterocyclic group, which is an unsaturated, or a partially or completely saturated monocyclic, bicyclic or tricyclic heterocyclic group containing 1 to 3 heteroatoms selected from nitrogen atom, oxygen atom, and sulfur atom, or
   (2) a C₃-C₈ cycloalkyl optionally substituted by 1 to 3 substituent(s) which may be the same or different from each other, and selected from the group consisting of a halogen, a C₁-C₆ alkyl, hydroxy, a C₁-C₆ alkoxy, amino, carboxy, a C₂-C₇ alkanoyl-oxy, a C₁-C₆ alkoxy-carbonyl and carbamoyl,
   or a pharmaceutically acceptable salt thereof.
[13] A medicament comprising a compound of formula [Ia] or [Ic] as defined in any one of [1] to [12], or a pharmaceutically acceptable salt thereof.
[14] A compound of formula [Ia] or [Ic] as defined in any one of [1] to [12], or a pharmaceutically acceptable salt thereof, for use in a method for treatment of the human or animal body by therapy.
[15] A compound of formula [Ia] or [Ic] as defined in any one of [1] to [12], or a pharmaceutically acceptable salt thereof, for use as a p38 MAP kinase inhibitor.
[16] A compound of formula [Ia] or [Ic] as defined in any one of [1] to [12], or a pharmaceutically acceptable salt thereof, for use in the prophylaxis or treatment of an inflammatory disease.
[17] A compound according to [16] for a use as defined in [16], wherein the inflammatory disease is arthritis.
[18] A compound of the formula: 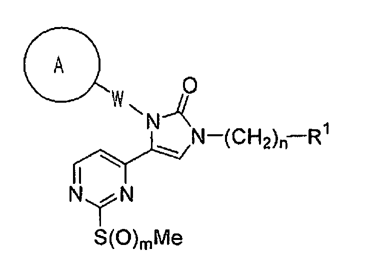 wherein m represents 1 or 2, and other symbols have the same meanings as defined in [1].
[19] In an aspect of embodiment [1], the ring A is a benzene ring which may be substituted by 1 or 2 substituent (s), which is (are) the same or different, and selected from the group consisting of a halogen atom, an optionally substituted alkyl, an optionally substituted alkoxy, an optionally substituted amino and cyano,
   W is a single bond,
   n is 0 or 1,
   R¹ is hydrogen atom, an optionally substituted alkyl, an optionally substituted cycloalkyl, on optionally substituted phenyl or an optionally substituted heterocyclic group,
   Z is CH or N,
   R² is hydrogen atom, -NR³R⁴, -OR⁵, -COR⁶ on -CHR⁷R⁸,
   where R³ to R⁸ each independently is hydrogen atom, an optionally substituted alkyl, an alkenyl, an alkoxy, an optionally substituted alkanoyl, an optionally substituted carbamoyl, an alkoxyoxalyl, an optionally substituted cycloalkyl, an optionally substituted phenyl, an optionally substituted heterocyclic group, a carbonyl substituted by an optionally substituted cycloalkyl or a carbonyl substituted by an optionally substituted heterocyclic group,
   or a pharmaceutically acceptable salt thereof.
[20] In an aspect of embodiment [1] the ring A is a benzene ring which may be substituted by 1 or 2 substituent (s), which is (are) the same or different, and selected from the group consisting of fluorine atom, chlorine atom, an alkyl and an alkoxy,
   W is a single bond,
   n is 0 or 1,
   R¹ is
   (1) hydrogen atom,
   (2) an alkyl optionally substituted by group(s) selected from the group consisting of phenyl, an alkoxy, an alkylamino, a dialkylamino, an alkanoylamino, an alkylsulfonylamino, a carbamoyl optionally substituted by alkyl (s), hydroxy, carboxy and cyano,
   (3) a cycloalkyl optionally substituted by group(s) selected from the group consisting of the following (i) to (v):
      (i) hydroxy,
      (ii) an alkoxy optionally substituted by alkoxy(s),
      (iii) an amino optionally substituted by group(s) selected from the group consisting of an alkyl, an alkanoyl and an alkylsulfonyl,
      (iv) a carbamoyl optionally substituted by alkyl(s),
      (v) an alkyl optionally substituted by group (s) selected from the group consisting of hydroxy and amino,
   (4) a phenyl optionally substituted by group (s) selected from the group consisting of the following (i) to (iv):
      (i) a halogen atom,
      (ii) an alkyl optionally substituted by halogen atom(s),
      (iii) cyano, and
      (iv) an alkoxy, or
   (5) a heterocyclic group,
      Z is CH or N,
      R² is hydrogen atom, -NR³R⁴, -OR⁵, or -COR⁶,
      where R³ to R⁶ each independently is:
   (1) hydrogen atom,
   (2) an alkyl optionally substituted by group (s) selected from the group consisting of the following (i) to (vi) :
      (i) hydroxy,
      (ii) an alkoxy,
      (iii) an alkoxycarbonyl,
      (iv) a cycloalkyl optionally substituted by group(s) selected from the group consisting of the following a) to e) :
         a) hydroxy,
         b) an amino optionally substituted by alkyl(s),
         c) an alkanoylamino,
         d) an alkyl optionally substituted by group(s) selected from the group consisting of hydroxy, amino and a carbamoyl optionally substituted by alkyl(s), and
         e) a carbamoyl optionally substituted by alkyl(s),
      (v) a phenyl optionally substituted by alkoxy(s), and
      (vi) a heterocyclic group,
   (3) an alkenyl,
   (4) an alkoxy,
   (5) an alkanoyl optionally-substituted by group(s) selected from the group consisting of an alkoxy, an amino optionally substituted by alkanoyl(s), and an alkoxycarbonyl,
   (6) a cycloalkyl optionally substituted by group(s) selected from the group consisting of the following (i) to (v):
      (i) hydroxy,
      (ii) an alkoxy,
      (iii) an amino optionally substituted by group(s) selected from the group consisting of an alkyl, an alkanoyl, an alkoxycarbonyl and an alkylsulfonyl,
      (iv) an alkyl optionally substituted by group(s) selected from the group consisting of hydroxy, amino and a carbamoyl, optionally substituted by alkyl(s),
      (v) a carbamoyl optionally substituted by alkyl(s),
   (7) a heterocyclic group optionally substituted by group (s) selected from the group consisting of an alkyl optionally substituted by phenyl(s) and an alkoxycarbonyl,
   (8) a carbonyl substituted by a cycloalkyl optionally substituted by group(s) selected from the group consisting of hydroxy and amino, or
   (9) a heterocyclic group-substituted carbonyl,
   or a pharmaceutically acceptable salt thereof.
[21] In an aspect of embodiment [1] the compound is of the formula [Ib]: 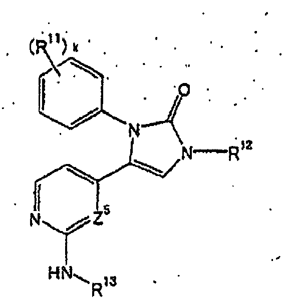 wherein R¹¹ is a group selected from the group consisting of hydrogen atom, a halogen atom, a C₁ - C₄ alkyl, and a C₁ - C₄ alkoxy,
   k is 1 or 2, and when k is 2, two of R¹¹s may be the same or different,
   R¹² is
   (1) a C₁ - C₄ alkyl,
   (2) a C₃ - C₄ cycloalkylmethyl,
   (3) carbamoylmethyl, or
   (4) a benzyl in which the benzene ring is optionally substituted by 1 to 3 group(s) selected from the group consisting of cyano, a halogen atom, a C₁ - C₃ alkoxy, a C₁ - C₃ alkyl and a halogen-substituted C₁ - C₃ alkyl,
   Z⁵ is CH or N,
   R¹³ is
   (1) a C₁ - C₆ alkyl optionally substituted by 1 to 3 group(s) selected from the group consisting of the following (i) and (ii) :
      (i) a C₅ - C₇ cycloalkyl optionally substituted by 1 to 3 group(s) selected from the group consisting of the following a) to e) :
         a) hydroxy
         b) an amino optionally substituted by C₁- C₄ alkyl(s),
         c) a C₂ - C₄ alkanoylamino,
         d) a C₁ - C₄ alkyl optionally substituted by group (s) selected from the group consisting of hydroxy, amino, and a carbamoyl optionally substituted by C₁ - C₄ alkyl(s), and
         e) a carbamoyl which may be substituted by C₁ - C₄ alkyl (s), and
      (ii) hydroxy, or
   (2) a C₅ - C₇ cycloalkyl optionally substituted by 1 to 3 group(s) selected from the group consisting of the following (i) to (iii):
      (i) hydroxy,
      (ii) a C₁ - C₄ alkyl optionally substituted by 1 to 3 group(s) selected from the group consisting of hydroxy, amino and a carbamoyl optionally substituted by C₁ - C₄ alkyl(s), and
      (iii) a carbamoyl optionally substituted by 1 or 2 C₁ - C₄ alkyl(s),
   or a pharmaceutically acceptable salt·thereof.
[22] In an aspect of embodiment [21], R¹¹ is a group selected from the group consisting of hydrogen atom, fluorine atom, chlorine atom, methyl and methoxy,
   k is 1 or 2, and when k is 2, two of R¹¹s may be the same or different,
   R¹² is a C₁ - C₄ alkyl, cyclopropylmethyl or carbamoylmethyl, or a pharmaceutically acceptable salt thereof.
[23] In an aspect of embodiment [21], R¹¹ is hydrogen atom or fluorine atom,
   k is 1,
   R¹² is ethyl, isopropyl, isobutyl, cyclopropylmethyl or carbamoylmethyl,
   R¹³ is
   (1) a C₁- C₆ alkyl, optionally substituted by 1 to 3 group(s) selected from the group consisting of the following (i) and (ii):
      (i) a C₅ - C₇ cycloalkyl optionally substituted by 1 to 3 group(s) selected from the group consisting of hydroxy and a hydroxy C₁ - C₄ alkyl, and
      (ii) hydroxy, or
   (2) a C₅- C₇ cycloalkyl optionally substituted by 1 to 3 group(s) selected from the group consisting of the following (i) to (iii):
      (i) hydroxy,
      (ii) a C₁ - C₄ alkyl optionally substituted by 1 to 3 group(s) selected from the group consisting of hydroxy, amino and a carbamoyl optionally substituted by C₁ - C₄ alkyl(s),
      (iii) a carbamoyl optionally substituted by 1 or 2 C₁ - C₄alkyl(s),
   or a pharmaceutically acceptable salt thereof.

### Best Mode for Carrying out the Invention

In the present invention, "an alkyl" and alkyls in "an alkylthio", "an alkylsulfinyl" and "an alkylsulfonyl" are, unless indicated otherwise, a straight or branched chain C₁-C₆ alkyl, such as methyl, ethyl, propyl, isopropyl, butyl, isobutyl, pentyl, hexyl. Preferred is a C₁-C₄alkyl.

"An alkoxy" and alkoxys in "an alkoxycarbonyl" and "an alkoxyoxalyl" are, unless indicated otherwise, a straight and branched chain C₁-C₆ alkoxy, such as methoxy, ethoxy, propoxy, isopropoxy, butoxy, isobutoxy, pentoxy, hexoxy. Preferred is a C₁-C₄ alkoxy.

"An alkenyl" is, unless indicated otherwise, a straight or branched chain C₂-C₇ alkenyl, such as vinyl, allyl, 3-butenyl, 2-pentenyl, 3-hexenyl. Preferred is a C₂-C₅ alkenyl.

"An alkynyl" is, unless indicated otherwise, a straight or branched chain C₂-C₇ alkynyl, such as ethynyl, propargyl, 3-butynyl, 2-pentynyl, 3-hexynyl. Preferred is a C₂-C₅ alkynyl.

"An alkanoyl" is, unless indicated otherwise, a straight or branched chain C₂-C₇ alkanoyl, such as acetyl, propionyl, butyryl, isobutyryl, pentanoyl, hexanoyl. Preferred is a C₂-C₅ alkanoyl.

"A cycloalkyl" is, unless indicated otherwise, a C₃-C₈ cycloalkyl, and preferred is a C₃-C₆ cycloalkyl.

"A cycloalkane" is, unless indicated otherwise, a C₃-C₈ cycloalkane, and preferred is a C₅-C₇ cycloalkane.

"A halogen atom" is, unless indicated otherwise, fluorine atom, chlorine atom, bromine atom, iodine atom, and preferred are fluorine atom and chlorine atom.

"A heterocyclic group" is, unless indicated otherwise, a monocyclic, bicyclic or tricyclic heterocyclic group containing 1 to 3 heteroatoms selected from nitrogen atom, oxygen atom, and sulfur atom, wherein a part or the whole of the heterocyclic group may be saturated. Preferred is a 5- or 6-membered monocyclic heterocyclic group, and specific examples are furyl, tetrahydrofuryl, tetrahydropyranyl, thienyl, thiazolyl, isothiazolyl, oxazolyl, isoxazolyl, oxadiazolyl, tetrazolyl, pyrrolyl, pyrrolidinyl, pyrrolinyl, imidazolidinyl, pyrazolidinyl, pyridyl, pyridazinyl, pyrimidinyl, pyrazinyl, triazinyl, piperidinyl, pyrazolyl, piperazinyl, morpholinyl, imidazolyl, triazolyl, imidazolinyl, pyrazolinyl.

"A monocyclic or bicyclic aromatic heterocycle" is, unless indicated otherwise, a monocyclic or bicyclic aromatic heterocycle containing 1 to 3 heteroatoms selected from nitrogen atom, oxygen atom, and sulfur atom. Additionally, "monocyclic aromatic heterocycle" is exemplified by a monocyclic aromatic heterocycle containing 1 to 3 heteroatoms selected from nitrogen atom, oxygen atom, and sulfur atom, for example, 5-or 6-membered monocyclic aromatic heterocycle. Specific examples for the monocyclic and bicyclic aromatic heterocycle include thiophene, furan, furazane, pyrrole, imidazole, pyrazole, thiazole, isothiazole, oxazole, isoxazole, oxadiazole, pyridine, pyridazine, pyrimidine, pyrazine, triazine, quinazoline, isoquinoline, phthalazine, naphthyridine, quinazoline, quinoline, chromene, indolizine, isoindole, indole, purine, benzofuran, benzothiophene. Preferred monocyclic aromatic heterocycles are thiophene, furan.

When a substituent of the ring A in the compound [Ia] is an optionally substituted alkyl, the optional substituents of the alkyl are, unless indicated otherwise, a halogen atom, hydroxy, amino. The said alkyl may have 1 to 3 substituents mentioned above, and when the number of the substituents is two or more, each of the substituents may be the same or different. Specific examples for the substituted alkyl include hydroxymethyl, trifluoromethyl, aminomethyl, chloroethyl.

When a substituent of the ring A is an optionally substituted alkoxy, the optional substituents of the alkoxy are, unless indicated otherwise, hydroxy, amino. The said alkoxy may have 1 to 3 substituents mentioned above, and when the number of the substituents is two or more, each of the substituents may be the same or different.

When a substituent of the ring A is an optionally substituted amino, the optional substituents of the amino are, unless indicated otherwise, an alkyl (said alkyl may be substituted with 1 to 3 groups which are the same or different, selected from the group consisting of an alkoxy, amino and carboxy), an alkanoyl. The said amino may have 1 or 2 substituents mentioned above, and when the number of the substituents is two, each of the substituents may be the same or different.

When a substituent of the ring A is an optionally substituted carbamoyl, the optional substituents of the carbamoyl are, unless indicated otherwise, alkyl. The said carbamoyl may have 1 or 2 substituents mentioned above, and when the number of the substituents is two, each of the substituents may be the same or different.

A substituent of the ring A in the compound [Ia] is preferably exemplified by a halogen atom, nitro, an optionally substituted alkyl, an optionally substituted alkoxy, an optionally substituted amino, and cyano. Particularly preferred are a halogen atom, a C₁-C₄alkyl, a C₁-C₄alkoxy, and specific examples are fluorine atom, chlorine atom, methyl, methoxy.

When R¹ of the compound [Ia] is an optionally substituted alkyl, the optional substituents of the alkyl are, unless indicated otherwise, an alkynyl, cyano, an alkoxy, hydroxy, amino (said amino may be substituted with 1 or 2 substituents selected from the group consisting of an alkyl, an alkanoyl, and an alkylsulfonyl.), carboxy, an alkoxycarbonyl, carbamoyl (said carbamoyl may be substituted with 1 or 2 alkyl(s).), phenyl, naphthyl. The said alkyl may have 1 to 3 substituents mentioned above, and when the number of the substituents is two or more, each of the substituents may be the same or different. Specific examples for the substituents include cyano, an alkoxy, hydroxy, amino, carboxy, a carbamoyl which may be substituted by an alkyl, phenyl.

When R¹ is an optionally substituted cycloalkyl, the optional substituents of the cycloalkyl are, unless indicated otherwise, (1) hydroxy, (2) an alkoxy (said alkoxy may be substituted by 1 to 3 alkoxy(s)), (3) amino [said amino may be substituted by 1 or 2 group(s), being the same or different, and selected from the group consisting of the following (i) to (v) : (i) an alkyl, (ii) an alkanoyl, (iii) an alkoxycarbonyl, (iv) carbamoyl ( said carbamoyl may be substituted by 1 or 2 alkyl (s).), and (v) an alkylsulfonyl], (4) carboy, (5) an alkyl (said alkyl may be substituted by a group selected form the group consisting of hydroxy, an alkoxy and amino), (6) a carbamoyl which may be substituted by alkyl (s). The said cycloalkyl may have 1 to 3 substituents mentioned above, and when the number of the substituents is two or more, each of the substituents may be the same or different.

when R¹ is an optionally substituted phenyl, the optional substituents of the phenyl are, unless indicated otherwise, (1) a halogen atom, (2) nitro, (3) an alkyl (said alkyl may be substituted by 1 to 3 group (s), being the same or different, selected from the group consisting of a halogen atom, hydroxy, amino, carboxy, and phenylsulfonyl), (4) an alkenyl, (5) cyano, hydroxy, (7) an alkoxy (said alkoxy may be substituted by 1 to 3 group (s) , being the same or different, and selected from the group consisting of a halogen atom, carboxy, an alkoxycarbonyl, carbamoyl, phenyl and morpholinylcarbonyl), (8) amino [said amino may be substituted with 1 or 2 group(s), being the same or different, and selected from the group consisting of the following (i) to (iv) : (i) an alkyl, (ii) an alkanoyl, (iii)carbamoyl (said carbamoyl may be substituted by 1 or 2 group(s), being the same or different, and selected from the group consisting of an alkyl and a cycloalkyl), and (iv) an alkylsulfonyl], (9) an alkanoyl, (10) carboxy, (11) an alkoxycarbonyl, (12)carbamoyl, [said carbamoyl may be substituted by 1 or 2 group(s), being the same or different, and selected from the group consisting of the following (i) and (ii): (i) an alkyl (said alkyl may be substituted by 1 to 3 hydroxy(s)) and (ii) a cycloalkyl] , (13) an alkylthio, (14) an alkylsulfinyl, (15) an alkylsulfonyl, (16) phenyl, (17) tetrazolyl, (18) a heterocyclic group-substituted carbonyl (said heterocyclic group may be substituted by 1 to 3 group(s) being the same or different, and selected from the group consisting of an alkyl and an alkoxycarbonyl). When R¹ is an optionally substituted phenyl, said phenyl may have 1 to 3 substituent(s) mentioned above, and when the number of the substituents is 2 or more, each of the substituents may be the same or different. Preferred substituents are (1) a halogen atom, (2) an alkyl (said alkyl may be substituted by 1 to 3 group(s), being the same or different, and selected from the group consisting of a halogen atom, hydroxy, amino, carboxy, and phenylsulfonyl), (3) cyano, (4) an alkoxy (said alkoxy may be substituted by 1 to 3 group (s) , being the same or different, and selected from'the group consisting of a halogen atom, carboxy, an alkoxycarbonyl, carbamoyl, phenyl and morpholinyl carbonyl). There is no limitation regarding positions of the substituents, as long as it is possible to substitute, and a particularly preferred position is 2-position.

when R¹ is a phenyl substituted by a heterocyclic group-substituted carbonyl, examples for the heterocyclic group include the above-mentioned heterocyclic groups, and preferred are 5- or 6-membered monocyclic nitrogen-containing aliphatic heterocyclic groups. Specific examples are pyrrolidinyl, piperidyl, piperazinyl, morpholinyl.

When R¹ is an optionally substituted heterocyclic group, examples for the heterocyclic group include the above-mentioned heterocyclic groups, and preferred are 5- or 6-membered monocyclic heterocyclic groups. Specific examples are furyl, tetrahydrofuryl, thienyl, thiazolyl, isoxazolyl, oxadiazolyl, pyridyl, pyrimidinyl, pyrazinyl, piperidinyl, pyrrolidinyl, pyrazolyl, tetrazolyl, tetrahydropyranyl. Particularly preferred are piperidinyl, tetrahydropyranyl. Further, the optional substituents of the heterocyclic group are, unless indicated otherwise, a halogen atom, nitro, an alkyl (said alkyl may be substituted by a group selected from the group consisting of hydroxy, an alkoxy, a carbamoyl which may be substituted by alkyl(s) and carboxy(s)), cyano, hydroxy, amino, an alkanoyl, carboxy, an alkoxycarbonyl, carbamoyl (said carbamoyl may be substituted by 1 or 2 alkyl(s)), an alkylsulfonyl, phenyl. The said heterocyclic group may have 1 to 3 substituent(s) mentioned above, and when the number of the substituents is 2 or more, each of the substituents may be the same or different. A preferred combination of n and R¹ in the compound [Ia] are exemplified by (1) those in which n is 0 and R¹ is an optionally substituted alkyl, (2) those in which n is 1 and R¹ is an optionally substituted cycloalkyl, (3) those in which n is 1 and R¹ is an optionally substituted phenyl, and (4) those in which n is 1 and R¹ is an optionally substituted heterocyclic group. Particularly preferred are (1) those in which n is 0 and R¹ is an optionally substituted alkyl, (2) those in which n is 1 and R¹ is an optionally substituted phenyl. Further preferred are (1) those in which n is 0 and R¹ is a C₁-C₄ alkyl, (2) those in which n is 1 and R¹ is a phenyl (said phenyl may be substituted by a group selected from the group consisting of cyano, fluorine atom, chlorine atom and methyl).

When R³ to R⁸ in the compound [Ia] is an optionally substituted alkyl, the optional substituents of the alkyl are, unless indicated otherwise, (1) hydroxy, (2) an alkoxy group, (3) amino (said amino may be substituted by 1 or 2 group(s) , being the same or different, and selected from the group consisting of an alkyl, an alkanoyl and an alkylsulfonyl), (4) an alkoxycarbonyl, (5) a cycloalkyl [said cycloalkyl may be substituted by 1 to 3 group(s), being the same or different, and selected from the group consisting of hydroxy, an amino which may be substituted by alkyl(s), an alkanoylamino, an alkylsulfonylamino, an alkyl (said alkyl may be substituted by a group selected from hydroxy, an alkoxy, amino and a carbamoyl which may be substituted by alkyl (s)), carboxy and a carbamoyl which may be substituted by alkyl(s)], (6) phenyl [said phenyl may be substituted by 1 to 3 group (s), being the same or different, and selected from the group consisting of the following (i) to (vi) : (i) a halogen atom, (ii) an alkoxy, (iii) amino (said amino may be substituted by 1 or 2 group (s) , being the same or different, and selected from the group consisting of an alkyl and an alkoxycarbonyl), (iv) an alkoxycarbonyl, (v) carbamoyl, and (vi) morpholinylcarbonyl], (7) a heterocyclic group [said heterocyclic group may be substituted by 1 to 3 group(s) , being the same or different, and selected from the group consisting of the following (i) to (v): (i) an alkyl (said alkyl may be substituted by 1 to 3 hydroxy(s)), (ii) hydroxy, (iii) amino, (iv) an alkoxycarbonyl, and(v) carbamoyl]. When R³ to R⁸ is an optionally substituted alkyl, said alkyl may have 1 to 3 substituent(s) mentioned above, and when the number of the substituents is 2 or more, each of the substituents may be the same or different.

When R³ to R⁸ is a heterocyclic group-substituted alkyl, said heterocyclic group are exemplified by the above-mentioned heterocyclic groups, and preferred are 5- or 6-membered monocyclic heterocyclic groups. Specific examples are pyridyl, pyrimidinyl, pyrazinyl, piperidyl, pyrrolidinyl, morpholinyl, thienyl, furyl.

When R³ to R⁸ is an optionally substituted amino, the optional substituents of the amino are, unless indicated otherwise, an alkyl (said alkyl may be substituted by 1 to 3 group(s), being the same or different, and selected from the group consisting of hydroxy, an alkoxy and a heterocyclic group), a cycloalkyl (said cycloalkyl may be substituted by 1 to 3 hydroxy(s)), a heterocyclic group. The said amino may have 1 or 2 substituent(s) mentioned above, and when the number of the substituents is 2 or more, each of the substituents may be the same or different.

When R³ to R⁸ is an amino substituted by a heterocyclic group-substituted alkyl or an amino substituted by a heterocyclic group, the heterocyclic group are exemplified by the above-mentioned heterocyclic groups. Preferred are 5- or 6-membered monocyclic heterocyclic groups, specific examples are pyridyl, piperidyl, pyrrolidinyl, morpholinyl.

When R³ to R⁸ is an optionally substituted alkanoyl, the optional substituents of the alkanoyl are, unless indicated otherwise, hydroxy, an alkoxy, amino (said amino may be substituted by 1 or 2 group(s), being the same or different, and selected from the group consisting of an alkyl and an.alkanoyl), an alkoxycarbonyl. The said alkanoyl may have 1 to 3 substituent(s) mentioned above, and when the number of the substituents is 2 or more, each of the substituents may be the same or different.

When R³ to R⁸ is an optionally substituted carbamoyl, the optional substituents of the carbamoyl are, unless indicated otherwise, an alkyl, a cycloalkyl, a heterocyclic group. The said carbamoyl may have 1 or 2 substituent (s) mentioned above, and when the number of the substituents is 2, each of the substituents may be the same or different.

When R³ to R⁸ is carbamoyl substituted by a heterocyclic group, the heterocyclic group is exemplified by the above-mentioned heterocyclic group, and preferred are 5- or 6-membered monocyclic heterocyclic groups. Specific examples are pyridyl, pyrimidinyl, piperidinyl.

When R³ to R⁸ is an optionally substituted cycloalkyl, the optional substituents of the cycloalkyl are, unless indicated otherwise, a halogen atom, an alkyl (said alkyl may be substituted by 1 to 3 group(s) selected from the group consisting of hydroxy, an alkoxy, amino and a carbamoyl which may be substituted by an alkyl), hydroxy, an alkoxy, amino (said amino may be substituted by 1 or 2 group(s), being the same or different, and selected from the group consisting of an alkyl, an alkanoyl, an alkoxycarbonyl and an alkylsulfonyl), carboxy, an alkanoyloxy, an alkoxycarbonyl, carbamoyl (said carbamoyl may be substituted by 1 or 2 group(s), being the same or different, and selected from the group consisting of an alkyl, a cycloalkyl and a heterocyclic group). When R³ to R⁸ is an optionally substituted cycloalkyl, the said cycloalkyl may have 1 to 3 substituent(s) mentioned above, and when the number of the substituents is 2 or more, each of the substituents may be the same or different.

When R³ to R⁸ is a cycloalkyl substituted by a heterocyclic group-substituted carbamoyl, the heterocyclic group is exemplified by the above-mentioned heterocyclic groups, and preferred are 5- or 6-membered monocyclic heterocyclic groups. Specific examples are pyridyl, pyrimidinyl, piperidinyl.

When R³ to R⁸ is an optionally substituted phenyl, the optional substituents for the phenyl are, unless indicated otherwise, an alkyl, hydroxy, an alkoxy, a halogen atom, amino (said amino may be substituted by 1 or 2 alkyl(s)). The said phenyl may have 1 to 3 substituent(s) mentioned above, and when the number of the substituent is 2 or more, each of the substituents may be the same or different.

When R³ to R⁸ is an optionally substituted heterocyclic group, the heterocyclic group is exemplified by the above-mentioned heterocyclic groups, and preferred are 5- or 6-membered monocyclic heterocyclic groups. Specific examples are piperazinyl, piperidyl, pyridyl, pyrimidinyl, pyrazinyl, pyrazolyl, pyrrolidinyl, morpholinyl, oxazolyl, thiazolyl, tetrahydropyranyl. Further, the optional substituents of the heterocyclic group are, unless indicated otherwise, an alkyl (said alkyl may be substituted by 1 to 3 group(s), being the same or different, and selected from the group consisting of phenyl, hydroxy, an alkoxy, amino and a carbamoyl which may be substituted by an alkyl), carboxy, an alkoxycarbonyl, an alkanoyl, an alkylsulfonyl, oxo. The said heterocyclic group may have 1 to 3 substituent(s) mentioned above, and when the number of the substituents is 2 or more, each of the substituents may be the same or different.

When R³ to R⁸ is a carbonyl substituted by an optionally substituted cycloalkyl, the optional substituents of the cycloalkyl are, unless indicated otherwise, hydroxy, an alkoxy, amino (said amino may be substituted by 1 or 2 group(s), being the same or different, and selected from the group consisting of an alkyl and an alkanoyl), an alkoxycarbonyl. The said cycloalkyl may have 1 to 3 substituent (s) mentioned above, and when the number of the substituents is 2 or more, each of the substituents may be the same or different.

When R³ to R⁸ is a carbonyl substituted by an optionally substituted phenyl, the optional substituents of the phenyl are, unless indicated otherwise, a halogen atom, hydroxy, an alkoxy, amino (said amino may be substituted by 1 or 2 group(s) , being the same or different, selected from the group consisting of an alkyl and an alkanoyl).

The said phenyl may have 1 to 3 substituent (s) mentioned above, and when the number of the substituents is 2 or more, each of the substituents may be the same or different.

When R³ to R⁸ is a carbonyl substituted by an optionally substituted heterocyclic group, the heterocyclic group is exemplified by the above-mentioned heterocyclic groups, and preferred are 5- or 6-membered monocyclic heterocyclic groups. Specific examples are piperidyl, pyrrolidinyl, pyridyl, pyrimidinyl, morpholinyl. Further, the optional substituents of the heterocyclic group are, unless indicated otherwise, a halogen atom, an alkyl, hydroxy, amino (said amino may be substituted by 1 or 2 alkyl(s)), an alkanoyl, oxo. The said heterocyclic group may have 1 to:3 substituent(s) mentioned above, and when the number of the substituents is 2 or more, each of the substituents may be the same or different.

R² in the compound [Ia] are preferably exemplified by -NR³R⁴ and -OR⁵, and particularly preferably exemplified by -NR³R⁴, and further more preferably exemplified by -NHR⁴.

When R² is -NHR⁴, preferred examples of R⁴ may include an optionally substituted alkyl, an alkenyl, an optionally substituted alkanoyl, an optionally substituted carbamoyl, an optionally substituted cycloalkyl, an optionally substituted phenyl, an optionally substituted heterocyclic group, a carbonyl substituted by an optionally substituted cycloalkyl and a carbonyl substituted by an optionally substituted heterocyclic group. Particularly preferred examples are an optionally substituted alkyl and an optionally substituted cycloalkyl, and more preferred examples are a C₃-C₆ alkyl (said alkyl may be substituted by hydroxy(s)), a C₅-C₇ cycloalkyl (said cycloalkyl may be substituted by a group selected from the group consisting of hydroxy, hydroxymethyl and carbamoyl).

Although an optical isomer based on an asymmetric carbon can be present in the compounds [Ia] and [Ib] of the present invention, the present invention includes any of these optical isomers as well as mixtures thereof. The compounds [Ia] and [Ib] can be used for a pharmaceutical use, in either a free form or in a form of a pharmaceutically acceptable salt. A pharmaceutically acceptable salt of the compound [Ia] and [Ib] are exemplified by an inorganic acid salt such as a hydrochloride, a sulfate, a phosphate and a hydrobromide, and an organic acid salt such as acetate, fumarate, oxalate, citrate, methanesulfonate, benzenesulfonate, tosylate and maleate. Further, in case of having a substituent such as carboxy, there are mentioned a salt with a base (for example, an alkali metal salt such as a sodium salt, a potassium salt, and an alkaline earth metal such as a calcium salt). The compounds [Ia] and [Ib] of the present invention or a salt thereof include an internal salt thereof and a solvate thereof, such as a hydrate.

The compounds [Ia] and [Ib] of the present invention or a pharmaceutically acceptable salt thereof have an excellent p38 MAP kinase inhibitory action and is useful for the prophylaxis and treatment for diseases related to the activation of p38 MAP kinase and the excessive production of inflammatory mediators concerned with p38 MAP kinase such as TNF-α, IL-1. Therefore, the compounds [Ia] and [Ib] of the present invention or a pharmaceutically acceptable salt thereof is expected to be useful for a therapeutic and prophylactic agent for inflammatory diseases, such as arthritis (e.g. rheumatoid arthritis, osteoarthritis, infectious arthritis, .gouty arthritis, traumatic arthritis, synovitis, periarthritis), inflammatory bowel disease (e.g. ulcerative colitis, Crohn's disease), inflammatory dermal disease [(e.g. psoriasis, dermatitis (e.g. atopic dermatitis, contact dermatitis, urticaria, eczema)], inflammatory respiratory disease (e.g. asthma, bronchitis, pneumonia, pleurisy, pharyngitis, rhinitis), inflammatory optical disease (e.g. conjunctivitis, keratitis, uveitis), nephritis, hepatitis, systemic inflammatory disease (e.g. Behcet's syndrome, systemic lupus erythematosus), shock (e.g. septic shock, endotoxin shock), cerebrovascular disease (e.g. cerebral hemorrhage, cerebral infarction, cerebral, edema), ischemic cardiac diseases : (e.g. angina pectoris, cardiac infarction, congestive heart failure), osteoporosis, multiple sclerosis, diabetes, malignant tumor, cachexia, Alzheimer's disease, Parkinson's disease, acquired immunodeficiency syndrome, arterial sclerosis, disseminated intravascular coagulation syndrome, rejection and graft-versus-host diseases by organ transplantation.

The compounds [Ia] and [Ib] of the present invention or a pharmaceutically acceptable salt thereof can be administered orally or parenterally, and can be used as conventional pharmaceuticals such as tablets, granules, capsules, powder, injections, inhalants. These pharmaceuticals can be prepared according to the conventional methods.

An administration amount of the compound [Ia] and [Ib] of the present invention or a pharmaceutically acceptable salt thereof depends on an administration method, age, body weight, and condition of the patient, and usually, it is preferably 0.003 to 30 mg/kg, and particularly preferably, 0.01 to 10 mg/kg.

The compounds [Ia], [Ib] and [Ic] of the present invention can be prepared suitably by a method selected from the following method A] to [Method D], however, it is not limited to these. Production method will be described in detail using the compounds [Ia] as follow, however, the compounds [Ib] can be produced in a similar manner.

### [Method A]

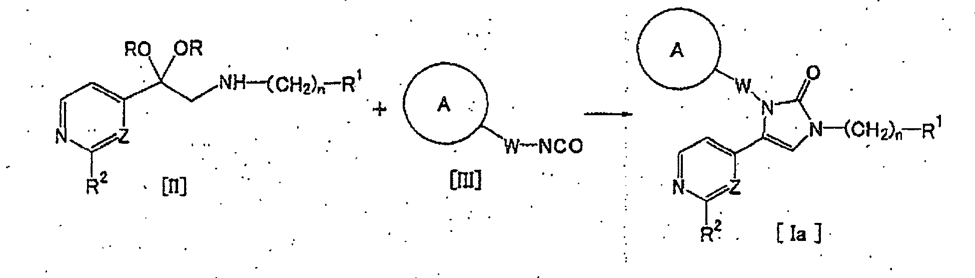

(wherein R is an alkyl, and other symbols have the same meanings as mentioned above.)

The compound [Ia] of the present invention can be produced by reacting a. compound [II] with a compound [III], followed by treading the reaction product with an acid. This reaction can be carried out in a solvent (Journal of Medicinal Chemistry, 9, 858(1966)). As the solvent, there is no limitation as long as it does not affect the reaction, for example, there are mentioned tetrahydrofuran (THF), chloroform, methylene chloride, dioxane, ethyl acetate, ether, toluene. The present reaction proceeds preferably at -20 to 80°C, particularly preferably at 0 to 30°C. Further, as an acid for an acid treatment, there are mentioned, for example, hydrochloric acid, sulfuric acid, phosphoric acid, p-toluenesulfonic acid, methanesulfonic acid. Additionally, as an alkyl of R in the formula [II], there are mentioned, for example, methyl, ethyl, propyl, butyl, and particularly preferred are methyl and ethyl.

### [Method B]

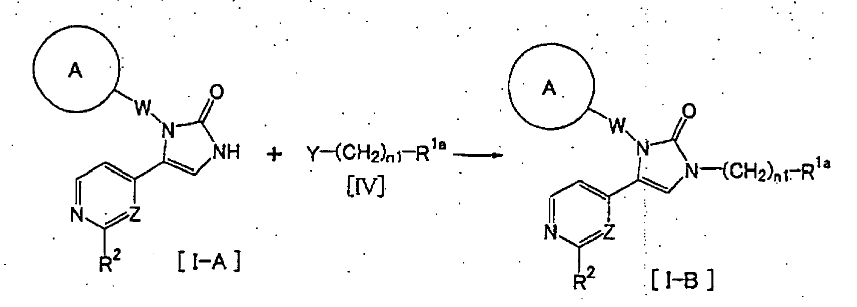

(wherein Y is a halogen atom, hydroxy, or dihydroxyboranyl, n1 is 0, 1,2,3.or 4, R^{1a} is hydrogen atom, an optionally substituted alkyl, an optionally substituted cycloalkyl, an optionally substituted phenyl, or an optionally substituted heterocyclic group (provided that the case where n1 is 0 and R^{1a} is hydrogen atom is excluded.), and other symbols have the same meanings as the above.)

The compound [I-B] which is categorized in the compound [Ia] can be produced by reacting a compound [I-A], which is a compound [Ia] where n is 0 and R¹ is hydrogen atom, with a compound [IV] for alkylation.

When Y in the formula [IV] is a halogen atom, this reactions can be carried out in a solvent, in the presence of a base. Any solvent can be used as long as it does not affect the reaction, and there are mentioned, for example, dimethylformamide (DMF), dimethylsulfoxide, 1-methylpyrrolidone, 1,3,-dimethyl-2-imidazolidinone. As the base, there are mentioned, for example, sodium hydride, sodium hydroxide, potassium t-butoxide, butyllithium, lithium diisopropylamide. The reaction proceeds preferably at -24to 104°C, particularly preferably at 0 to 30°C. Further, as the halogen atom at Y, there are mentioned chlorine, bromine and iodine, and bromine and iodine are particularly preferred.

When Y in the formula [IV] is hydroxy, the reaction can be carried out in a solvent, in the presence of an additive and an activator (Synthesis, 1 (19981)). Any solvent can be used as long as it does not affect the reaction, and there are mentioned, for example, methylene chloride, THE, dioxane, chloroform. As the additive, there are mentioned, for example, triphenylphosphine, tributylphosphine, trimethylphosphine. As the activator, there are mentioned, for example, diethyl azodicarboxylate, dimethyl azodicarboxylate, 1,1-azobis(N,N-dimethylformamide), 1,1-(azodicarbonyl) dipiperidine. This reaction proceeds' preferably at -30 to 100°C, and particularly preferably at 0 to 50°C.

When Y in the formula [IV] is dihydroxyboranyl, the reaction can be carried out in a solvent, in the presence of a catalyst and a base (Tetrahedron Letters, 39, 29333((1998)). Any solvent can be used as long as it does not affect the reaction, and there are mentioned, for example, methylene chloride, chloroform, DMF. As the catalyst, there are mentioned, for example, copper (II) acetate. As the base, there are mentioned, for example, triethylamine, diisopropylethylamine, 4-methylmorpholine, pyridine. This reaction proceeds preferably at -10 to 100°C, and particularly preferably at 20 to 60°C.

### [Method C]

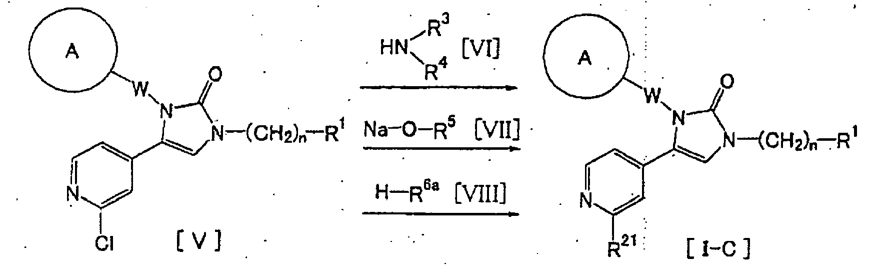

(wherein R²¹ is -NR³R⁴, -OR⁵ or -COR^{6a} , R^{6a} is an alkoxy, and other symbols have the same meanings as the above.)

The compound [I-C] which is categorized in the compound [Ia] of the present invention can be produced by reacting a compound [V] with a compound [VI], a compound [VII] or a compound [VIII].

The reaction between the compound [V] and the compound [VI] can be carried out in a solvent, in the presence of a catalyst, a base and an additive (Journal of Organic Chemistry, 61, 7240(1996)). Any solvent can be used as long as it does not affect the reaction, and there are mentioned, for example, toluene, xylene, dimethoxyethane, dioxane.

As the catalyst, there are mentioned, for example, palladium acetate, bis(dibenzylideneacetane)dipalladium. As the base, there are mentioned, for example, sodium t-butoxide, potassium t-butoxide, lithium t-butoxide, triethylamine. As the additive, there are mentioned, for example, 2,2'-bis(diphenylphosphino)-1,1'binaphthyl. The reaction proceeds preferably at 30 to 150°C, and particularly preferably at 60 to 80°C.

The reaction between the compound [V] and the compound [VII] can be carried out in a solvent. Any solvent can be used as long as it does not affect the reaction, and there are mentioned, for example, THF, dioxane, DMF, toluene, methanol ethanol.
The reaction proceeds preferably at 20 to 150°C, and particularly preferably at 70 to 100°C.

The reaction between the compound [V] and the compound [VIII] can be carried out in a solvent, in the copresence of carbon monoxide, and in the presence of a catalyst and an additive (Tetrahedron, 55, 393 (1999)). Any solvent can be used as long as it does not affect the reaction, and there are mentioned, for example, toluene, dioxane, DMF. As the catalyst, there are mentioned, for example, palladium acetate, palladium chloride, bis (triphenylphosphine) palladium dichloride, tetrakis (triphenylphosphine) palladium, As the additive, there are mentioned, for example, 1,1'-bis(dipherylphosphino)ferrocene, 1,4-bis(diphenylphosphino)butane, 1,3-bis(diphenylphosphino) propane, triphenylphosphine. The reaction proceeds preferably at 30 to 250°C, and particularly preferably at 80 to 120°C.

### [Method D]

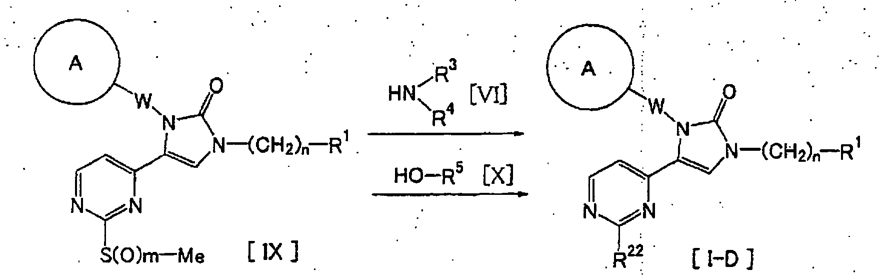

(wherein m is 1 or 2, R²² is -NR³R⁴ for -OR⁵ and other symbol have the same meanings as the above.)

The compound [I-D] which is categorized in the compound [Ia] of the present invention can be produced by reacting a compound [IX] with a compound [VI] or a compound [X].

The reaction between the compound [IX] and the compound [VI] can be carried out in a solvent. Any solvent can be used as long as it does not affect the reaction, and there are mentioned, for example, dioxane, THF, DMF, dimethylsulfoxide.

The reaction proceeds preferably at 0 to 150 °C, and particularly preferably at 50 to 100°C.

The reaction between the compound [IX] and the compounds [X] can be carried out in a solvent, in the presence of a base. Any solvent can be used as long as it does not affect the reaction, and there are mentioned, for example, THF, dioxane, DMF, dimethylsulfoxide. As the base, there are mentioned, for example, sodium hydride, sodium hydroxide, potassium' t-butoxide, butyllithium. The redaction proceeds preferably at -30 to 100 °C, and particularly preferably at 0 to 30°C.

The compound [Ia] produced above can also be derived to other compounds [Ia] by converting a functional group using properly a conventionally known organic chemistry reaction. Such a method for converting a functional group may be suitably selected depending on a kind of a desired functional group. For example, a conversion of a functional group of R² in the compound [Ia] can be carried out according to the following (method a) to (method g).

### (Method a)

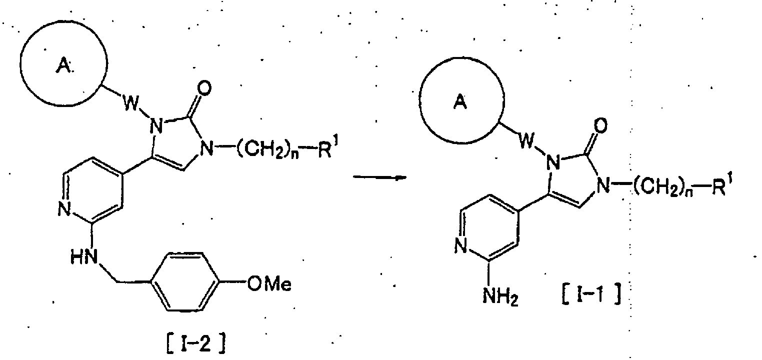

(wherein the symbols have the same meanings as the above.)

The compound [I-1] can be produced by reacting a compound [I-2] with a hydrogen halide. As the hydrogen halide, there are mentioned hydrogen fluoride, hydrogen chloride, hydrogen bromide, hydrogen iodide, and particularly preferred is hydrogen bromide. This reaction proceeds preferably at 0 to 150°C, particularly preferably at 60 t 80°C.

### (Method b)

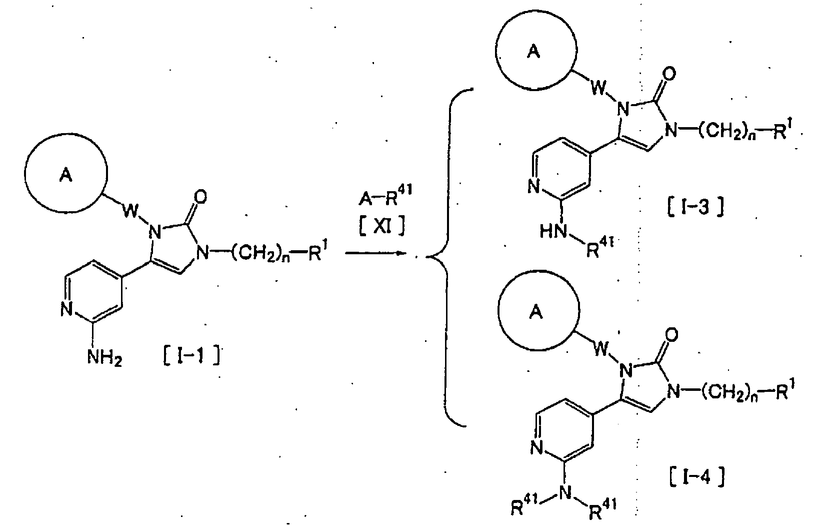

(wherein R⁴¹ is an alkanoyl which may be substituted, an alkylsulfonyl, carbonyl substituted by a cycloalkyl which may be substituted, carbonyl substituted by a phenyl which may be substituted, or carbonyl substituted by a heterocyclic group which may be substituted. A is a halogen atom or hydroxy.
other symbols have the same meanings as the above.)

The compound [I-3] and compound [I-4] can be produced by reacting a compound [I-1] with a compound [XI].

When A in the formula [XI] is a halogen atom, this reaction, can be carried out in a solvent in the presence of a base. Any solvent can be used as long as it does not affect the reaction, and there are mentioned, for example, methylene chloride, chloroform, THF, DMF. As the base, there are mentioned, for example, triethylamine, diisopropylethylamine, 4-methylmorpholine, pyridine. The reaction proceeds preferably at -40 to 100°C, particularly preferably at -10 to 30.°C. Further, as the halogen atom at X, there are mentioned fluorine, chlorine, bromine, and iodine, and particularly preferred are chlorine and bromine.

When A in the formula [XI] is hydroxy, this reaction can be carried out in a solvent in the presence of a condensing agent. Any solvent can be used as long as it does not affect the reaction, and there are mentioned, for example, methylene chloride, chloroform, THF, DMF. As the condensing agent, there are mentioned, for example, 1,1'-carbonyldiimidazole, 1,3-dicyclohexylcarbodiimide, 1,(3-dimethylaminopropyl)-3-ethylcarbodiimide hydrochloride. The reaction proceeds preferably at -40 to 100°C, particularly preferably at -10 to 30°C.

### (Method c)

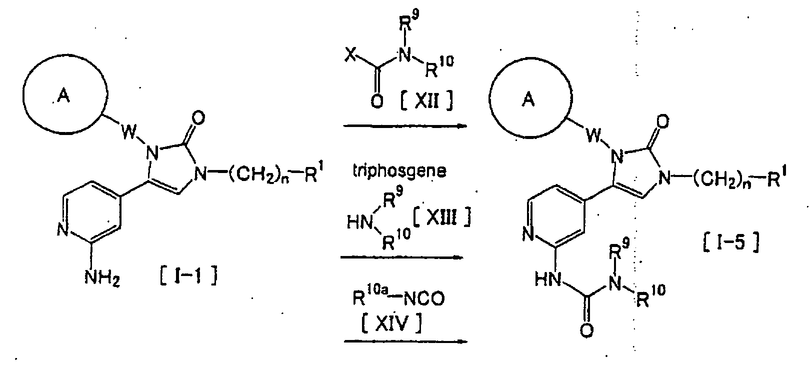

(wherein R⁹ and R¹⁰ are independently hydrogen atom, or an alkyl.
R^{10a} is an alkyl. X is a halogen, atom. Other symbols have the same meanings as the above.)

The compound [I-5] can be produced by reacting a compound [I-1] with a compound [XII], with triphosgene'and a compound [XIII], or with a compound [XIV].

The compound [I-5] can be produced by reacting a compound [I-1] with a compound [XII] in a solvent. Any solvent can be used as long as it does not affect the reaction, and there are mentioned, for example, methylene chloride, chloroform, THF.

As the halogen atom at X in the formula [XII], fluorine, chlorine, bromine, and iodine are mentioned, and preferred is chlorine. The reaction proceeds preferably at -20 to 100°C and particularly at 10 to 60°C.

Further, the compound [I-5] can be produced by reacting a compound (I-1) with triphosgene in a solvent, and then, by reacting with a compound [XIII] . Any solvent can be used as long as it does not affect the reaction, and there are mentioned, for example, methylene chloride, chloroform, THF. The reaction proceeds preferably at -20 to 100°C and particularly at 10 to 60°C.

Still further, a compound [I-5] in which R⁹ is a hydrogen atom and R¹⁰ is an alkyl can be produced by reacting a compound [I-1] with a compound [XIV] in a solvent. Any solvent can be used as long as it does not affect the reaction, and there are mentioned, for example, THF, methylene chloride, chloroform.

The reaction proceeds preferably at -20 to 100°C and particularly at 10 to 60°C.

### (Method d)

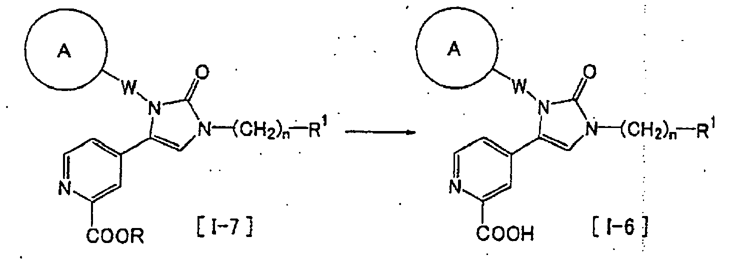

(wherein R is an alkyl, and other symbols have the same meanings as the above.)

The compound [I-6] can be produced by hydrolyzing a compound [I-7] by a conventional method.

### (Method e)

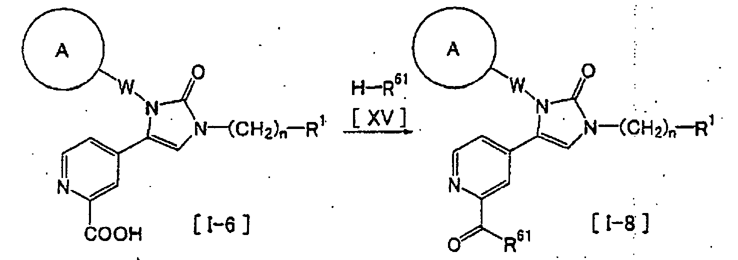

(wherein R⁶¹ is an amino which may be substituted, and other symbols have the same meanings as the above.)

The compound [I-8] can be-produced by reacting a compound [I-5] with a compound [XV] in a solvent, in the presence of as condensing agent. Any solvent can be used as long as it does not affect the reaction, and there are mentioned, for example, methylene chloride, acetonitrile, DMF, THF. As the condensing agent, there are mentioned, for example, 1,1'-carbonyldiimidazole, 1,3-dicyclohexylcarbodiimide, 1,(3-dimethylaminopropyl)-3-ethylcarbodiimide hydrochloride. The reaction proceeds preferably at -30 to 100°C and particularly at 0 to 70°C.

### (Method f)

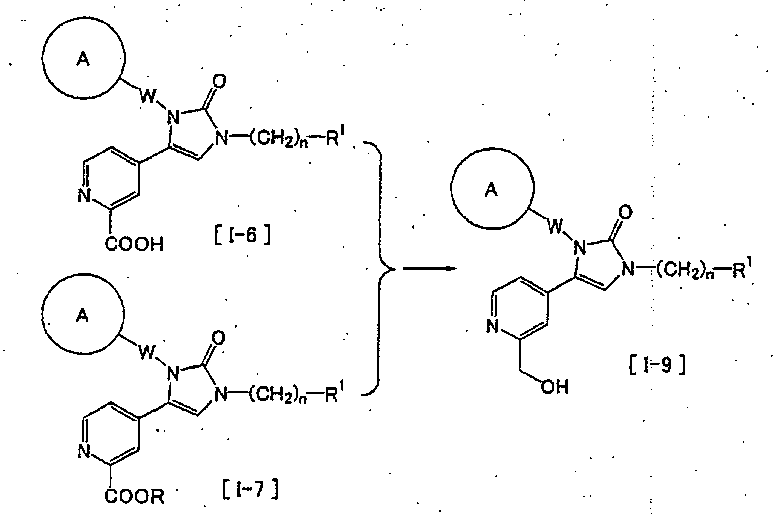

### (wherein symbols have the same meanings as the above.)

the compound [I-9] can be produced by reducing a compound [I-6] or a compound [I-7] in a solvent. Any solvent can be used as long as it does not affect the reaction, and there are mentioned, for example, THF, diethyl ether. As the reducing agent, there are mentioned, for example, lithium aluminum hydride, sodium borohydride, lithium borohydride.

The reaction proceeds preferably at -20 to 70°C and particularly at 0 to 40°C.

### (Method g)

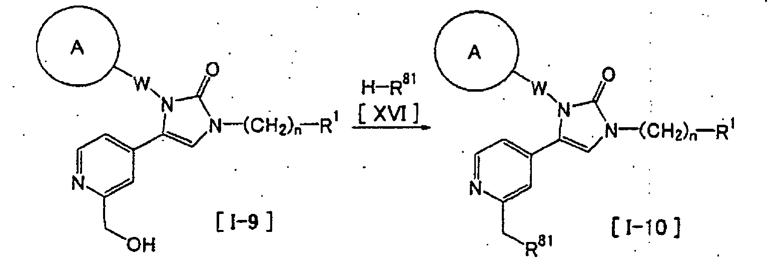

(wherein R⁸¹ is an optionally substituted amino, and other symbols have the same meanings as the above.)

The compound [I-10] can be produced by reacting a compound [I-9] with a compound [XVI] in a solvent, in the presence of a base and an activating agent. Any solvent can be used as long as it does not affect the reaction, and there are mentioned, for example, methylene chloride, THF, chloroform, toluene. As the base, there are mentioned, for example, triethylamine, diisopropylethylamine, pyridine. As the activating agent, there are mentioned, for example, methanesulfonyl chloride, p-toluenesulfonyl chloride. The reaction proceeds preferably at -10 to 60°C and particularly at 0 to 30°C.

The compound [Ia] of the present invention obtained according to the above described [Method A] to [Method D] or (Method a) to (Method g) can be optionally converted to a pharmaceutically acceptable salt. Conversion to a pharmaceutically acceptable salt may be carried out by methods known to the person skilled in the art.

In the following, production methods for starting materials used in the above methods are described.

The starting material [II] can be produced as follows. 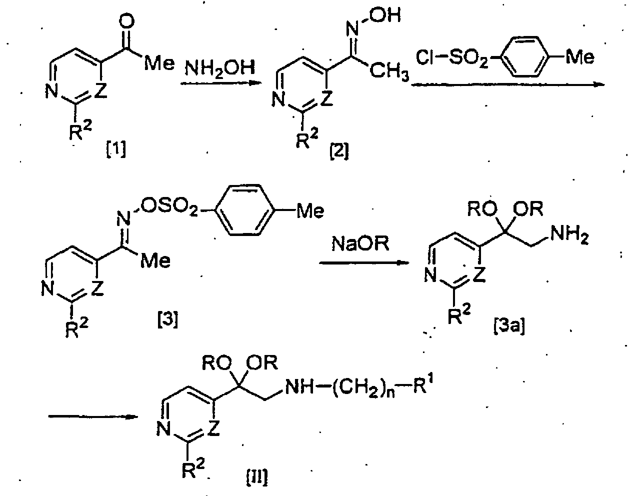 (wherein the symbols have the same meanings as the above.)

The reaction for producing the compound [2] from the compound [1] and hydroxylamine can be carried out in a solvent. Any solvent can be used as long as it does not affect the reaction, and there are mentioned, for example, ethanol, methanol. The reaction proceeds preferably at 0 to 150°C, and particularly preferably at 60 to 80°C.

The reaction for producing the compound [3] from the compound [2] and tosyl chloride can be carried out in a solvent. Any solvent can be used as long as it does not affect the reaction, and there are mentioned, for example, methylene chloride, chloroform, THF, toluene. As the base, there are mentioned, for example, triethylamine, diisopropylethylamine, pyridine. The reaction proceeds preferably at -20 to 80°C, and particularly preferably at 0 to 30°C.

The reaction for producing the compound [3a] from the compound [3] can be carried out in a solvent, by reacting the compound [3] with sodium alkoxide, followed by treating the reactant with an acid. Any solvent can be used as long as it does not affect the reaction, and there are mentioned, for example, methanol, ethanol, dioxane, THF, dimethoxyethane.

As the acid, there are mentioned, for example, hydrogen chloride. The reaction proceeds preferably at -20 to 60°C, and particularly preferably at 0 to 30°C.

The reaction for producing the compound [II] from the compound [3a] can be carried out by reacting a corresponding aldehyde using a conventional reductive alkylation (Journal of Organic Chemistry, 61, 3849(1996)).

A starting material [V] can be produced, for example, as follows. 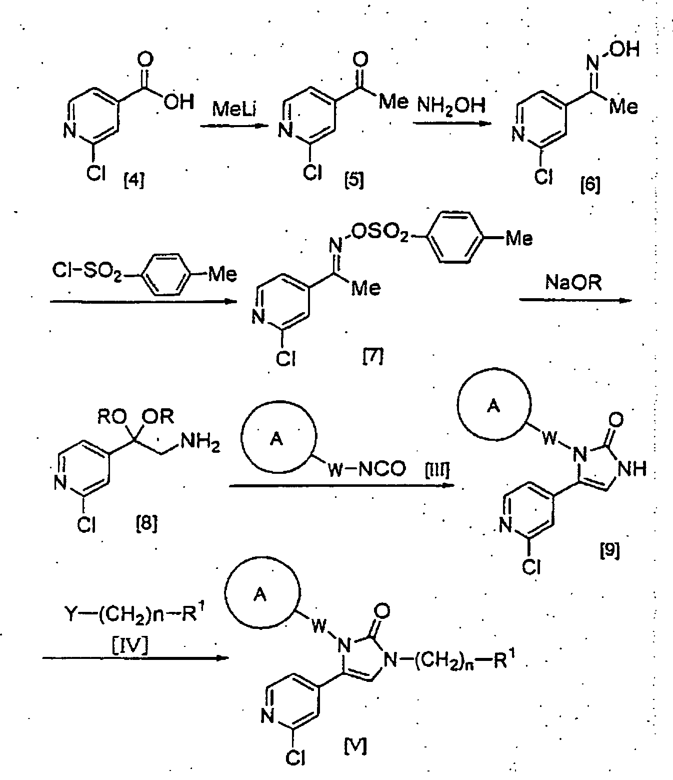 (wherein the symbols have the same meanings as the above.)

The reaction for producing the compound [5] from the compound [4] and methyl lithium can be carried out in a solvent. Any solvent can be used as long as it does not affect the reaction, and there are mentioned, for example, THF, diethyl ether, dimethoxyethane. The reaction proceeds preferably at -90 to 0°C, and particularly preferably at -60 to -40°C.

The method for producing the compound [8] from the compound [5] via the compound [6] and the compound [7] can be carried out in a similar manner to the above-mentioned method for producing the compound [II] from the compound [1] via the compound [2] and the compound [3].

The reaction for producing the compound [9] from the compound [8] and the compound [III] can be carried out in a similar manner to the above-mentioned [Method A].

The reaction for producing.the compound [V] from the compound [9] and the compound [IV] can be carried out in a similar manner to the above-mentioned [Method B]

A starting material [IX] can be produced, for example, as follows. 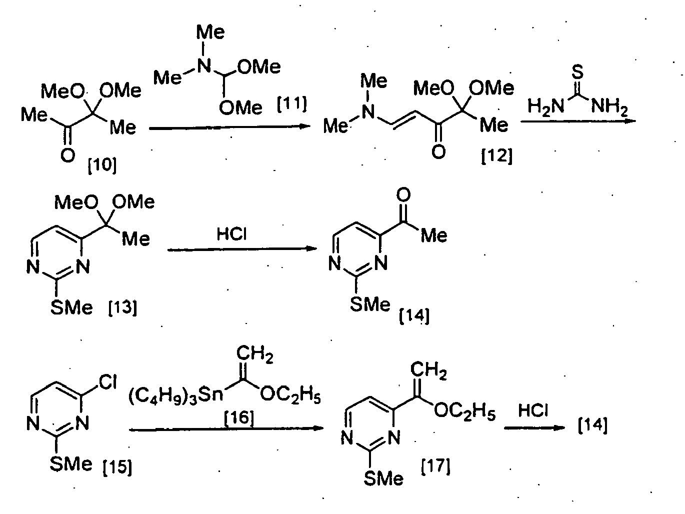 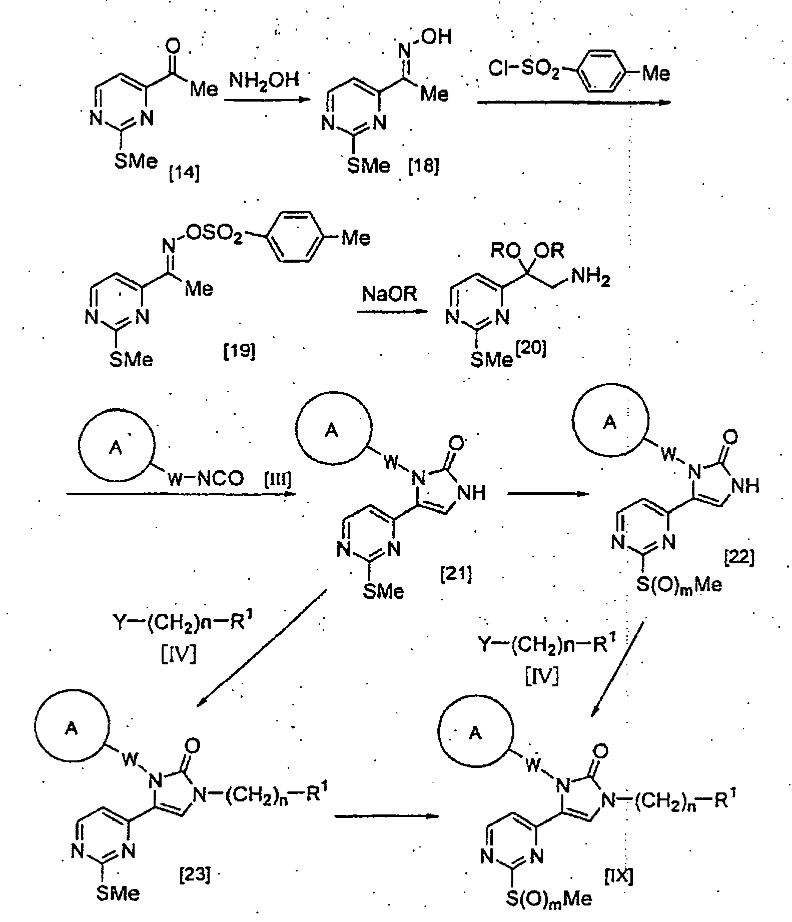 (wherein m is 1 or 2, and other symbols have the same meanings as the above.)

The reaction for producing the compound [12] from the compound [10] and the compound [11] can be carried out in a solvent or without solvent. Any solvent can be used as long as it does not affect the reaction, and there are mentioned, for example, toluene, xylene, dioxane. The reaction proceeds preferably at 50 to 150°C, and particularly preferably at 80 to 120°C.

The reaction for producing the compound [13] from the compound [12] can be carried out by reacting the compound [12] with thiourea in a solvent, in the presence of a base, and then, by reacting an alkylating agent. Any solvent can be used as long as it does not affect the reaction, and there are mentioned, for example, methanol, THF, dioxane. As the base, there are mentioned, for example, sodiummethoxide, sodium hydroxide, potassium t-butoxide. As the alkylating agent, there are mentioned, for example, methyl iodide, dimethyl sulfate.
The reaction proceeds preferably at 0 to 100 C, and particularly preferably at 30 to 70°C.

The reaction for producing the compound [14] from the compound [13] can be carried out in a solvent, in the presence of an acid. Any solvent can be used as long as it does not affect the reaction, and there are mentioned, for example, water, acetone, THF, dioxane. As the acid, there are mentioned, for example, hydrochloric acid, sulfuric acid, phosphoric acid, p-toluenesulfonic acid. The reaction proceeds preferably at -10 to 80°C, and particularly-preferably at 0 to 30°C.

The compound [14] can be also produced from the compound [15] via the compound [17].

The reaction for producing the compound [17] from the compound [15] and the compound [16] can be carried out in a solvent, in the presence of a catalyst . Any solvent can be used as long as it does not affect the reaction, and there are mentioned, for example, DMF, toluene, xylene. As the catalyst, there are mentioned, for examples, bis(triphenylphosphine)palladium dichloride, tetrakis(triphenylphosphine)palladium. The reaction proceeds preferably at 50 to 150°C, and particularly preferably at 70 to 90°C.

The reaction for producing the compound [14] from the compound [17] can be carried out in a similar manner to the above-mentioned method for producing the compound [14] from the compound [13].

The reaction for producing the compound [20] from the compound [14] via the compound [18] and the compound [19] can be carried out in a similar manner to the above-mentioned method for producing the compound [II] from the compound [1] via the compound [2] and the compound [3].

The reaction for producing the compound [21] from the compound [20] and the compound [III] can be carried out in a similar manner to the above-mentioned [Method A].

The reaction for producing the compound [22] from the compound [21] can be carried out in a solvent, using an oxidizing agent. Any solvent can be used as long as it does not affect the reaction, and there are mentioned, for example, water, methanol, THF, dioxane, chloroform, methylene chloride. As the oxidizing agent, there are mentioned, for example, Oxon (trade name, manufactured by DuPont Co. Ltd.), 3-chloroperoxybenzoic acid, hydrogen peroxide. The reaction proceeds preferably at -20 to 60°C, and particularly preferably at 10 to 30°C.

The reaction for producing the compound [IX] from the compound [22] and the compound [IV] can be carried out in a similar manner to the above-mentioned [Method B].

The compound [IX] can be also produced from the compound [21] via the compound [23].

The reaction for producing the compound [23] from the compound [21] and the compound [IV] can be carried out in a similar manner to the above-mentioned [Method B].

The reaction for producing the compound [IX] from the compound [23] can be carried out in a similar manner to the reaction for producing the compound [22] from the compound [21].

Incidentally, in the above production methods, it is possible to optionally protect or deprotect a functional group. As the protecting group for the functional group, those used in a field of conventional organic synthetic chemistry can be used, examples of which include those described in "Protective Groups in Organic Synthesis" by T. W. Greene, P. M. G. Wuts, (published by John Wiley and Sons, 1991) . For conditions for introducing protecting groups or condition; for de-protection, the method described in the above reference can be mentioned.

Further, each compound and each intermediate produced in the above production methods can be purified by means of a conventional method, for example, column chromatography, recrystallization. As a solvent for recrystallization, there are mentioned, for example, an alcohol solvent such as methanol, ethanol, 2-propanol, an ether solvent such as diethyl ether, an ester solvent such as ethyl acetate, an aromatic solvent such as toluene, a ketone solvent such as acetone, a hydrocarbon solvent such as hexane, water, and a mixed solvent thereof. Further, the compounds [Ia], [Ib] and [Ic] of the present invention can be converted to a pharmaceutically acceptable salt according to the conventional method, and recrystallization can be carried out afterwards.

### Examples

Hereinbelow, the present invention will be explained'in more detail with reference to the following Examples, which should not be construed as limiting the scope of the present invention.

Each of the following symbols used in the present specification represents the meaning as described below.
Me : methyl
Et : ethyl
THF: tetrahydrofuran
DMF: N,N-dimethylformamide
t- : tert-

### Example 1

### 1-(4-Fluorophenyl)-5-(pyridin-4-yl)-4-imidazolin-2-one

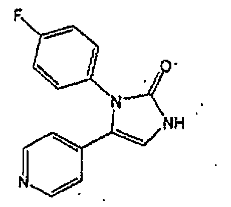

A solution of 3.00 g of 2,2-diethoxy-2-pyridin-4-ylethylamine (a compound obtained in Reference Example 2) dissolved in 30 ml of THF was cooled by water, and 1.97 g of 4-fluorophenylisocyanate was added by dropwise. After addition, the reaction mixture was concentrated under reduced pressure, and then, 30 ml of conc. hydrochloric acid was added to the obtained residue, and the mixture was stirred at room temperature overnight. To 180 ml of an ice cold aqueous 2N NaOH solution was added the reaction mixture for neutralization, and precipitated crystals were collected by filtration. They were washed with water and ether, air-dried at 60°C, to give 3.10 g of the title compound as colorless crystals. Melting point: 261°C (decomposed)

### Example 2

### 1-Cyclopentylmethyl-3-(4-fluorophenyl)-4-(pyridin-4-yl)-4-imidazolin-2-one·hydrochloride

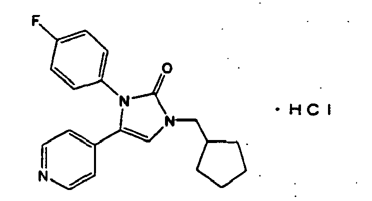

128 mg of 1-(4-Fluorophenyl)-5-(pyridin-4-yl)-4-imidazolin-2-one (the compound of Example 1), 61 µl of cyclopentylmethanol, 197 mg of triphenylphosphine and 295 µl of diethyl azodicarboxylate were dissolved in 2.5 ml of methylene chloride, and the mixture was stirred at room temperature for 24 hours. The reaction mixture was concentrated under reduced pressure, and the residue was purified by silica gel column chromatography (chloroform : ethyl acetate = 19 : 1). The obtained compound was treated with hydrochloric acid, to give 75 mg of the title compound as powder.

### Example 3

### 1-(Oxolan-3-yl)-3-(4-fluorophenyl)-4-(pyridin-4-yl)-4-imida zolin-2-one

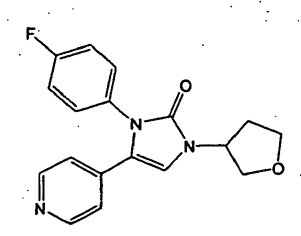

The title compound was given by treating the corresponding starting material in a similar manner to that in Example 2. Melting point : 132-134°C

### Example 4

### 1-(2-Cyanobenzyl)-3-(4-fluorophenyl)-4-[(2-(1-(S)-phenyl*ethylamino)pyridin-4-yl)]-4-imidazolin-2-one

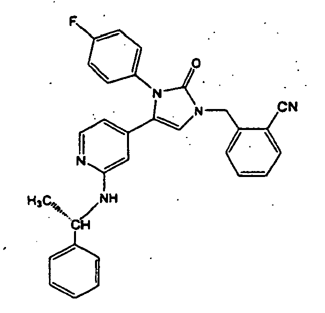

50 mg of 4-(2-Chloropyridin-4-yl)-3-(4-fluorophenyl)-1-(2-cyanobenzy 1) -4-imidazolin-2-one (a compound of Reference Example 1 (6)), 79 µl of (S)-(-)-α-methylbenzylamine, 5.5 mg of palladium acetate, 15 mg of 2,2'-bis(diphenylphsophino)-1,1-binaphthyl and 17 mg of sodium t-butoxide were suspended in 1 ml of toluene, and the mixture was stirred at 70 °C for 18 hours, under nitrogen flow. The reaction mixture was diluted by ethyl acetate, and insoluble matter was removed by filtration through Celite. To the filtrate was added 6N hydrochloric acid, and after separation, an aqueous layer was made alkaline with aqueous sodium bicarbonate solution. The mixture was extracted with chloroform, washed with saturated brine, and dried over anhydrous magnesium sulfate. After concentration under reduced pressure, the residue was purified by silica gel column chromatography (hexane : ethyl acetate = 1 : 2), to give 38 mg of the title compound as colorless powder.

### Examples 5 - 12

Compounds in Table 1 were obtained by treating the corresponding starting materials in a similar manner to that in Example 4.

**Table 1**

| | | | |
|---|---|---|---|
| 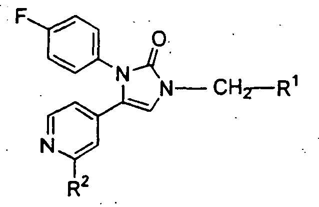 | | | |

| Example | R¹ | R² | Physical properties, etc. |
|---|---|---|---|
| 5 | 2-Cyanophenyl | 4-Methoxybenzylamino | Melting point 167°C |
| 6 | 2-Cyanophenyl | 2-Thienylmethylamino | Melting point 171°C |
| 7 | 2-Cyanophenyl | (S)-1-t-Butoxycarbonylethylamino | Melting point 191-193°C |
| 8 | 2-Cyanophenyl | Isopropylamino | Melting point 170-171°C |
| 9 | 2-Cyanophenyl | Allylamino | Melting point 163°C |
| 10** | 2-Methoxyphenyl | 2-Pyridylmethylamino | Melting point 248-250°C |
| 11 | 2-Fluorophenyl | 2-(2-Pyridyl)ethylamino | Melting point 132-134°C |
| 12** | 2-Trifluoromethylphenyl | 2-(2-Pyridyl)ethylamino | Powder |

| | | | |
|---|---|---|---|
| **:Dihydrochloride | | | |

### Example 13

### 4-(2-Aminopyridin-4-yl)-1-(2-cyanobenzyl)-3-(4-fluorophenyl)-4-imidazolin-2-one

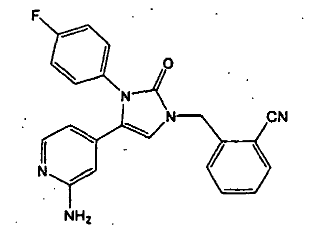

To 1.5 g of 1-(2-cyanobenzyl)-3-(4-fluorophenyl)-4-[2-(4-methoxybenzylamino)pyridin-4-yl]-4-imidazolin-2-one (Compound of Example 5) was added 3 ml of 25% hydrogen bromide-acetic acid solution, and the mixture was stirred at 70°C for one hour. The reaction mixture was concentrated under reduced pressure, and the residue was made alkali with an aqueous sodium bicarbonate solution. The mixture was extracted with ethyl acetate, washed with brine and dried over anhydrous magnesium sulfate. After concentration under reduced pressure, the residue was purified by silica gel column chromatography (chloroform:methanol=20:1) to give 572 mg of the title compound as colorless crystal. Melting point : 182-183°C.

### Example 14

### 4-(2-N-Isobutyroylaminopyridin-4-yl)-1-(2-cyanobenzyl)-3-(4-fluorophenyl)-4-imidazolin-2-one

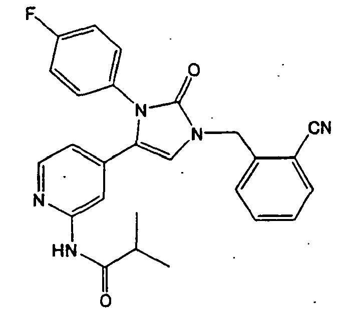

### Example 15

### 4-(2-N,N-Diisobutyroylaminopyridin-4-yl)-1-(2-cyanobenzyl)-3-(4-fluorophenyl)-4-imidazolin-2-one

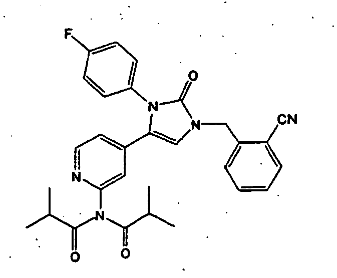

A suspension of 50 mg of 4-(2-aminopyridin-4-yl)-1-(2-cyanobenzyl)-3-(4-fluorophenyl)-4-imidazolin-2-one (Compound of Example 13) and 20 µl of isobutyroyl chloride in methylene chloride was ice-cooled, and after adding 54 µl of triethylamine by dropwise, and the mixture was stirred at room temperature for 3 hours. Water was added to the reaction mixture, and the mixture was extracted with ethyl acetate, washed with brine and dried over anhydrous magnesium sulfate. After concentration under reduced pressure, the residue was purified by silica gel column chromatography (chloroform: acetone=20:1) to give 22 mg of the title compound (Example 14) as colorless crystal and 10 mg of the title compound (Example 15) as colorless crystal, respectively. Melting point:196°C (Example 14), 185-187°C (Example 15).

### Example 16

### 4-(2-Ethoxycarbonylpyridin-4-yl)-1-(2-cyanobenzyl)-3-(4-fluorophenyl)-4-imidazolin-2-one

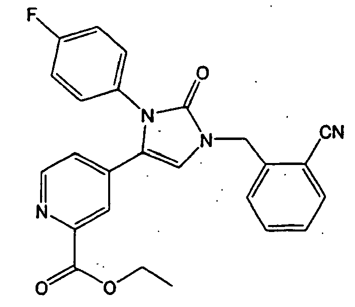

In 20 ml of ethanol were suspended 1 g of 4-(2-chloropyridin-4-yl)-3-(4-fluorophenyl)-1-(2-cyanobenzyl)-4-imidazolin-2-one [Compound of Reference example 1(6)], 55 mg of palladium acetate, 137 mg of 1,1'-bis(diphenylphosphino)-ferrocene and 608 mg of sodium acetate, the mixture was stirred under carbon monoxide atmosphere at 80°C for 12 hours. The reaction mixture was concentrated under reduced pressure, the residue was suspended in ethyl acetate, treated with activated charcoal and then filtered. After concentration under reduced pressure, the residue was purified by silica gel column chromatography (hexane:ethyl acetate=1:2) to give 887 mg of the title compound as colorless crystal. Melting point:164°C.

### Example 17

### 1-(2-Cyanobenzyl)-3-(4-fluorophenyl)-4-[2-(3-hydroxypropylamino)pyrimidin-4-yl]-4-imidazolin-2-one

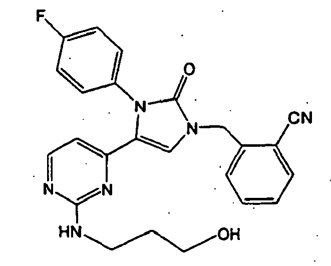

A mixture of 70 mg of 1-(2-cyanobenzyl)-3-(4-fluorophenyl)-4-(2-methylsulfinylpyrimidin-4-yl)-4-imidazolin-2-one (Compound of Reference example 6 (2) or Reference example 7(2)), 60.6 mg of 3-aminopropanol and 2 ml of dioxane was stirred at 80°C for 5 hours. The reaction mixture was concentrated and then purified by silica gel column chromatography (chloroform: methanol=19:1) and crystallized from ether to give 44.6 mg of the title compound. Melting point: 166-167°C.

### Examples 18 to 24

The corresponding starting materials were treated in the same manner as in Example 17 to give Compounds in Table 2.

**Table 2**

| | | | |
|---|---|---|---|
| 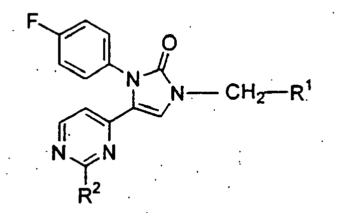 | | | |

| Example | R¹ | R² | Physical properties, etc. |
|---|---|---|---|
| 18 | 2-Cyanophenyl | 2-Furylmethylamino | Melting point 174-175°C |
| 19 | 2-Cyanophenyl | 3-Methoxypropylamino | Melting point 168-169°C |
| 20 | 2-Cyanophenyl | Isobutylamino | Melting point 145-146°C |
| 21 | 2-Cyanophenyl | Allylamino | Melting point 189-190°C |
| 22 | 2-Cyanophenyl | 4-Hydroxybutylamino | Melting point 166-167°C |
| 23 | 2-Methoxyphenyl | Isopropylamino | Melting point 171-172°C |
| 24 | 2-Fluorophenyl | Isopropylamino | Melting point 120-122°C |

### Example 25

### 1-(2-Cyanobenzyl)-3-(4-fluorophenyl)-4-(2-isopropoxypyrimidin-4-yl)-4-imidazolin-2-one

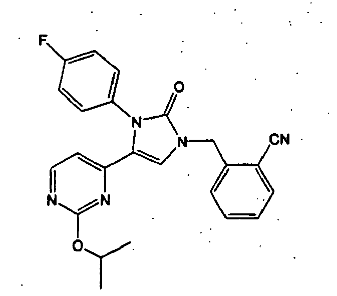

In 5 ml of isopropanol was suspended 100 mg of 1-(2-cyanobenzyl)-3-(4-fluorophenyl)-4-(2-methylsulfinylpyrimidin-4-yl)-4-imidazolin-2-one (Compound of Reference example 6 (2) or Reference example 7(2)), 26.3 mg of sodium hydride was added to the mixture and the resulting mixture was stirred at room temperature for 5 hours. To the reaction mixture were successively added an aqueous citric acid solution and an aqueous sodium bicarbonate solution, and the resulting mixture was extracted with ethyl acetate. The organic layer was washed, dried and concentrated, and the residue was purified by silica gel column chromatography (chloroform:methanol=30:1) to give 68 mg of the title compound as powder.

### Examples 26 to 79

The compound of Reference example 1(5) and the corresponding starting materials were subjected to N-alkylation in the same manner as in Example 2 or Reference example 1(6), and then, subjected to amination in the same manner as in Example 4 to give the compounds shown in Tables 3 to 6.

**Table 3**

| | | | |
|---|---|---|---|
| 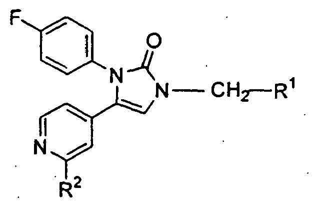 | | | |

| Example | R¹ | R² | MS ((M+H]⁺) |
|---|---|---|---|
| 26 | 2-Cyanophenyl | Benzylamino | 476 |
| 27 | 2-Cyanophenyl | Cyclopropylamino | 426 |
| 28 | 2-Cyanophenyl | 2-Furylmethylamino | 466 |
| 29 | 2-Cyanophenyl | 2-Pyridylmethylamino | 477 |
| 30 | 2-Cyanophenyl | Cyclopentylamino | 454 |
| 31 | 2-Cyanophenyl | 4-Chlorobenzylamino | 510 |
| 32 | 2-Cyanophenyl | 2-Methoxybenzylamino | 506 |
| 33 | 2-Cyanophenyl | 3-Methoxybenzylamino | 506 |
| 34 | 2-Cyanophenyl | 3-Pyridylmethylamino | 477 |
| 35 | 2-Cyanophenyl | 2-Methylpyridin-4-ylmethyl amino | 491 |
| 36 | 2-Cyanophenyl | 2-(2-Pyridyl)-ethylamino | 491 |
| 37 | 2-Cyanophenyl amino | (4-Methyl-1-piperazinyl)- | 484 |
| 38 | 2-Cyanophenyl | 3-Methoxypropylamino | 458 |
| 39 | 2-Cyanophenyl | 3-Propoxypropylamino | 486 |

**Table 4**

| | | | |
|---|---|---|---|
| 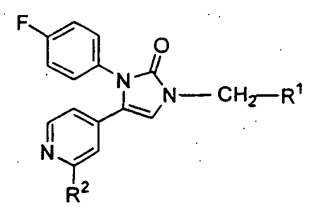 | | | |

| Example | R¹ | R² | MS ((M+H]⁺) |
|---|---|---|---|
| 40 | 2-Cyanophenyl | Cyclopropylmethylamino | 440 |
| 41 | 2-Cyanophenyl | 3-Isopropoxypropylamino | 486 |
| 42 | 2-Fluorophenyl | 2-Pyridylmethylamino | 470 |
| 43** | 2-Trifluoromethylphenyl | 2-Pyridylmethylamino | 520 |
| 44 | 2-Cyanophenyl | Isobutylamino | 442 |
| 45 | 2-Cyanophenyl | 2-Ethoxyethylamino | 458 |
| 46 | 2-Trifluoromethylphenyl | Isopropylamino | 471 |
| 47 | 2-Fluorophenyl | Isopropylamino | 421 |
| 48 | 2-Methoxyphenyl | Isopropylamino | 433 |
| 49 | 2-Fluorophenyl | Isobutylamino | 435 |
| 50 | 2-Methoxyphenyl | Isobutylamino | 447 |
| 51 | 2-Cyanophenyl | t-Butylamino | 442 |
| 52 | 2-Cyanophenyl | 4-Tetrahydropyranylamino | 470 |
| 53 | 2-Cyanophenyl | (S)-1-(2-Pyridyl)ethylamino | 491 |

| | | | |
|---|---|---|---|
| **:Dihydrochloride | | | |

**Table 5**

| | | | |
|---|---|---|---|
| 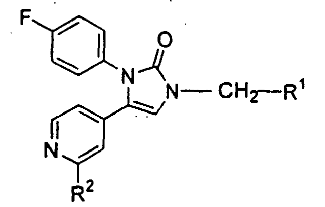 | | | |

| Example | R¹ | R² | MS ([M+H]⁺) |
|---|---|---|---|
| 54 | 2-Fluorophenyl | trans-4-Hydroxycyclohexylamino | 477 |
| 55 | 4-Methoxyphenyl | Isopropylamino | 433 |
| 56 | 2-Cyanophenyl | trans-4-Hydroxycyclohexylamino | 484 |
| 57 | 4-Methoxyphenyl | (S)-1-(2-Pyridyl)ethylamino | 495 |
| 58 | 2-Fluorophenyl | 4-Methoxybenzylamino | 499 |
| 59 | cis-4-Methoxymethoxycyclohexyl | Isobutylamino | 483 |
| 60 | cis-4-Methoxymethoxycyclohexyl | trans-4-Hydroxycyclohexylamino | 524 |
| 61 | cis-4-Methoxymethoxycyclohexyl | Isopropylamino 469 Isopropylamino | 469 |
| 62 | 2-Fluorophenyl | (1-Methyl-4-piperidyl)-amino | 476 |
| 63 | 2-Fluorophenyl | (1-t-Butoxycarbonyl-4-p iperidyl)amino | 562 |
| 64 | 2-Cyanophenyl | (1-Methyl-4-piperidyl)-amino | 483 |
| 65* | Cyclopentyl | Isopropylamino | 395 |
| 66 | Cyclopentyl | trans-4-Hydroxycyclohexylamino | 451 |
| 67* | 4-Tetrahydropyranyl | Isopropylamino | 411 |

| | | | |
|---|---|---|---|
| *:Monohydrochloride;**:Dihydrochloride | | | |

**Table 6**

| | | | |
|---|---|---|---|
| 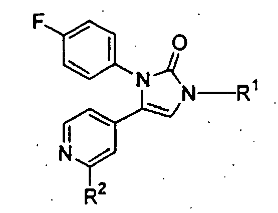 | | | |

| Example | R¹ | R² | MS ([M+H]⁺) |
|---|---|---|---|
| 68* | 4-Tetrahydropyranyl | trans-4-Hydroxycyclohexylamino | 467 |
| 69 | 2-Methoxyethyl | trans-4-Hydroxycyclohexylamino | 427 |
| 70 | Methoxymethyl | trans-4-Hydroxycyclohexylamino | 413 |
| 71 | Methoxymethyl | Isopropylamino | 357 |
| 72 | Methyl | trans-4-Hydroxycyclohexylamino | 383 |
| 73* | Ethyl | trans-4-Hydroxycyclohexylamino | 397 |
| 74 | Isopropyl | trans-4-Hydroxycyclo- | 411 |
| 75** | Isopropyl | trans-4-Aminocyclohexylamino | 410 |
| 76* | Isopropyl | trans-4-Acetylaminocyclohexylamino | 452 |
| 77* | N-Isopropylcarbamoylmethyl | Isopropylamino | 412 |
| 78** | Isopropyl | trans-4-Dimethylaminocyclohexylamino | 438 |
| 79** | Isopropyl | trans-4-Carbamoylmethylamino-cyclohexylamino | 467 |

| | | | |
|---|---|---|---|
| *:Monohydrochloride ; **:Dihydrochloride | | | |

### Example 80

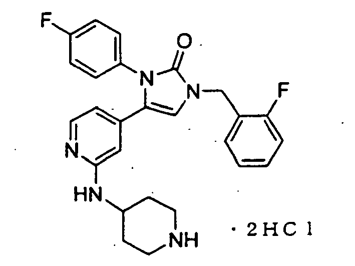

To 146 mg of the compound in Example 63 were added 0.2 ml of ethyl acetate and 1.7 ml of a 4N hydrogen chloride-ethyl acetate solution, and the mixture was stirred at room temperature for 3 hours. The reaction mixture was concentrated under reduced pressure, and ethyl acetate was added to the residue and powder was collected by filtration to give 128 mg of the title compound. MS 462 ([M+H]⁺)

### Example 81

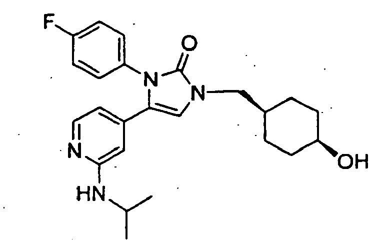

To 2 ml of methanol was dissolved 148 mg of the compound in Example 61, 1 ml of conc. hydrochloric acid was added to the mixture and the resulting mixture was stirred at room temperature overnight. The reaction mixture was concentrated under reduced pressure, and the residue was neutralized with a 4N aqueous NaOH solution and extracted with chloroform. After drying and concentration, diethyl ether and diisopropyl ether were added to the residue and the resulting powder was collected by filtration to give 58 mg of the title compound.
MS 425([M+H]⁺)

### Examples 82 to 107

The compounds of Examples 26 to 79 or the corresponding starting materials obtained in the similar method were treated in the same manner as in Example 80 or Example 81 to give the compounds shown in Tables 7 to 9.

**Table 7**

| | | | |
|---|---|---|---|
| 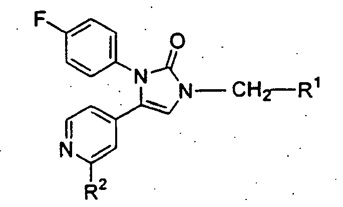 | | | |

| Example | R¹ | R² | MS ([M+H]⁺) |
|---|---|---|---|
| 82** | 4-Piperidyl | Isopropylamino | 410 |
| 83** | 2-Cyanophenyl | 4-Piperidylamino | 469 |
| 84 | cis-4-Hydroxycyclohexyl | Isobutylamino | 439 |
| 85** | cis-4-Aminocyclohexyl | Isopropylamino | 424 |
| 86** | cis-4-Aminocyclohexyl | trans-4-Hydroxycyclohexylamino | 480 |
| 87 | cis-4-Hydroxycyclohexyl | trans-4-Hydroxycyclohexylamino | 481 |
| 88 | cis-4-Hydroxycyclohexyl | (1-Methyl-4-Piperidyl) amino | 480 |
| 89 | trans-4-Aminocyclohexyl | trans-4-Hydroxycyclohexylamino | 480 |
| 90** | 4-Piperidyl | Isobutylamino | 424 |
| 91** | 4-Piperidyl | trans-4-Hydroxycyclohexylamino | 466 |
| 92** | trans-4-Aminocyclohexyl | Isobutylamino | 438 |
| 93** | cis-4-Aminocyclohexyl | Isobutylamino | 438 |
| 94*** | cis-4-Aminocyclohexyl | 4-Piperidylamino | 465 |

| | | | |
|---|---|---|---|
| **:Dihydrochloride ; ***:Trihydrochloride | | | |

**Table 8**

| | | | |
|---|---|---|---|
| 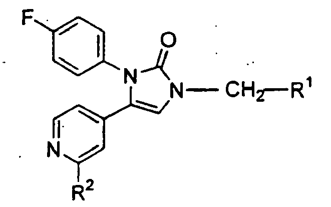 | | | |

| Example | R¹ | R² | MS ([M+H]⁺) |
|---|---|---|---|
| 95** | cis-4-Hydroxycyclohexyl | 4-Piperidylamino | 466 |
| 96*** | trans-4-Aminocyclohexyl | 4-Piperidylamino | 465 |
| 97** | trans-4-Aminocyclohexyl | Isopropylamino | 424 |
| 98** | 2-Fluorophenyl | trans-4-Aminocyclohexyl amino | 476 |
| 99** | 2-Cyanophenyl | trans-4-Aminocyclohexyl amino | 483 |
| 100* | trans-4-Hydroxycyclohexyl | Isopropylamino | 425 |
| 101* | trans-4-Hydroxycyclohexyl | Isobutylamino | 439 |
| 102* | trans-4-Hydroxycyclohexyl | trans-4-Hydroxycyclohexylamino | 481 |
| 103 | 1-Hydroxycyclopropyl | Isopropylamino | 383 |
| 104* | 1-Hydroxycyclopropyl | trans-4-Hydroxycyclohexylamino | 439 |

| | | | |
|---|---|---|---|
| *:Monohydrochloride;**:Dihydrochloride;***:Trihydrochloride | | | |

**Table 9**

| | | |
|---|---|---|
| 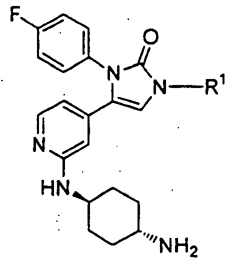 | | |

| Example | R¹ | MS ([M+H]⁺) |
|---|---|---|
| 105 | Methoxymethyl | 412 |
| 106** | 2-Methoxyethyl | 426 |
| 107** | Ethyl | 396 |

| | | |
|---|---|---|
| **:Dihydrochloride | | |

### Examples 108 to 126

The 11compound of Reference example 8 and a corresponding isocyanate were reacted in the same manner as in Example 1 to carry out cyclization, and the corresponding amine was reacted in the same manner as in Example 4 to give the compounds shown in Tables 10 and 11.

**Table 10**

| | | |
|---|---|---|
| 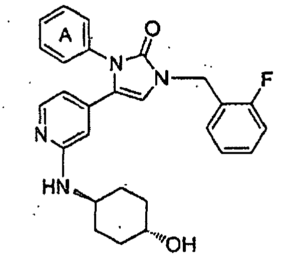 | | |

| Example | Ring A | MS ([M+H]⁺) |
|---|---|---|
| 108 | Phenyl | 459 |
| 109* | 2-Fluorophenyl | 477 |
| 110* | 3-Fluorophenyl | 477 |
| 111* | 3,4-Difluorophenyl | 495 |
| 112* | 2,4-Difluorophenyl | 495 |
| 113* | 4-Chlorophenyl | 493 |
| 114* | 4-Methylphenyl | 473 |
| 115* | 4-Methoxyphenyl | 489 |
| 116* | 3-Methoxyphenyl | 489 |
| 117* | 4-Fluorobenzyl | 491 |
| 118* | 3-Trifluoromethylphenyl | 527 |
| 119* | 3-Chlorophenyl | 493 |
| 120* | 3-Methylphenyl | 473 |
| 121* | 4-Fluoro-3-Methoxyphenyl | 507 |
| 122* (reference Example) | 3-Hydroxyphenyl | 475 |
| 123* | 2-Thienyl | 465 |

| | | |
|---|---|---|
| * : Monohydrochloride | | |

**Table 11**

| | | |
|---|---|---|
| 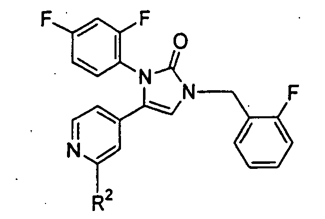 | | |

| Example | R² | MS ([M+H]⁺) |
|---|---|---|
| 124* | Isopropylamino | 439 |
| 125* | Isobutylamino | 453 |
| 126** | (1-Methyl-4-piperidyl)amino | 494 |

| | | |
|---|---|---|
| * : Monohydrochloride ; ** : Dihydrochloride | | |

### Example 127

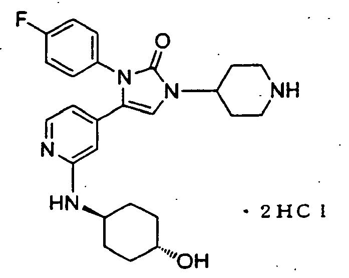

The compoud of Reference example 9 was subjected to amination in the same manner as in Example 4, and then, treated in the same manner as in Example 80 to give the title compound.
MS 452 ([M+H]⁺)

### Examples 128 to 141

The compound of Reference example 8 or Reference example 10 and a corresponding starting compound were subjected to amination in the same manner as in Example 4, and then, the resulting compound was treated with a corresponding isocyanate in the same manner as in Example 1 to carry out cyclization to give the compounds shown in Tables 12 and Table 13.

**Table 12**

| | | |
|---|---|---|
| 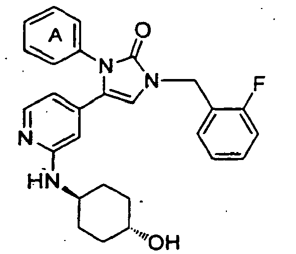 | | |

| Example | Ring A | MS ([M+H] ⁺) |
|---|---|---|
| 128* | 3-Amino-4-fluorophenyl | 492 |
| 129* | 3-Aminophenyl | 474 |
| 130* | 3-hydroxymethylphenyl | 489 |
| 131* | 2-Aminophenyl | 474 |
| 132* | 2-Nitrophenyl | 504 |
| 133* | 4-Fluoro-2-nitrophenyl | 522 |
| 134* | 2-Cyanophenyl | 484 |
| 135* | 3,5-Difluorophenyl | 495 |
| 136* | 2-Carbamoylphenyl | 502 |

| | | |
|---|---|---|
| *:Monohydrochloride | | |

**Table 13**

| | | |
|---|---|---|
| 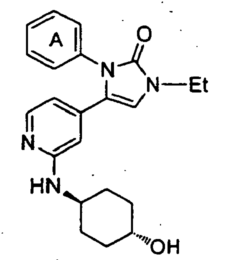 | | |

| Example | Ring A | MS ([M+H]⁺) |
|---|---|---|
| 137* | 3-Chlorophenyl | 413 |
| 138* | 3-Methylphenyl | 393 |
| 139* | 3, 4-Difluorophenyl | 415 |
| 140* | 4-Chlorophenyl | 413 |
| 141* | 2-Cyanophenyl | 404 |

| | | |
|---|---|---|
| * : Monohydrochloride | | |

### Examples 142 to 156

The compound of Reference example 11 and a corresponding starting compound were subjected to N-alkylation in the same manner as in Reference example 8, and then, the resulting compound was treated with a corresponding isocyanate to carry out cyclization in the same manner as in Example 1 to give the compounds shown in Table 14 and Table 15.

**Table 14**

| | | |
|---|---|---|
| 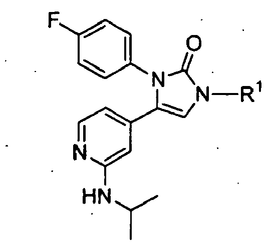 | | |

| Example | R¹ | MS ([M+H]⁺) |
|---|---|---|
| 142* | 4-Tetrahydropyranyl | 397 |
| 143** | 1-Methyl-4-piperidyl | 410 |
| 144* | Cyclohexyl | 395 |
| 145* | Cyclopentyl | 381 |
| 146* | Cyclobutyl | 367 |
| 147* | 4-Piperidyl | 396 |

| | | |
|---|---|---|
| * : Monohydrochloride ; ** : Dihydrochloride | | |

**Table 15**

| | | | | |
|---|---|---|---|---|
| 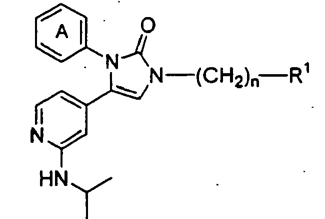 | | | | |

| Example | Ring A | n | R¹ | MS ( [M+H)⁺) |
|---|---|---|---|---|
| 148* | phenyl | 1 | trans-4-Hydroxycyclohexyl | 407 |
| 149* | 3-Fluorophenyl | 1 | trans-4-Hydroxycyclohexyl | 425 |
| 150* | 3-Chlorophenyl | 1 | trans-4-Hydroxycyclohexyl | 441 |
| 151* | 3-Methylphenyl | 1 | trans-4-Hydroxycyclohexyl | 421 |
| 152* | 3-Methoxyphenyl | 1 | trans-4-Hydroxycyclohexyl | 437 |
| 153* | 2, 4-Difluorophenyl | 1 | trans-4-Hydroxycyclohexyl | 443 |
| 154* | 3, 4-Difluorophenyl | 1 | trans-4-Hydroxycyclohexyl | 443 |
| 155* | 4-Chlorophenyl | 1 | trans-4-Hydroxycyclohexyl | 441 |
| 156* | 2-Carbamoylphenyl | 0 | Isopropyl | 380 |

| | | | | |
|---|---|---|---|---|
| * : Monohydrochloride | | | | |

### Examples 157 to 161

By using the compound of Example 147, it was reacted with a corresponding starting compound to carry out acylation in the same manner as in Example 14 to give the compounds of Examples 157 and 158 shown in Table 16. Also, by using the compound of Example 147, it was reacted with a corresponding starting compound to carry out N-alkylation in the same manner as in Reference example 10 to give the other compounds shown in Table 16. Incidentally, in synthesis of the compound of Example 160, t-butyl bromoacetate.was used as a corresponding starting compound, and after the reaction, the ester was hydrolyzed under the same conditions as in Example 80.

**Table 16.**

| | | |
|---|---|---|
| 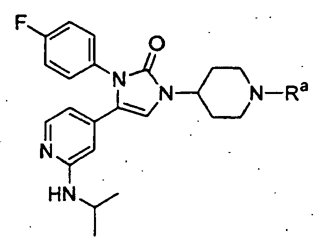 | | |

| Example | R^{a} | MS ([M+H]⁺) |
|---|---|---|
| 157* | Acetyl | 438 |
| 158* | Ethoxycarbonyl | 468 |
| 159** | Carbamoylmethyl | 453 |
| 160** | Carboxymethyl | 454 |
| 161** | N-Methylcarbamoylmethyl | 467 |

| | | |
|---|---|---|
| * : Monohydrochloride ; ** : Dihydrochloride | | |

### Examples 162 to 168

By using the compound of Reference example 11, it was reacted with a corresponding starting compound to carry out N-alkylation in the same manner as in Reference example 10, and then, the resulting compound was subjected to cyclization in the same manner as in Example 1 to give the compound of Table 17.

**Table 17**

| | | |
|---|---|---|
| 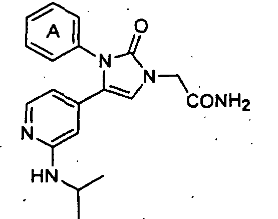 | | |

| Example | Ring A | MS ([M+H]⁺) |
|---|---|---|
| 162* | 3-Fluorophenyl | 370 |
| 163* | 3-Chlorophenyl | 386 |
| 164* | 3-Methylphenyl | 366 |
| 165* | 3-Trifluoromethylphenyl | 420 |
| 166* | Phenyl | 352 |
| 167* | 2,4-Difluorophenyl | 388 |
| 168* | 4-Chlorophenyl | 386 |

| | | |
|---|---|---|
| * : Monohydrochloride | | |

### Example 169

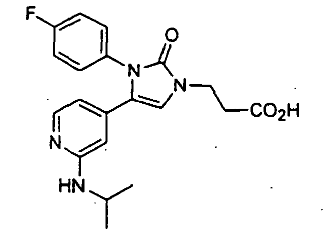

The compound (2.12 g) of Reference example 12 was subjected to cyclization in the same manner as in Example 1 and simultaneously t-butyl ester was hydrolyzed to give 1.28 g of the title compound.
MS 385 ([M+H]⁺)

### Example 170

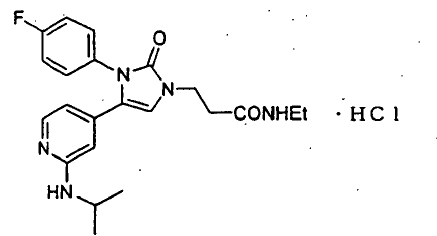

(1) A mixture comprising 100 mg of the compound of Example 169, 48 mg of 1-hydroxybenzotriazole, 60 mg of 1-(3-dimethylaminopropyl)-3-ethylcarbodiimide hydrochloride and 1 ml of methylene chloride was stirred at room temperature for one hour. To the reaction mixture was added 1 ml of a 2N ethylamine-THF solution, and the resulting mixture was stirred at room temperature overnight. The reaction mixture was successively washed with water, a saturated aqueous sodium bicarbonate solution and brine, and dried over anhydrous magnesium sulfate. To the residue obtained by concentration under reduced pressure was added diethyl ether to collect colorless crystal by filtration.
(2) The compound obtained in (1) was dissolved in 2 ml of a mixed solvent comprising chloroform-methanol, and after adding 0.2 ml of 4N hydrochloric acid-ethyl acetate, and the resulting mixture was concentrated under reduced pressure. To the residue was added ethyl acetate and collected by filtration to give 75 mg of the title compound.
   MS 412 ( [M+H]⁺)

### Examples 171 to 173

The compound of Example 169 was reacted with a corresponding amine in the same manner as in Example 170 to give the compounds shown in Table 18.

**Table 18**

| | | |
|---|---|---|
| 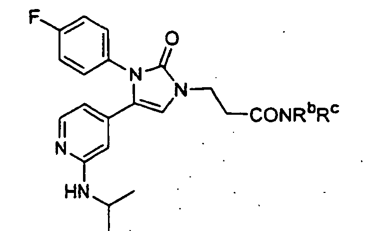 | | |

| Example | NR^{b}R^{c} | MS ([M+H]⁺) |
|---|---|---|
| 171* | Amino | 384 |
| 172* | Methylamino | 398 |
| 173* | Dimethylamino | 412 |

| | | |
|---|---|---|
| * : Monohydrochloride | | |

### Examples 174 to 178

The compound of Reference example 11 was reacted with a corresponding isocyanate in the same manner as in Example 1 to give the compounds shown in Table 19.

**Table 19**

| | | |
|---|---|---|
| 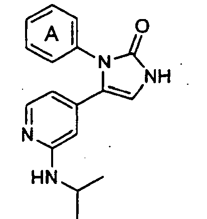 | | |

| Example | Ring A | MS ([M+H]⁺) |
|---|---|---|
| 174* | 3,4-Difluorophenyl | 331 |
| 175* | 4-Methoxyphenyl | 325 |
| 176* | 3-Trifluoromethylphenyl | 363 |
| 177* | 3-Chlorophenyl | 329 |
| 178* | 3-Methylphenyl | 309 |

| | | |
|---|---|---|
| * : Monohydrochloride | | |

### Example 179

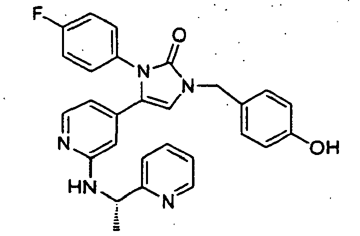

To 5 ml of 25% HBr-acetic acid solution was added 490 mg of the compound of Example 57, and the mixture was stirred at 70°C for 15 hours. After cooling the reaction mixture, an aqueous sodium bicarbonate solution was added to neutralize the mixture, and the resulting mixture was extracted with ethyl acetate, washed with brine and dried over magnesium sulfate. After concentration under reduced pressure, the residue was purified by silica gel column chromatography to give 237 mg of the title compound as colorless powder.
MS 482 ([M+H]⁺)

### Example 180

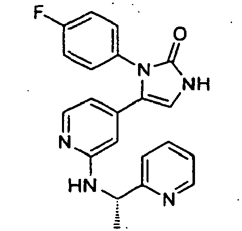

To 200 mg of the compound of Example 179 was added 2 ml of 25% HBr-acetic acid solution, and the mixture was stirred under heating at 80°C for 3 days. After cooling the reaction mixture, an aqueous sodium bicarbonate solution was added thereto to make alkaline, and the mixture was extracted with ethyl acetate, washed with brine and dried over magnesium sulfate. After concentration under reduced pressure, the residue was purified by silica gel column chromatography to give 71 mg of the title compound as colorless powder.
MS 376 ([M+H]⁺)

### Examples 181 to 183

By using the compound of Example 55, it was reacted in the same manner as in Examples 179 and 180 to give the compounds of Examples 181 and 182 shown in Table 20. Also, in the same manner as in Example 55, a corresponding compound having isobutylamino group was synthesized, and subsequently the compound was reacted in the same manner as in Example 180 to give the compound of Example 183.

**Table 20**

| | | | | |
|---|---|---|---|---|
| 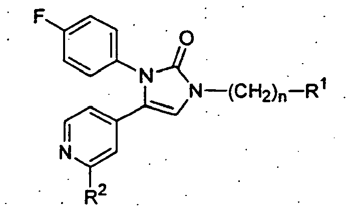 | | | | |

| Example | R¹ | n | R² | MS ([M+H]⁺) |
|---|---|---|---|---|
| 181 | 4-Hydroxyphenyl | 1 | Isopropylamino | 419 |
| 182 | Hydrogen atom | 0 | Isopropylamino | 313 |
| 183 | Hydrogen atom | 0 | Isobutylamino | 327 |

### Examples 184 and 185

By using the compound of Example 70 or the compound of Example 105, it was reacted under the same conditions (conc. hydrochloric acid was used in place of HBr-acetic acid) as in Example 179 to give the compounds shown in Table 21.

**Table 21**

| | | |
|---|---|---|
| 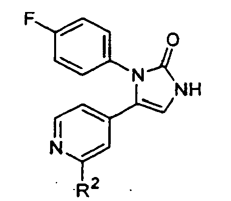 | | |

| Example | R² | MS ([M+H]⁺) |
|---|---|---|
| 184 | trans-4-Hydroxycyclohexylamino | 369 |
| 185 | trans-4-Aminocyclohexylamino | 368 |

### Examples 186 to 197

The compound of Reference example 13 was subjected to amination in the same manner as in Example 4, and then, reacted with a corresponding isocyanate in the same manner as in Example 1, and, if necessary, subjected to acetylation according to the conventional manner to give the compounds shown in Table 22.

**Table 22**

| | | | |
|---|---|---|---|
| 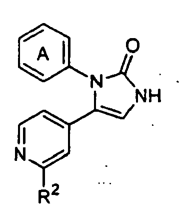 | | | |

| Example | Ring A | R² | MS ([M+H]⁺) |
|---|---|---|---|
| 186 | 3-Fluorophenyl | Isobutylamino | 327 |
| 187 | 3-Fluorophenyl | Isopropylamino | 313 |
| 188 | 2, 4-Difluorophenyl | Isopropylamino | 331 |
| 189 | 2-Fluorophenyl | Isopropylamino | 313 |
| 190 | 2,4-Difluorophenyl | Isobutylamino | 345 |
| 191 | 3-Methoxyphenyl | Isopropylamino | 325 |
| 192 | Phenyl | Isopropylamino | 295 |
| 193 | 2-Fluorophenyl | trans-4-Acetoxycyclohexylamino | 411 |
| 194 | 3-Fluorophenyl | trans-4-Acetoxycyclohexylamino | 411 |
| 195 | 2,4-Difluorophenyl | trans-4-Acetoxycyclohexylamino | 429 |
| 196 | Phenyl | trans-4-Acetoxycyclohexylamino | 393 |
| 197 | 3-Methoxyphenyl | trans-4-Acetoxycyclohexylamino | 423 |

### Example 198

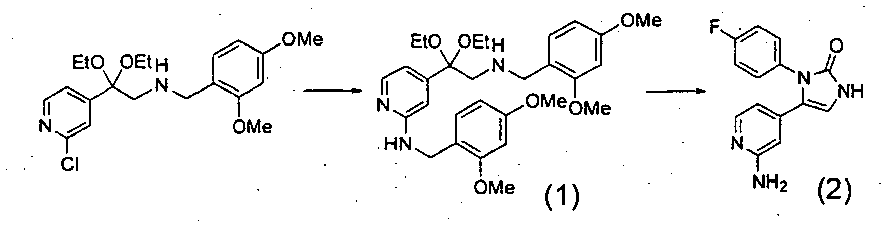

The compound (6.30 g) of Reference example 13 was reacted with 2, 4-dimethoxybenzylamine in the same manner as in Example 4 to give Compound (1). Then, Compound (1) was treated in the same manner as in Example 1 to give 744 mg of Compound (2).
MS 271 ([M+H]⁺)

### Examples 199 to 221

The compound of Example 182, 192, 189, 187 or 188 was reacted with a corresponding halide in the same manner as in Reference example 1(6) to subject to alkylation to give the compounds shown in Tables 23 and 24. Incidentally, the compound of Example 211 was synthesized by protecting the amino group with a t-butoxycarbonyl for the reaction and deprotecting in the same manner as in Example 80. Also, the compound of Example 214 was synthesized by eliminating a methoxymethyl group of the compound of Example 213 in the same manner as in Example 81.

**Table 23**

| | | | |
|---|---|---|---|
| 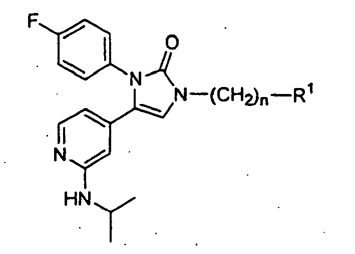 | | | |

| Example | n | R¹ | MS ([M+H]⁺) |
|---|---|---|---|
| 199* | 0 | Methyl | 327 |
| 200 | 0 | 3-hydroxypropyl | 371 |
| 201 | 0 | Butyl | 369 |
| 202* | 0 | 2-Methoxyethyl | 371 |
| 203* | 0 | Carbamoylmethyl | 370 |
| 204 | 0 | Ethyl | 341 |
| 205* | 0 | Isopropyl | 355 |
| 206* | 1 | Cyclobutyl | 381 |
| 207* | 0 | Isobutyl | 369 |
| 208* | 0 | Cyanomethyl | 352 |
| 209* | 0 | Isopentyl | 383 |
| 210* | 1 | Cyclopropyl | 367 |
| 211** | 0 | 3-Aminopropyl | 370 |
| 212* | 0 | Propyl | 355 |
| 213 | 0 | 2-Methoxymethoxyethyl | 401 |
| 214* | 0 | 2-Hydroxyethyl | 357 |
| 215* | 0 | 1-Carbamoylethyl | 384 |

| | | | |
|---|---|---|---|
| * : Monohydrochloride ; ** : Dihydrochloride | | | |

**Table 24**

| | | | |
|---|---|---|---|
| 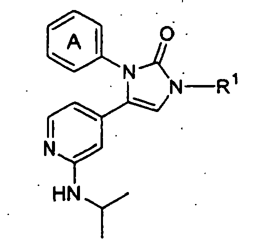 | | | |

| Example | Ring A | R¹ | MS ([M+H]⁺) |
|---|---|---|---|
| 216 | Phenyl | Ethyl | 323 |
| 217 | 2-Fluorophenyl | Ethyl | 341 |
| 218 | 3-Fluorophenyl | Ethyl | 341 |
| 219* | 2,4-Difluorophenyl | Ethyl | 359 |
| 220 | Phenyl | Methoxymethyl | 339 |
| 221 | 2,4-Difluorophenyl | Methoxymethyl | 375 |

| | | | |
|---|---|---|---|
| * : Monohydrochloride | | | |

### Examples 222 to 225

The corresponding starting materials obtained in the same manner as in Example 192 were reacted with a corresponding halide in the same manner as in Reference example 1 (6) to subject to alkylation to give the compounds shown in Table 25.

**Table 25**

| | | | |
|---|---|---|---|
| 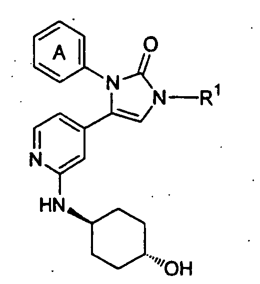 | | | |

| Example | Ring A | R¹ | MS ([M+H]⁺) |
|---|---|---|---|
| 222 | 3-Fluorophenyl | Ethyl | 397 |
| 223 | 2,4-Difluorophenyl | Ethyl | 415 |
| 224 | 3-Methoxyphenyl | Ethyl | 409 |
| 225 | 2,4-Difluorophenyl | Methoxymethyl | 431 |

### Example 226

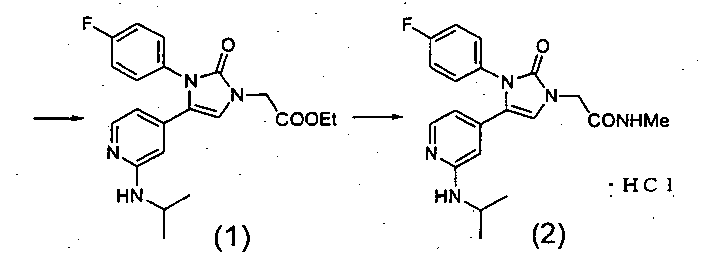

The compound of Example 182 was reacted with a corresponding halide in the same manner as in Reference example 1 (6) to subject to alkylation to synthesize Compound (1). A mixture comprising 226 mg of Compound (1), 1.1 ml of 1N aqueous NaOH solution and 1.1 ml of ethanol was stirred at room temperature for 3 hours. The resulting mixture was neutralized with 1N hydrochloric acid, and precipitated crystals were collected by filtration to give 184 mg of the corresponding carboxylic acid. 148 mg of the obtained crystals was reacted with methylamine in the same manner as in Example 170 to give 96 mg of Compound (2). MS 384 ([M+H]⁺)

### Example 227

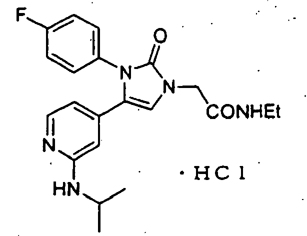

The compound of Example 226(1) was reacted with ethylamine in the same manner as in Example 226(2) to give the title compound.
MS 398 ([M+H]⁺)

### Examples 228 and 229

The compound of Reference example 1 (5) was reacted with a corresponding compound in the same manner as in Reference example 1 (6), subsequently the resulting compound was treated in the same manner as in Examples 5 and 13 to give the compounds shown in Table 26. Incidentally, the compound of Example 229 was synthesized by using 2,4-dimethoxybenzyl in place of 4-methoxybenzyl, and deprotecting with conc. hydrochloric acid/THF (70°C).

**Table 26**

| | | | |
|---|---|---|---|
| 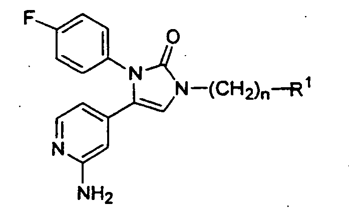 | | | |

| Example | n | R¹ | MS ([M+H]⁺) |
|---|---|---|---|
| 228 | 1 | 2-Fluorophenyl | 379 |
| 229 | 0 | Isopropyl | 313 |

| | | | |
|---|---|---|---|
| * : Monohydrochloride | | | |

### Example 230

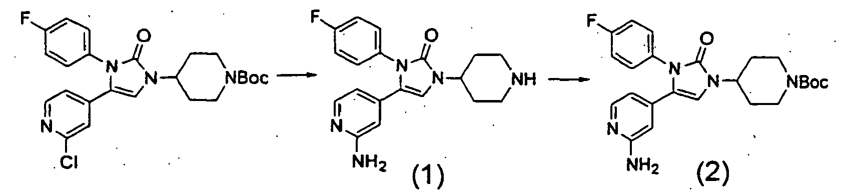

The compound (1.5 g) of Reference example 9 was reacted with 2, 4-dimethoxybenzylamine and deprotected in the same manner as in Example 229 to give 707 mg of Compound (1). This compound (1) (707 mg) was dissolved in 7 ml of THF, and.410 mg of Boc₂O was added and the resulting mixture was stirred at room temperature for 30 minutes. After concentration under reduced pressure, diethyl ether was added to the mixture and precipitates were collected by filtration to give 770 mg of Compound (2) as colorless crystals.
MS 454 [M+H]⁺)

### Examples 231 to 242

By using the compounds of Example 13 and Examples 228 to 230, they were reacted with an acid halide in the same manner as in Example 14, and if necessary, by removing t-butoxycarbonyl in the same manner as in Example 80 to give the compounds shown in Table 27.

**Table 27**

| | | | | |
|---|---|---|---|---|
| 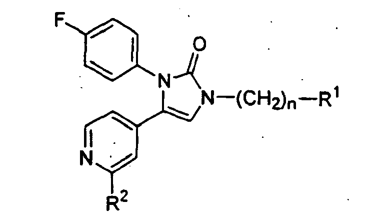 | | | | |

| Example | n | R¹ | R² | MS ([M+H]⁺) |
|---|---|---|---|---|
| 231 | 1 | 2-Cyanophenyl | Acetylamino | 428 |
| 232 | 1 | 2-Cyanophenyl | 2-Pyridylcarbonylamin O | 491 |
| 233 | 1 | 2-Fluorophenyl | Acetylamino | 421 |
| 234 | 1 | 2-Fluorophenyl | Propionylamino | 435 |
| 235 | 1 | 2-Fluorophenyl | Isobutyrylamino | 449 |
| 236 | 1 | 2-Fluorophenyl | Methoxycarbonylacetyl amino | 479 |
| 237 | 1 | 2-Fluorophenyl | 3-Methoxypropionylamino | 465 |
| 238 | 1 | 2-Fluorophenyl | Cyclopropylcarbonylamino | 447 |
| 239* | 0 | Isopropyl- | Cyclopropylcarbonylamino | 381 |
| 240* | 0 | Isopropyl | Cyclopentylcarbonylamino | 409 |
| 241** | 0 | 4-Piperidyl | Isobutyrylamino | 424 |
| 242** | 0 | 4-Piperidyl | Cyclopropylcarbonylamino | 422 |

| | | | | |
|---|---|---|---|---|
| * : Monohydrochloride ; ** : Dihydrochloride | | | | |

### Example 243

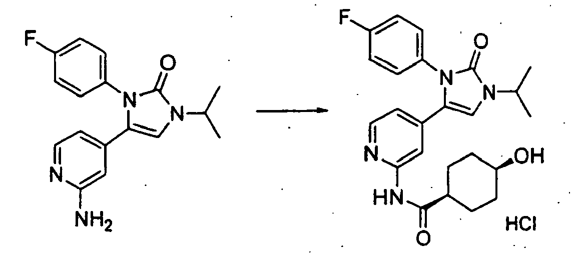

In 45 ml of acetonitrile was dissolved 4.5 g of cis-4-(methoxy-methoxy) cyclohexane carboxylic acid, 3.73 g of 1,1-carbonyldiimidazole was added to the solution, and the mixture was stirred at room temperature for one hour. To the mixture were added 4.07 g.of the compound of Example 229 and 45 ml of acetonitrile, and the resulting mixture was refluxed under heating for 4 days. Water and an aqueous sodium bicarbonate solution were added to the reaction mixture, and the mixture was extracted with ethyl acetate. The extract was washed with brine and dried over anhydrous magnesium sulfate. After concentration under reduced pressure, 50 ml of methanol was added to the residue and the mixture was stirred for 30 minutes. The reaction mixture was concentrated under reduced pressure, and the obtained residue was purified by silica gel column chromatography to give an amide compound. This compound was treated in the same manner as in Example 81 to obtain 5.26 g of the title compound.
MS 439 ([M+H]⁺)

### Examples 244 to 263

By using the compounds of Examples 228 to 230, they were reacted with a corresponding carboxylic acid in the same manner as in Example 243, and if necessary, by removing t-butoxycarbonyl in the same manner as in Example 80 to give the compounds shown in Tables 28 and 29.

**Table 28**

| | | |
|---|---|---|
| 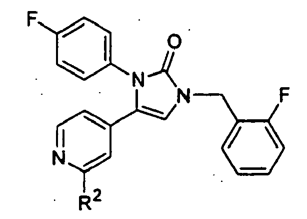 | | |

| Example | R² | MS ([M+H]⁺) |
|---|---|---|
| 244 | (Acetylamino)acetylamino | 478 |
| 245** | (S)-2-Amino-propionylamino | 450 |
| 246** | (S)-2-Methylamino-propionylamino | 464 |
| 247** | (S)-2-Amino-3-methoxy-propionylamino | 480 |
| 248** | 3-Amino-propionylamino | 450 |
| 249** | (S)-2-Pyrrolidinylcarbonylamino | 476 |
| 250** | cis-4-Amino-cyclohexylcarbonylamino | 504 |
| 251** | 4-Piperidylcarbonylamino | 490 |
| 252 | 3-Acetylamino-propionylamino | 492 |
| 253 | (1-Acetyl-4-piperidyl)carbonylamino | 532 |

| | | |
|---|---|---|
| ** : Dihydrochloride | | |

**Table 29**

| | | | | |
|---|---|---|---|---|
| 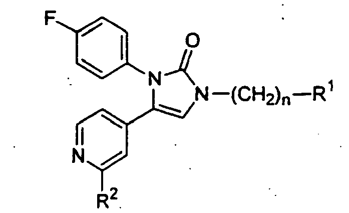 | | | | |

| Example | n | R¹ | R² | MS ([M+H]⁺) |
|---|---|---|---|---|
| 254 | 1 | 2-Fluorophenyl | (S)-5-Oxopyrrolidin-2-ylcarbonylamino | 490 |
| 255* | 1 | 2-Fluorophenyl | cis-4-Hydroxy-cyclohexylcarbonylamino | 505 |
| 256 | 1 | 2-Fluorophenyl | cis-4-Acetylaminocyclohexylcarbonylamino | 546 |
| 257 | 1 | 2-Fluorophenyl | (S)-1-Acetylpyrrolidin-2-ylcarbonylamino | 518 |
| 258** | 1 | 2-Fluorophenyl | trans-4-Amino-cyclohexylcarbonylamino | 504 |
| 259* | 1 | 2-Fluorophenyl-2-Fluorophenyl | trans-4-Hydroxy-cyclohexylcarbonylamino | 505 |
| 260* | 0 | Isopropyl | (S)-5-Oxopyrrolidin-2-ylcarbonylamino | 424 |
| 261** | 0 | Isopropyl | cis-4-Amino-cyclohexylcarbonylamino | 438 |
| 262** | 0 | 4-Piperidyl | trans-4-Hydroxy-cyclohexylcarbonylamino | 480 |
| 263** | 0 | 4-Piperidyl | cis-4-Hydroxy-cyclo-hexylcarbonylamino | 480 |

| | | | | |
|---|---|---|---|---|
| * : Monohydrochloride ; ** : Dihydrochloride | | | | |

### Examples 264 to 267

By using the compounds of Reference examples 14 and 15, they were reacted with a corresponding isocyanate in the same manner as in Example 1, subsequently, the resulting compounds were reacted with a corresponding carboxylic acid in the same manner as in Example 243 to give the compounds shown in Table 30.

**Table 30**

| | | | |
|---|---|---|---|
| 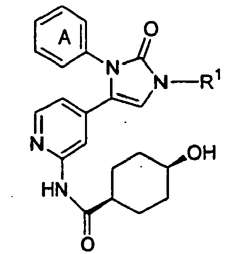 | | | |

| Example | Ring A | R¹ | MS ([M+H]⁺) |
|---|---|---|---|
| 264* | 3-Chlorophenyl | Isopropyl | 455 |
| 265* | 3-Methylphenyl | Isopropyl | 435 |
| 266* | 3-Chlorophenyl | ethyl | 441 |
| 267* | 3-Methylphenyl | ethyl | 421 |

| | | | |
|---|---|---|---|
| * : Monohydrochloride | | | |

### Example 268

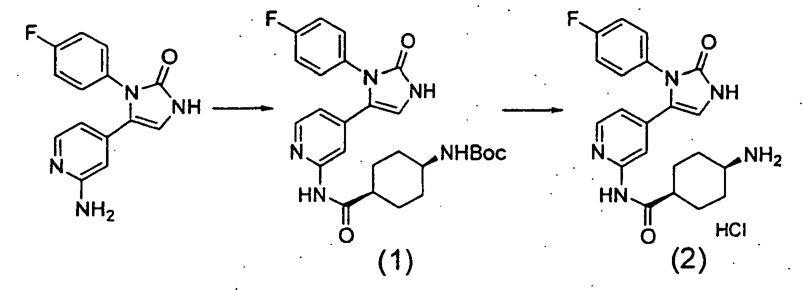

In 5 ml of acetonitrile were dissolved 540 mg of cis-4-(t-butoxycarbonyl (amino) cyclohexane carboxylic acid and 396 mg of 1,1'-carbonyldiimidazole, and the mixture was stirred at room temperature for an hour. Then, to the reaction mixture were added 200 mg of the compound of Example 198 and 5 ml of acetonitrile, and the mixture was refluxed under heating for 2 days. To the reaction mixture was added an aqueous sodium bicarbonate solution, and the mixture was extracted with chloroform. The extract was washed with brine and dried over anhydrous magnesium sulfate. After concentration under reduced pressure, the residue was dissolved in 5 ml of methanol; and 102 mg of potassium carbonate was added to the mixture. The resulting mixture was diluted with chloroform, washed with brine and dried over anhydrous magnesium sulfate. After concentration under reduced pressure, the residue was purified by silica gel column chromatography to give 255 mg of Compound (1) as colorless powder.
MS 496 ([M+H]⁺)

Compound (1) (50 mg) was dissolved in a mixed solvent of methanol and chloroform, 0.5 ml of 4N hydrochloric acid-ethyl acetate solution was added to the mixture, and the resulting mixture was stirred at room temperature overnight. The reaction mixture was concentrated under reduced pressure to give 46 mg of Compound (2) as yellowish powder.
MS 396 ([M+H]⁺)

### Example 269

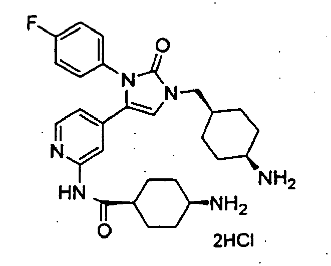

Compound (1) (100 mg) of Example 268 was dissolved in 5 ml of methylene chloride, and to the mixture were added 132 mg of diethylazodicarboxylate (40% solution in toluene), 79 mg of triphenylphosphine and 55 mg of t-butyl (4-hydroxymethylcyclohexyl) carbamate, and the resulting mixture was stirred at room temperature for 21 hours. The reaction mixture was concentrated under reduced pressure, the obtained residue was purified by silica gel column chromatography, and dissolved in 1 ml of methanol. 1 ml of 4N Hydrochloric acid-dioxane was added to the mixture, and the resulting mixture was stirred at room temperature for an hour. The reaction mixture was concentrated to give 118 mg of the title compound as yellowish powder.
MS 507 ([M+H]⁺)

### Example 270

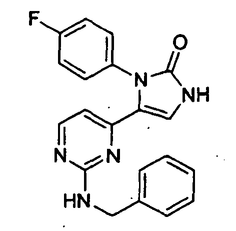

The compound of Reference example 7(1) was reacted with benzylamine in the same manner as.in Example 17 to give the title compound.
MS 362 ([M+H]⁺)

### Examples 271 to 336

The compound of Reference example 5 (4) was reacted in the same manner as in Example 2 or Reference example 1(6), oxidized with 3-chloroperoxybenzoic acid in the same manner as in Reference example 6(2), subsequently reacted with a corresponding amine in the same manner as in Example 17, and further, if necessary, t-butoxycarbonyl or methoxymethyl is removed in the same manner as in Example 80 or 81 to give the compounds shown in Tables 31 to 35.

**Table 31**

| | | |
|---|---|---|
| 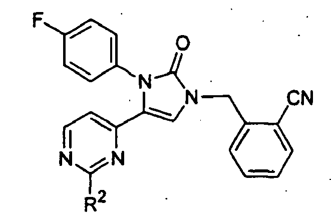 | | |

| Example | R² | MS ([M+H]⁺) |
|---|---|---|
| 271 | Benzylamino | 477 |
| 272 | 2-Methoxyethylamino | 445 |
| 273 | Cyclopropylamino | 427 |
| 274 | Butylamino | 443 |
| 275 | Isopropylamino | 429 |
| 276 | Ethylamino | 415 |
| 277 | Cyclopropylmethylamino | 441 |
| 278 | trans-4-Hydroxycyclohexylamino | 485 |
| 279 | (S)-1-Hydroxymethyl-ethylamino | 445 |
| 280 | (S)-1-Hydroxymethyl-propylamino | 459 |

**Table 32**

| | | |
|---|---|---|
| 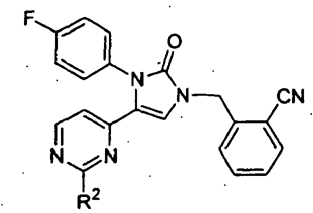 | | |

| Example | R² | MS ([M+H]⁺) |
|---|---|---|
| 281 | (S)-1-Hydroxymethyl-2-methylpropylamino | 473 |
| 282 | (R)-1-Hydroxymethyl-ethylamino | 445 |
| 283* | 1-Methyl-4-piperidylamino | 484 |
| 284 | 1-Benzyl-4-piperidylamino | 560 |
| 285 | 1-Ethoxycarbonyl-4-piperidylamino | 542 |
| 286 | 1-Hydroxymethyl-cyclopentylamino | 485 |
| 287 | 1-t-Butoxycarbonyl-4-piperidylamino | 570 |
| 288** | 4-Piperidylamino | 470 |
| 289 | 4-Methoxybenzylamino | 507 |
| 290** | trans-4-Aminocyclohexylamino | 484 |

| | | |
|---|---|---|
| * :Monohydrochloride ; **:Dihydrochloride | | |

**Table 33**

| | | | | |
|---|---|---|---|---|
| 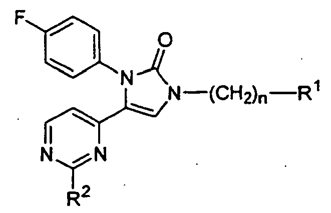 | | | | |

| Example | n | R¹ | R² | MS ([M+H]⁺) |
|---|---|---|---|---|
| 291 | 1 | 2-Fluorophenyl | trans-4-Hydroxycyclohexylamino | 478 |
| 292 | 1 | 2-Methoxyphenyl | trans-4-Hydroxycyclohexylamino | 490 |
| 293** | 1 | 4-Pieridyl 4-Piperidyl | trans-4-Hydroxytrans-4-Hydroxycyclohexylamino | 467 |
| 294** | 1 | 4-Piperidyl | Isopropylamino | 411 |
| 295 | 1 | 2-Fluorophenyl | Isobutylamino | 436 |
| 296** | 1 | 4-Piperidyl | Isobutylamino | 425 |
| 297** | 1 | 2-Fluorophenyl | 4-Piperidylamino | 463 |
| 298* | 0 | Methyl | trans-4-Hydroxycyclohexylamino | 384 |
| 299** | 0 | Methyl | trans-4-Aminocyclohexylamino | 383 |
| 300* | 0 | Ethyl | trans-4-Hydroxycyclohexylamino | 398 |
| 301* | 0 | Ethyl | Isobutylamino | 356 |
| 302* | 0 | Isopropyl | trans-4-Hydroxycyclohexylamino | 412 |
| 303** | 0 | Isopropyl | trans-4-Aminocyclo hexylamino | 411 |
| 304** | 0 | ethyl | trans-4-Aminocyclo hexylamino | 397 |
| 305* | 1 | cis-4-Hydroxycyclohexyl | Isopropylamino | 426 |

| | | | | |
|---|---|---|---|---|
| *:Monohydrochloride;**:Dihydrochloride | | | | |

**Table 34**

| | | | | |
|---|---|---|---|---|
| 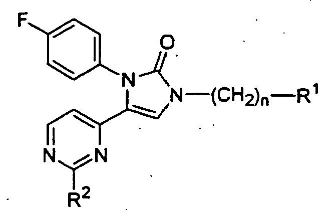 | | | | |

| Example | n | R¹ | R² | MS ([M+H]⁺) |
|---|---|---|---|---|
| 306* | 1 | cis-4-Hydroxycyc lohexyl | Isobutylamino | 440 |
| 307* | 1 | trans-4-Hydroxyc yclohexyl | Isopropylamino | 426 |
| 308* | 1 | trans-4-Hydroxyc yclohexyl | Isobutylamino | 440 |
| 309** | 1 | cis-4-Aminocyclo hexyl | Isopropylamino | 425 |
| 310** | 1 | cis-4-Aminocyclo hexyl | Isobutylamino | 439 |
| 311** | 1 | cis-4-Aminocyclo hexyl | trans-4-Hydroxycyclohexylamino | 481 |
| 312* | 0 | Ethyl | trans-4-acetylaminocyclohexylamino | 439 |
| 313* | 0 | Isopropyl | trans-4-acetylaminocyclohexylamino | 453 |
| 314*** | 1 | cis-4-Aminocyclo hexyl | trans-4-Aminocyclohexylamino | 480 |
| 315** | 1 | trans-4-Aminocyc lohexyl | Isopropylamino | 425 |
| 316** | 1 | trans-4-Aminocyc lohexyl | Isobutylamino | 439 |
| 317** | 1 | trans-4-Aminocyc lohexyl | trans-4-Hydroxycyclohexylamino | 481 |
| 318*** | 1 | trans-4-Aminocyc lohexyl | trans-4-Aminocyclohexylamino | 480 |
| 319* | 1 | cis-4-Hydroxycyc lohexyl | trans-4-Hydroxycyclohexylamino | 482 |
| 3 20* | 0 | Isobutyl | trans-4-Hydroxycyclohexylamino | 426 |

| | | | | |
|---|---|---|---|---|
| *:Monohydrochloride; **:Dihydrochloride; ***:Trihydrochloride | | | | |

**Table 35**

| | | | | |
|---|---|---|---|---|
| 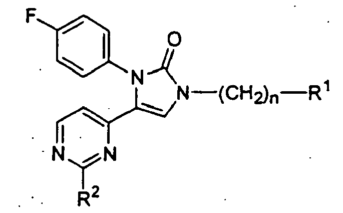 | | | | |

| Example | n | R¹ | R² | MS ([M+H]⁺) |
|---|---|---|---|---|
| 321* | 0 | propyl | trans-4-Hydroxycyclohexylamino | 412 |
| 322* | 0 | butyl | trans-4-Hydroxycyclohexylamino | 426 |
| 323* | 0 | Cyanomethyl | trans-4-Hydroxycyclohexylamino | 409 |
| 324* | 0 | 2-Methoxyethyl | trans-4-Hydroxycyclohexylamino | 428 |
| 325* | 0 | 3-hydroxypropyl | trans-4-Hydroxycyclohexylamino | 428 |
| 326* | 1 | Cyclopropyl | trans-4-Hydroxycyclohexylamino | 424 |
| 327* | 1 | Cyclobutyl | trans-4-Hydroxycyclohexylamino | 438 |
| 328* | 0 | Ethyl | 4-Tetrahydropyranylamino | 384 |
| 329* | 0 | Ethyl | (S)-1-Hydroxymethylethylamino | 358 |
| 330* | 0 | Ethyl | 2-Hydroxy-1,1-dimethylethylamino | 372 |
| 331* | 0 | Ethyl | 1-Hydroxymethyl-cyclo pentylamino | 398 |
| 332* | 0 | Ethyl | 3-Methoxypropylamino | 372 |
| 333 | 0 | Isopropyl | 2-Hydroxy-1,1-dimethy lethylamino | 386 |
| 334 | 0 | Isopropyl | 1-Hydroxymethyl-cyclo pentylamino | 412 |
| 335 | 0 | Ethyl | cis-4-Hydroxycyclohex ylamino | 398 |
| 336 | 0 | Isopropyl | cis-4-Hydroxycyclohex ylamino | 412 |

| | | | | |
|---|---|---|---|---|
| *:Monohydrochloride | | | | |

### Examples 337 to 343

The compound of Reference example 16 was reacted with a corresponding isocyanate in the same manner as in Example 1, oxidized with 3-chloroperoxybenzoic acid in the same manner as in Reference example 6(2), subsequently reacted with a corresponding amine in the same manner as in Example 17 to give the compounds shown in Table 36.

**Table 36**

| Example | Ring A | MS ([M+H]⁺) |
|---|---|---|
| 337* | 3-Fluorophenyl | 412 |
| 338* | 3-Methylphenyl | 408 |
| 339* | Phenyl | 394 |
| 340* | 3-Chlorophenyl | 428 |
| 341* | 4-Chlorophenyl | 428 |
| 342* | 2,4-Difluorophenyl | 430 |
| 343* | 3-Methoxyphenyl | 424 |

| | | |
|---|---|---|
| *:Monohydrochloride | | |

### Examples 344 to 349

The compound of Reference example 17(3) was reacted with a corresponding isocyanate in the same manner as in Example 1 to give the compounds shown in Table 37.

**Table 37**

| | | |
|---|---|---|
| 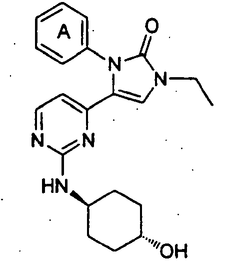 | | |

| Example | Ring A | MS ([M+H]⁺) |
|---|---|---|
| 344* | 3-Chlorophenyl | 414 |
| 345* | 3-Methylphenyl | 394 |
| 346* | 3-Trifluoromethylphenyl | 448 |
| 347* | 4-Chlorophenyl | 414 |
| 348* | Phenyl | 380 |
| 349* | 3-Fluorophenyl | 398 |

| | | |
|---|---|---|
| *:Monohydrochloride | | |

### Example 350

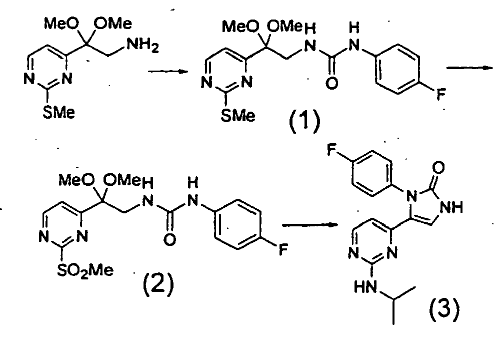

(1) To 300 ml of a diethyl ether solution containing 52.0 g of the compound of Reference example 5(3) was added dropwise 100 ml of a diethyl ether solution containing 30.2 g of 4-fluorophenyl isocyanate under ice-cooling, and the mixture was stirred at room temperature for 30 minutes. After concentration under reduced pressure, diisopropyl ether was added to the reaction mixture and crystals were collected by filtration to give 75.0 g of Compound (1) as colorless crystals.
(2) In chloroform was dissolved 30.0 g of Compound (1), and under ice-cooling, 46.4 g of 3-chloroperoxybenzoic acid was added to the solution and the mixture was stirred at room temperature for 2 hours. After concentration under reduced pressure, diethyl ether was added to the reaction mixture and crystals were collected by filtration to give 30.8 g of Compound (2) as colorless crystals.
(3) To the compound obtained by treating 20.0 g of Compound (2) with a corresponding starting material in the same manner as in Example 17 was added 100 ml of conc. hydrochloric acid, and the mixture was stirred at room temperature overnight. A 2N aqueous sodium hydroxide solution was added to the mixture to neutralize the same, ethyl acetate was added to the same and after stirring, precipitated crystals were collected by filtration to give 12.4 g of the title compound as colorless crystals.
   MS 314 ([M+H]⁺)

### Examples 351 to 354

The compound of Reference example 5(3) and a corresponding starting material were treated in the same manner as in Example 350 to give the compounds shown in Table 38.

**Table 38**

| | | | |
|---|---|---|---|
| 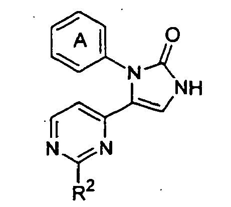 | | | |

| Example | Ring A | R² | MS ([M+H]⁺) |
|---|---|---|---|
| 351 | 4-Fluorophenyl | trans-4-Hydroxycyclohexylamino | 370 |
| 352* | 4-Fluorophenyl | Isobutylamino | 328 |
| 353 | 2,4-Difluorophenyl | Isopropylamino | 332 |
| 354 | Phenyl | Isopropylamino | 296 |

| | | | |
|---|---|---|---|
| *:Monohydrochloride | | | |

### Examples 355 to 367

By using the compound of Example 350, 353 or 354, or the compound produced by the same manner as in Example 350, they were treated in the same manner as in Reference example 1(6) to give the compounds shown in Table 39.

**Table 39**

| | | | |
|---|---|---|---|
| 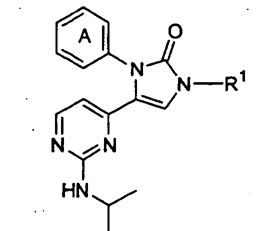 | | | |

| Example | Ring A | R¹ | MS ([M+H]⁺) |
|---|---|---|---|
| 355* | 4-Fluorophenyl | Methyl | 328 |
| 356* | 4-Fluorophenyl | Ethyl | 342 |
| 357* | 4-Fluorophenyl | Methoxymethyl | 358 |
| 358 | 2,4-Difluorophenyl | Ethyl | 360 |
| 359 | Phenyl | Ethyl | 324 |
| 360 | 4-Chlorophenyl | Ethyl | 358 |
| 361 | 3-Fluorophenyl | Ethyl | 342 |
| 362 | 3-Methoxyphenyl | Ethyl | 354 |
| 363 | 2,4-Difluorophenyl | Methoxymethyl | 376 |
| 364 | Phenyl | Methoxymethyl | 340 |
| 365 | 4-Chlorophenyl | Methoxymethyl | 374 |
| 366* | 4-Fluorophenyl | 2-Methoxyethyl | 372 |
| 367* | 4-Fluorophenyl | Cyanomethyl | 353 |

| | | | |
|---|---|---|---|
| *:Monohydrochloride | | | |

### Examples 368 to 382

The compound of Reference example 5(4) was reacted in the same manner as in Example 2 or Reference example 1(6), oxidized with 3-chloroperoxybenzoic acid in the same manner as in Reference example 6 (2) , subsequently reacted with a corresponding amine in the same manner as in Example 17, and if necessary, t-butoxycarbonyl was removed in the same manner as in Example 80 to give the compounds'shown in Table 40.

**Table 40**

| | | | |
|---|---|---|---|
| 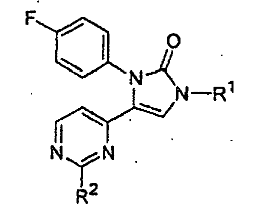 | | | |

| Example | R¹ | R² | MS ([M+H]⁺) |
|---|---|---|---|
| 368* | Ethyl | cis-4-Hydroxymethylcyclohexylamino | 412 |
| 369* | Ethyl | trans-4-Hydroxymethylcyclohexylamino | 412 |
| 370* | Ethyl | 3-Hydroxy-2,2-dimethylpropylamino | 386 |
| 371* | Isopropyl | cis-4-Hydroxymethylcyclohexylamino | 426 |
| 372* | Isopropyl | trans-4-Hydroxymethylcyclohexylamino | 426 |
| 373* | Isopropyl | 3-Hydroxy-2,2-dimethylpropylamino | 400 |
| 374* | Isopropyl | (S)-2-Hydroxypropylamino | 372 |
| 375* | Isopropyl | (R)-2-Hydroxypropylamino | 372 |
| 376* | Isopropyl | 1-Hydroxycyclohexylmethylamino | 426 |
| 377** | Isopropyl | 2-Hydroxy-1-hydroxymethyl-1-methylethylamino | 402 |
| 378** (reference Example) | Isopropyl | 4-Piperidyl | 397 |
| 379** | Isopropyl | (S)-1-(2-Pyridyl)ethylamino | 419 |
| 380* | Isopropyl | (1S,2S)-2-Hydroxycyclopentylamino | 398 |
| 381* | Ethyl | (1S,2S)-2-Hydroxycyclopentylamino | 384 |
| 382* | Ethyl | trans-4-Carbamoylcyclohexylamino | 425 |

| | | | |
|---|---|---|---|
| *:Monohydrochloride ; **:Dihydrochloride | | | |

### Examples 383 to 386

The compound of Example 303 or 304 was subjected to methanesulfonylation or methoxycarbonylation according to the conventional methods to give the compounds shown in Table 41.

**Table 41**

| | | | |
|---|---|---|---|
| 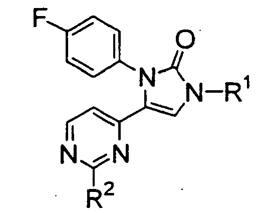 | | | |

| Examples | R¹ | R² | MS ([M+H]⁺) |
|---|---|---|---|
| 383* | Isopropyl | trans-4-Methanesulfonylaminocyclohexylamino | 489 |
| 384* | Isopropyl | trans-4-Methoxycarbonylaminocyclohexylamino | 469 |
| 385* | Ethyl | trans-4-Methanesulfonylaminocyclohexylamino | 475 |
| 386* | Ethyl | trans-4-Methoxycarbonylaminocyclohexylamino | 455 |

| | | | |
|---|---|---|---|
| *:Monohydrochloride | | | |

### Example 387

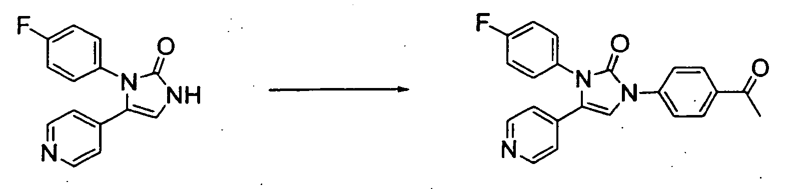

The compound of Example 1 (100 mg), 4-acetylphenylboronic acid (129 mg), copper (II) acetate (72 mg) and triethylamine (220 µl) were suspended in 10 ml of methylene chloride, and the suspension was stirred at room temperature for 24 hours. To the readction mixture, 28% aqueous ammonia was added and the mixture was extracted with chloroform, washed with brine, and dried over anhydrous magnesium sulfate. The resultant mixture was concentrated under reduced pressure, and ether was added to the residue and precipitated crystals were collected by filtration to give 92 mg of the title compound. Melting point: 206°C (decomposed)

### Examples 388 to 389

The compound of Example 1 and the corresponding starting materials were reacted in the same manner as in Example 387 to give the compounds shown in Table 42.

**Table 42**

| | | |
|---|---|---|
| 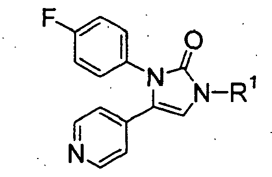 | | |

| Examples | R¹ | Melting point (°C) |
|---|---|---|
| 388 | 4-Pyridyl | 189 |
| 389 | 3-Thienyl | 193-195 |

### Example 390

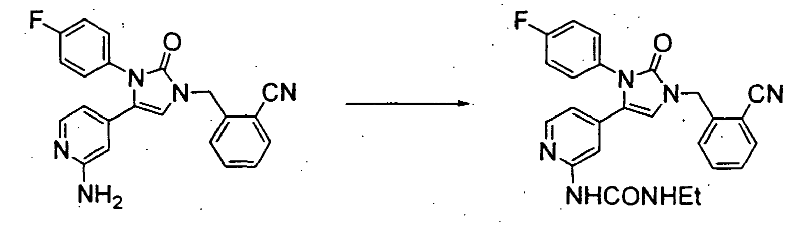

To a solution of the compound of Example 13 (50 mg) in THF was added ethyl isocyanate (12 µl), and the mixture was stirred at room temperature for 6 hours. The reaction mixture was concentrated under reduced pressure and purified by silica gel column chromatography to give 19 mg of the title conpound as colorless crystal. Melting point: 209 - 210°C

### Examples 391 to 394

The compound of Example 16 and the compounds prepared in the same manner as in Example 16 were subjected to hydrolysis followed by amidation according to the conventional methods, or subjected to reduction followed by mesylation and dimethylamination, to give the compounds shown in Table 43.

**Table 43**

| | | | |
|---|---|---|---|
| 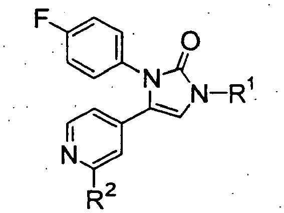 | | | |

| Examples | R¹ | R² | Melting point (°C) |
|---|---|---|---|
| 391 | 2-Cyanobenzyl | Carboxy | 135 (decomposed) |
| 392 | 2-Cyanobenzyl | Carbamoyl | 209-210 (decomposed) |
| 393 | 2-Fluorobenzyl | Hydroxymethyl | 157-158 (decomposed) |
| 394 | 2-Fluorobenzyl | Dimethylaminomethyl | 231-236 (decomposed) |

### Examples 395 to 398

The corresponding starting materials were reacted in the same manner as in Example 368 to give the compounds shown in Table 44.

**Table 44**

| | | | |
|---|---|---|---|
| 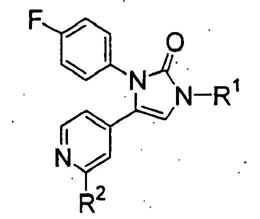 | | | |

| Example | R¹ | R² | MS (M+H]⁺) |
|---|---|---|---|
| 395* | Ethyl | trans-4-Hydroxycyclohexylmethylamino | 412 |
| 396* | Isopropyl | trans-4-Hydroxycyclohexylmethylamino | 426 |
| 397* | Ethyl | cis-4-Hydroxycyclohexylmethylamino | 412 |
| 398* | Isopropyl | cis-4-Hydroxycyclohexylmethylamino | 426 |

| | | | |
|---|---|---|---|
| *:Monohydrochloride | | | |

According to the production methods described in the above Examples and the present specification and methods conventionally employed in the field of organic synthetic chemistry, compounds, which is respectively combined with each of the substitutents shown in Tables 45 to 51, can be produced.

**Table 45**

| | |
|---|---|
| 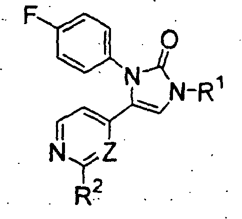 | |
| R¹ = methyl, ethyl, isopropyl, 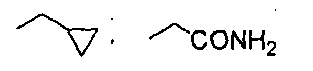 | |
| Z = CH, N | |
| R² = | 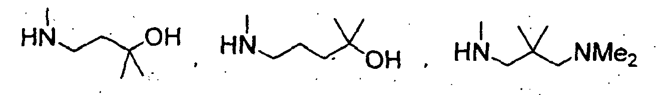 |
| | 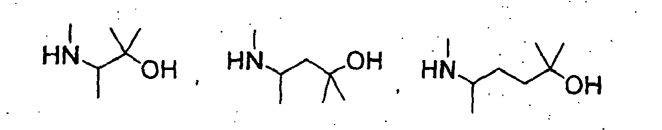 |
| | 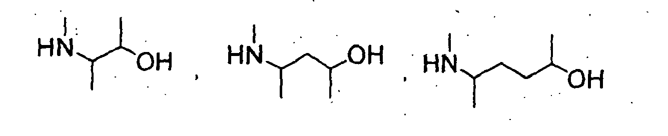 |
| | 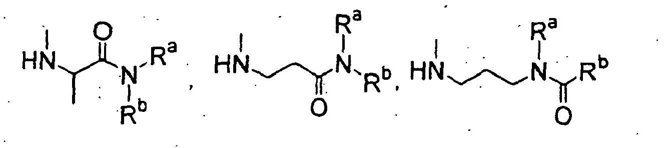 |
| | 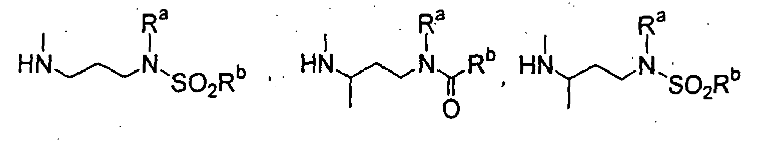 |
| | 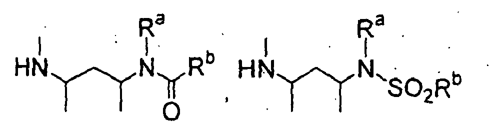 |
| R^{a}, R^{b} = each independently, hydrogen, C₁∼C₃ alkyl | |

**Table 46**

| | |
|---|---|
| 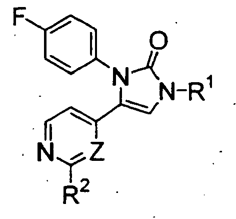 | |
| R¹ = methyl, ethyl, isopropyl, 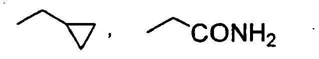 | |
| Z = CH, N | |
| R² = | 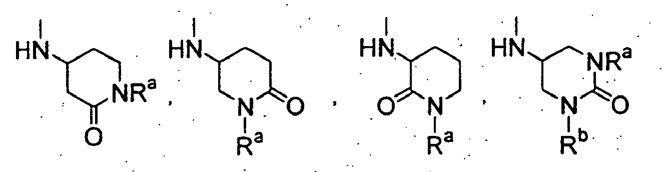 |
| | 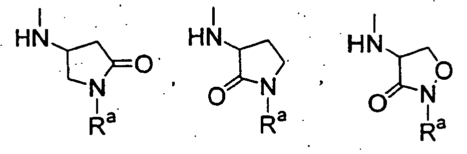 |
| | 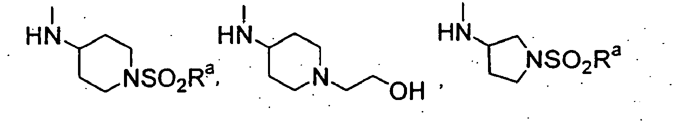 |
| | 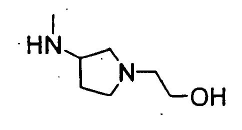 |
| | 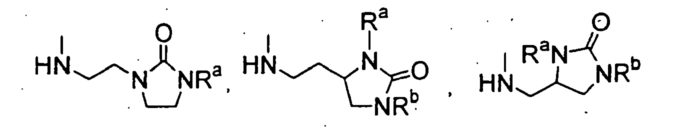 |
| R^{a}, R^{b} = each independently, hydrogen, C₁∼C₃ alkyl | |

**Table 47**

| | |
|---|---|
| 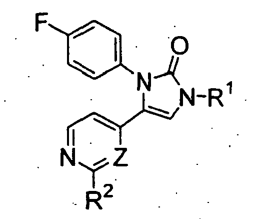 | |
| R¹ = methyl, ethyl, isopropyl, 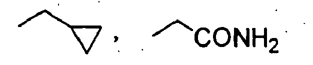 | |
| Z = CH, N | |
| R² = | 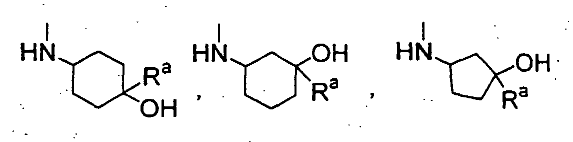 |
| | 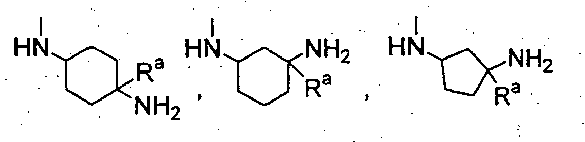 |
| | 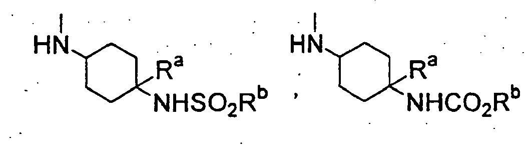 |
| | 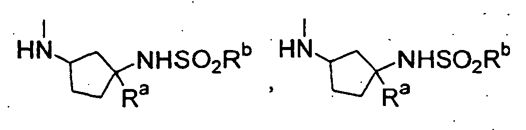 |
| | 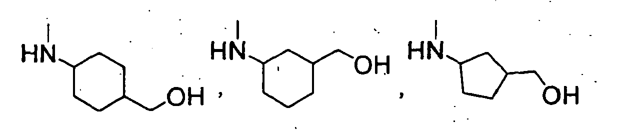 |
| | 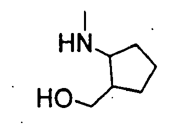 |
| R^{a}, R^{b} = each independently, hydrogen, C₁∼C₃ alkyl | |

**Table 48**

| | |
|---|---|
| 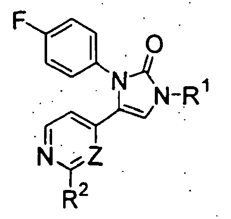 | |
| R¹ = methyl, ethyl, isopropyl, 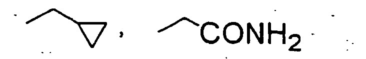 | |
| Z = CH, N | |
| R² = | 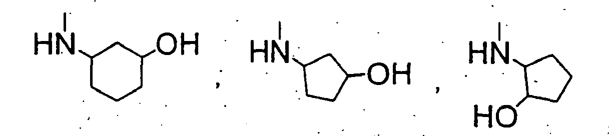 |
| | 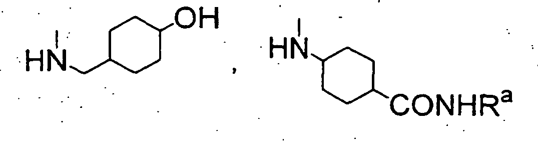 |
| | 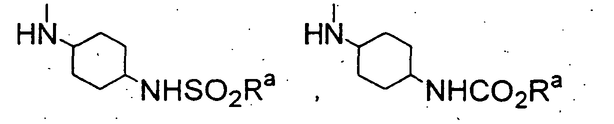 |
| | 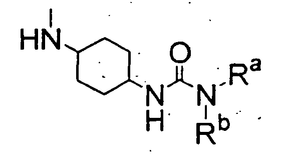 |
| | 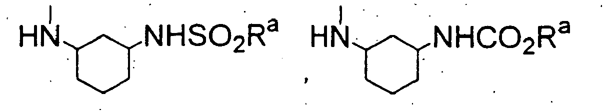 |
| | 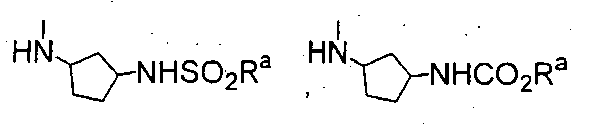 |
| R^{a}, R^{b} = each independently, hydrogen, C₁~C₃ alkyl | |

**Table 49**

| | |
|---|---|
| 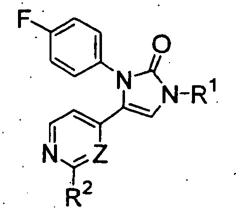 | |
| R¹ = methyl, ethyl, isopropyl, 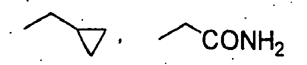 | |
| Z = CH, N | |
| R² = | 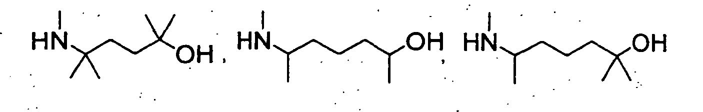 |
| | 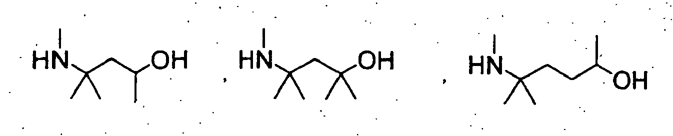 |
| | 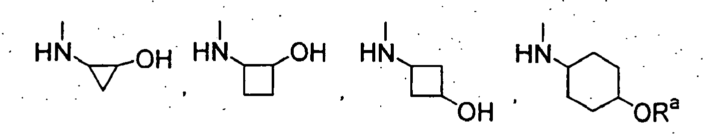 |
| | 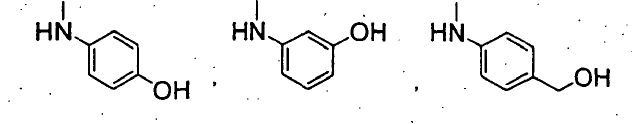 |
| | 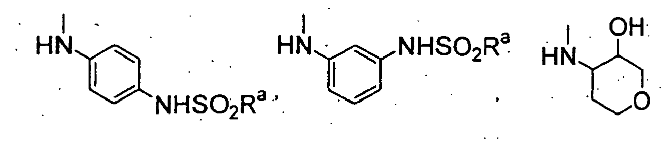 |
| R^{a}, R^{b} = each independently, hydrogen, C₁~C₃ alkyl | |

**Table 50**

| | |
|---|---|
| 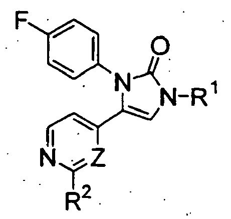 | |
| R¹ = methyl, ethyl, isopropyl, 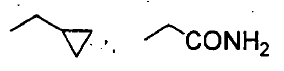 | |
| Z = CH, N | |
| R² = | 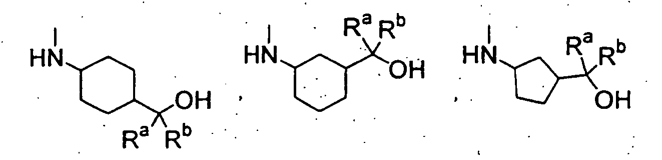 |
| | 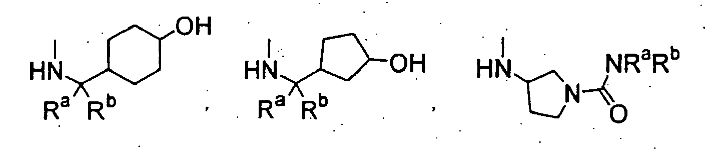 |
| | 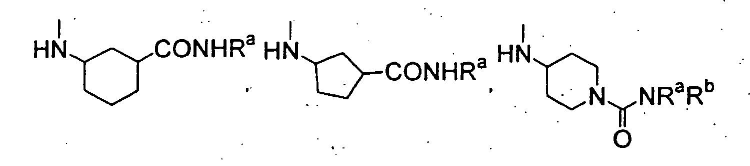 |
| | 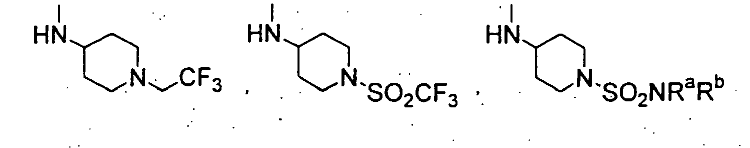 |
| | 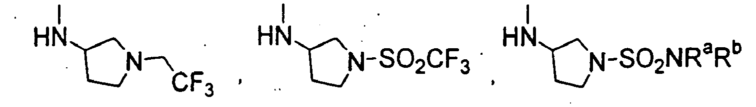 |
| R^{a}, R^{b} = each independently, hydrogen, C₁~C₃ alkyl | |

**Table 51**

| | |
|---|---|
| 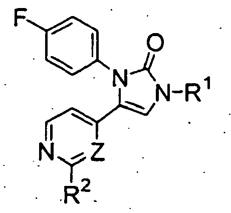 | |
| R¹ = methyl, ethyl, isopropyl, 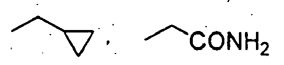 | |
| Z = CH, N | |
| R² = | 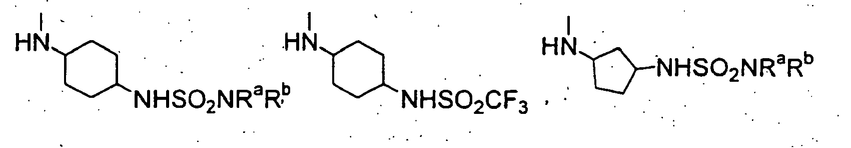 |
| | 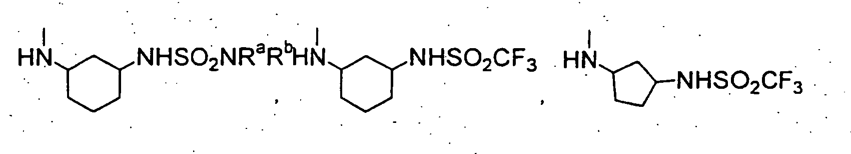 |
| | 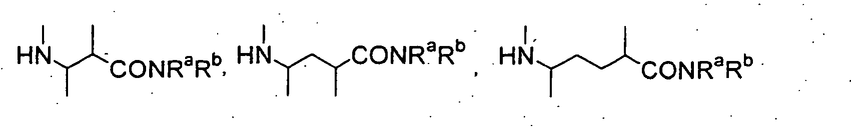 |
| | 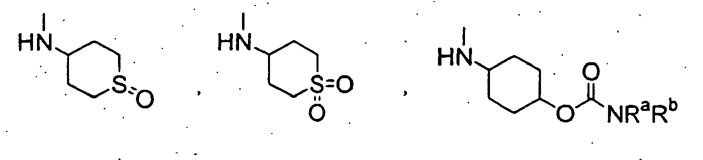 |
| | 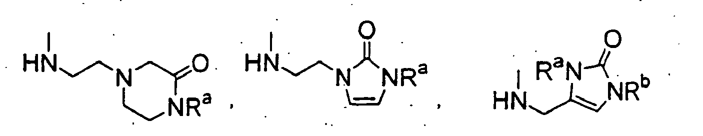 |
| R^{a}, R^{b} = each independently, hydrogen, C₁~C₃ alkyl | |

### Reference example 1

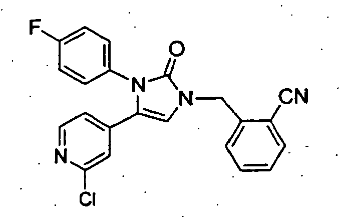

(1) In 440 ml of THF was suspended 22 g of 2-chloroisonicotinic acid, and under nitrogen flow, the mixture was cooled to -70°C or lower, 245 ml of methyl lithium (1.14 M solution in diethyl ether) was added dropwise to the mixture. After stirring at the same temperature for an hour, a temperature of the mixture was raised to 0°C over an hour, and stirred at the same temperature for further an hour. To the reaction mixture was added 500 ml of water, and the reaction mixture was extracted with ethyl acetate, washed with brine and dried over magnesium sulfate. Activated charcoal was added to the mixture, and after filtration, the filtrate was concentrated under reduced pressure to give 19.5 g of 4-acetyl-2-chloropyridine as colorless crystals. Melting point: 36°C.
(2) In 550 ml of ethanol were suspended 55.1 g of the compound obtained in (1), 49.2 g of hydroxylamine hydrochloride and 58.1 g of sodium acetate, and the mixture was refluxed under heating for an hour. After cooling the mixture to room temperature by allowing to stand, ethanol was distilled away under reduced pressure and precipitated crystals were collected by filtration and washed with water. The crystals were air-dried at 60°C overnight to give 55.g of 1-(2-chloropyridin-4-yl)ethanone oxime as colorless crystals. Melting point: 143°C.
(3) In methylene chloride were suspended 105 g of the compound obtained in (2) and 123 g of tosyl chloride, and under ice-cooling, 94 ml of triethylamine was added dropwise to the mixture, and the mixture was raised to room temperature and stirred for 4 hours. To the reaction mixture was added 500 ml of water, and the mixture was extracted with methylene chloride, washed with brine and dried over magnesium sulfate. After filtration, the mixture was concentrated under reduced pressure, and the resulting crystals were collected by filtration and washed with isopropyl ether to give 192 g of 1-(2-chloropyridin-4-yl)ethanone oxime tosylate as colorless crystals Melting point: 153°C.
(4) Under nitrogen flow, 3.11 g of sodium metal was added to 220 ml of anhydrous ethanol at room temperature, and the mixture was dissolved under stirring. The solution was ice-cooled, and 40 g of the compound obtained in (3) was added thereto, then the mixture was stirred at room temperature for an hour. To the mixture was added 220 ml of anhydrous ether, and insoluble matters were removed. To the filtrate was added 62 ml of 4N hydrochloric acid/dioxane solution under ice-cooling and the mixture was stirred for 15 minutes. After the reaction mixture was concentrated under reduced pressure, the residue was dissolved in water and the solution was made alkaline by addition of potassium carbonate. This mixture was extracted with ethyl acetate several times, and the combined extracts were washed with brine and dried over magnesium sulfate. After concentration under reduced pressure, 100 ml of hexane was added to the residue and red insoluble matters were removed by filtration. The filtrate was concentrated under reduced pressure, hexane was again added to the concentrate and insoluble matters were removed by filtration through Celite. The filtrate was concentrated under reduced pressure and dried by a vacuum pump to give 26.9 g of 2-(2-chloropyridin-4-yl)-2,2-diethoxyethylamine as reddish oily product.
(5) A solution, in which 20 g of the compound obtained in (4) was dissolved in 50 ml of. THF, was water-cooled, and 11.2 g of 4-fluorophenylisocyanate was added dropwise thereto. After dropwise addition, the reaction mixture was concentrated under reduced pressure, and 30 ml of conc. hydrochloric acid was added' to the obtained residue and the mixture was stirred at.room temperature overnight. The reaction mixture was added to ice-cooled 180 ml of 2N aqueous NaOH solution to neutralize the mixture, and after collecting the precipitated crystals by filtration, the crystals were washed with water and ether. The crystals were air-dried at 60°C to give 22.3 g of 5-(2-chloropyridin-4-yl)-1-(4-fluorophenyl)-4-imidazolin-2-one as colorless crystals. Melting point: 270°C.
(6) In 50 ml of DMF was suspended 10 g of the compound obtained in (5), and under ice-cooling, 1.46 g of 63% sodium hydride was added to the suspension, then the mixture was stirred at room temperature for 30 minutes. The mixture was again ice-cooled, and after adding 7.44 g of 2-cyanobehzyl bromide, the mixture was stirred at room temperature for 3 hours. The reaction mixture was poured into 2.50 ml of ice-cold water, extracted with ethyl acetate. The extract was washed with water and brine, and dried over anhydrous magnesium sulfate. After concentration under reduced pressure, the residue was purified by silica gel column chromatography to give 11.4 g of 4-(2-chloropyridin-4-yl)-3-(4-fluorophenyl)-1-(2-cyanobenzyl)-4-imidazolin-2-one as colorless crystals. Melting point: 109°C.

### Reference example 2

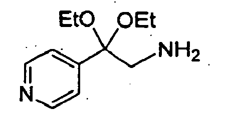

By using 4-acetylpyridine (commercially available product) as a starting material, the same treatments as in Reference examples 1(2) to (4) were carried out to give 2,2-diethoxy-2-pyridin-4-yl ethylamine as brownish oily product.

### Reference example 3

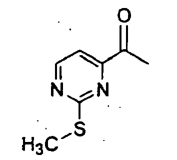

(1) A mixture of 100 g of 3,3-dimethoxy-2-butanone and 99.2, g of N,N-dimethylformamide dimethylacetal was stirred at 100°C for 42 hours. After cooling the reaction mixture, the mixture was concentrated under reduced pressure to give 141 g of 1-dimethylamino-4,4-dimethoxy-1-penten-3-one.
(2) In 800 ml of methanol was dissolved 141 g of the compound obtained in (1), and after adding 114 g of thiourea.and 292 g of 28% sodium methoxide-methanol, the mixture was stirred at 70°C for 3 hours. The mixture was ice-cooled, and after adding 215 g of methyl iodide drowise, the mixture was stirred at room temperature for an hour. After concentration of the reaction mixture, water was added to the mixture and the resulting mixture was extracted with ethyl acetate. The organic layer was washed, dried and concentrated to give 142 g of 4-(1,1-dimethoxyethyl)-2-methylsulfanylpyrimidine.
(3) In 570 ml of acetone was dissolved 142 g of the compound obtained in (2), and under ice-cooling, 114 ml of 6M hydrochloric acid was added to the solution and the mixture was stirred at room temperature for 3 hours. After adding 450 ml of water to the mixture, the solvent was removed and the residue was extracted with ethyl acetate. The organic layer was washed; dried and concentrated to give 107 g of 1-(2-methyl-sulfanylpyrimidin-4-yl)ethanone.

### Reference example 4

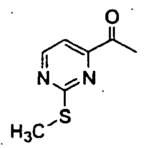

(1) A mixture comprising 16.4 g of 4-chloro-2-methyl-sulfanylpyrimidine, 38 g of tributyl (1-ethoxyvinyl) tin, 1.43 g of bis (triphenylphosphine)palladium (II) dichloride and 100, ml of DMF was stirred at 80°C for 3 hours. After cooling the reaction mixture, 300 ml of ethyl acetate and 17.8 g of potassium fluoride were added to the mixture, and the resulting mixture was stirred at room temperature overnight. After filtration with Celite, the filtrate was washed, dried and concentrated. The residue was purified by silica gel column chromatography (hexane:ethylacetate=20:1) to give 18.9 g of 4-(1-ethoxy-vinyl)-2-methylsulfanylpyrimidine.
(2) In 200 ml of acetone was dissolved 18.9 g of the compound obtained in (1), 60 ml of 4M hydrochloric acid was added to the solution and the mixture was stirred at room temperature for an hour. The reaction mixture was added to a saturated aqueous sodium bicarbonate solution, and extracted with ethyl acetate. The organic layer was washed, dried and concentrated to give 15.9 g of 1-(2-methylsulfanylpyrimidin-4-yl)ethanone.

### Reference example 5.

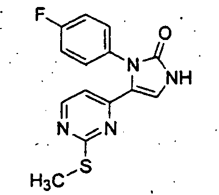

(1) In 180 ml of methanol was dissolved 17.6 g of the compound obtained in Reference example 3 (3) or Reference example 4(2), 14.5 g of hydroxylamine hydrochloride sand 17.2 g of sodium acetate were added to the solution, and the mixture was refluxed under heating for 30 minutes. After cooling the reaction mixture, the solvent was removed, water was added to the residue and the mixture was extracted with ethyl acetate. The organic layer was washed, dried and concentrated. To the residue was added hexane and the precipitated crystals were collected by filtration to give 18.3 g of 1-(2-methylsulfanylpyrimidin-4-yl)ethanone oxime. Melting point: 150-152°C.
(2) In 1200 ml of methylene chloride was suspended 89 g of the compound obtained in (1), and 81.2 ml of triethylamine and 102 g of tosyl chloride were added to the suspension, and the mixture was stirred at room temperature overnight. The reaction mixture was washed, dried and concentrated. To the residue was added diethyl ether and the precipitated crystals were collected by filtration to give 159 g of 1-(2-methylsulfanylpyrimidin-4- yl)ethanoneoxime tosylate. Melting point: 141-142°C.
(3) To 30 ml of methanol solution containing 12.9 g of 28% sodium methoxide-methanol was added dropwise 120 ml of a THF solution containing 15 g of the compound obtained in (2) under ice-cooling, and the mixture was stirred at room temperature overnight. To the mixture was added 100 ml of 4M hydrochloric acid-dioxane solution under ice-cooling, and after stirring at room temperature for 4 hours, the reaction mixture was concentrated. The residue was added to an aqueous potassium carbonate solution and.extracted with chloroform. The organic layer was dried and concentrated, and the residue was purified by silica gel column chromatography (chloroform:methanol= 15:1) to give 8.14 g of 2,2-dimethoxy-2-(2-methyl-sulfanylpyrimidin-4-yl)ethylamine.
(4) To 120 ml of a THF solution containing 8 g of the compound obtained in (3) was added dropwise under ice-cooling 30 ml of a THF solution containing 4.78 g of 4-fluorophenyl isocyanate, and the mixture was stirred at room temperature for 30 minutes. After 120 ml of conc. hydrochloric acid was added to the mixture under ice-cooling, the resulting mixture was stirred at room temperature overnight. Precipitated crystals were collected by filtration, washed with water and ether, and dried to give 7.35 g of 1-(4-fluorophenyl)-5-(2-methylsulfanylpyrimidin-4-yl)-4-imidazolin-2-one. Melting point: 260-261°C. Reference example 6

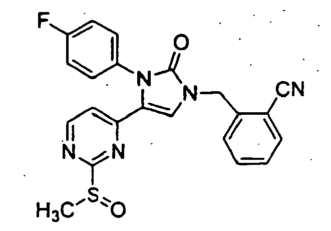

(1) To 40 ml of a DMF solution containing 2.6 g of the compound obtained in Reference example 5(4) was added 327 mg of sodium hydride at room temperature, and the mixture was stirred at room temperature for 30 minutes. To the mixture was added 1.77 g of 2-cyanobenzyl bromide, and after stirring at room temperature for 30 minutes, 33 mg of sodium hydride and 85 mg of 2-cyanobenzyl bromide were added to the mixture, and the resulting mixture was stirred at room temperature for an hour. Water was added to the reaction mixture and the mixture was extracted with ethyl acetate. The organic layer was washed, dried and concentrated, and crystallized from diethyl ether to give 3.28 g of 1-(2-cyanobenzyl)-3-(4-fluorophenyl)-4-(2-methylsulfanylpyrimidin-4-yl)-4-imidazolin-2-one:
   Melting point: 141-142°C.
(2) To a chloroform solution containing 3.27 g of the compound obtained in (1) was added 2.03 g of 3-chloroperoxybenzoic acid at room temperature, and the mixture was stirred at room temperature for an hour. To the reaction mixture was added 1.16 g of calcium hydroxide and the-mixture was stirred at room temperature for 2 hours, and then, filtered through Celite, and the filtrate was concentrated. The residue was crystallized from ethyl acetate to give 2.39 g of 1-(2-cyanobenzyl)-3-(4-fluorophenyl)-4-(2-methylsulfinylpyrimidin-4-yl)-4-imidazolin-2-one. Melting point: 133-136°C.

### Reference example 7

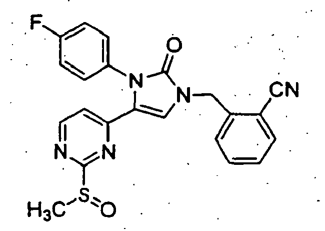

(1) To 150 ml of a methanol solution containing 1.47 g of the compound obtained in Reference example 5(4) was added dropwise 10 ml of an aqueous solution containing 1. 79 g of Oxone® at room temperature. After 30 minutes and 2 hours, 2 ml of an aqueous solution containing 299 mg of Oxone® was added dropwise, and the mixture was stirred at room temperature for 2 hours. After removing insoluble matters by filtration, the filtrate was concentrated, an aqueous sodium bicarbonate solution was added to the concentrate and the mixture was extracted with chloroform. The organic layer was washed, dried and concentrated, and the precipitated crystals were collected by a mixed solvent of ethyl acetate-ether (1:1) to give 1.03 g of 1-(4-fluorophenyl)-5-(2-methylsulfinylpyrimidin-4-yl)-4-imidazolin-2-one. Melting point: 208-211°C (decomposed).
(2) The compound (930 mg) obtained in (1) was treated in the same manner as in the above-mentioned Reference example 6(1) to give 541 mg of 1-(2-cyanobenzyl)-3-(4-fluorophenyl)-4-(2-methylsulfinylpyrimidin-4-yl)-4-imidazolin-2-one.

### Reference example 8

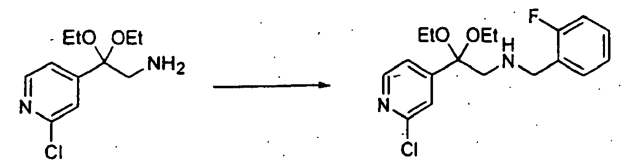

In 10 ml of methanol was dissolved 1.0 g of the compound obtained in Reference example 1 (4), 0.51 g of 2-fluorobenzaldehyde was added to the solution, and the mixture was stirred at room temperature for 30 minutes. To the mixture was added 155 mg of sodium borohydride, and the resulting mixture was further stirred at room temperature for an hour. After concentration under reduced pressure, water was added to the reside and the mixture was extracted with ethyl acetate. The extract was washed with brine and dried over anhydrous magnesium sulfate. After concentration under reduced pressure, the residue was purified by silica gel column chromatography (hexane:ethylacetate=2:1) to give 1.45 g the title compound as an oily product.

### Reference example 9

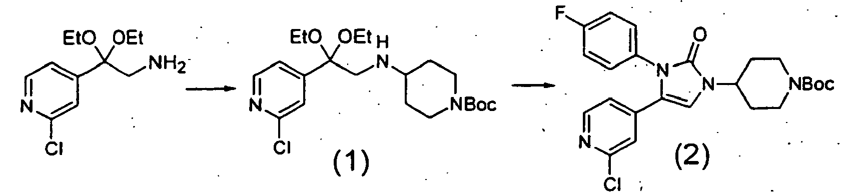

The compound (5 g) obtained in Reference example 1(4) and a corresopnding starting material were treated in the same manner as in Reference example 8 to give 8.47 g of Compound (1) . Compound (1) (3 g) was treated in the same manner as in Example 1 to carry out cyclization, subsequently the resulting compound was dissolved in 20 ml of THF, 1.1 g of Boc₂O was added thereto. The resulting mixture was stirred at room temperature for 30 minutes, concentrated under reduced pressure and diisopropyl ether was added to the residue, and the residue was collected by filtration to give 2.53 g of Compound (2).

### Reference example 10

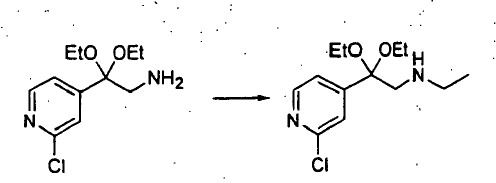

A mixture comprising 3.8 g of the compound obtained in Reference example 1(4), 1.7 ml of ethyl iodide and 3.0 ml of triethylamine was stirred at 50°C overnight. After neutralizing with 2N aqueous NaOH solution, the reaction mixture was extracted with chloroform and dried over anhydrous magnesium sulfate. The resulting mixture was purified by NH silica gel column chromatography (hexane:ethyl acetate=4:1) to give 1.9 g of the title compound as an oily product.

### Reference example 11

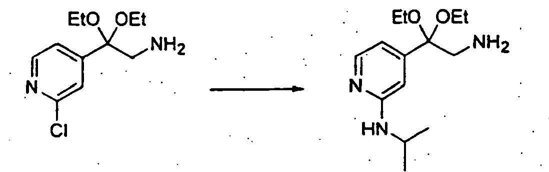

In 75 ml of toluene were suspended 5. 0 g of the compound obtained in Reference example 1(4), 35ml of isopropylamine, 458 mg of palladium acetate, 1.28 g of 2,2'-bis(diphenylphosphino)-1,1'-binaphthyl and 3.0 g of sodium t-butoxide, and under nitrogen flow, the mixture was stirred under heating at 70°C for 8 hours. After concentration under reduced pressure, water was added to the residue, and the mixture was extracted with chloroform, washed with brine and dried over anhydrous magnesium sulfate. After concentration under reduced pressure, the residue was purified by silica gel column chromatography (chloroform:methanol= 10:1) to give 9.3 g of the title compound as an oily product.

### Reference example 12

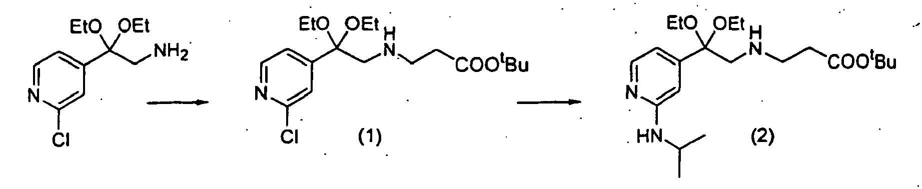

A mixture comprising 2.0. g of the compound obtained in Reference example 1(4), on 82 ml of t-butyl acrylate and 10 ml of THF was stirred under reflux for 4 days. The reaction mixture was concentrated under reduced pressure to give 3.1g of Compound (1) as an oily product. Then, Compound (1) and a corresponding starting material were treated in the same manner as in Example 4 to give 2.12 g of Compound (2) as an oily product.

### Reference example 13

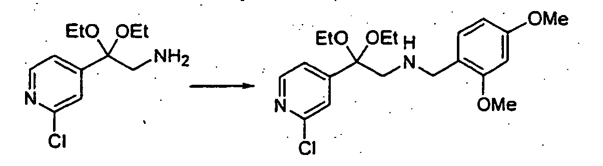

The compound (5.0 g) obtained in Reference example 1(4) was reacted with 2,4-dimethoxybenzaldehyde in the same manner as in Reference example 8 to give 6.4 g of the title compound.

### Reference example 14

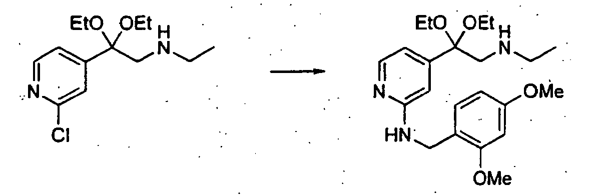

The compound (1.39 g) of Reference example 10 was reacted with 2,4-dimethoxybenzylamine in the same manner as in Reference example 11 to give 1.58 g of the title compound.

### Reference example 15

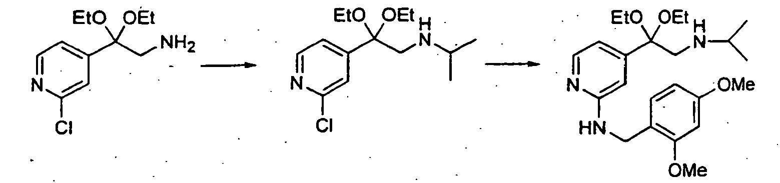

The compound (10.0 g) of Reference example 1(4) was reacted with a corresponding starting material in the same manner as in Reference example 8, and then, reacted with 2,4-dimethoxybenzylamine in the same manner as in Reference example 11 to give 9.75 g of the title compound.

### Reference example 16

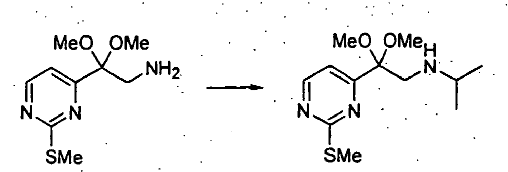

The compound (26.8 g) of Reference example 5(3) and a corresponding starting material were treated in the same manner as in Reference example 8 to give 30.8 g of the title compound. Reference example 17 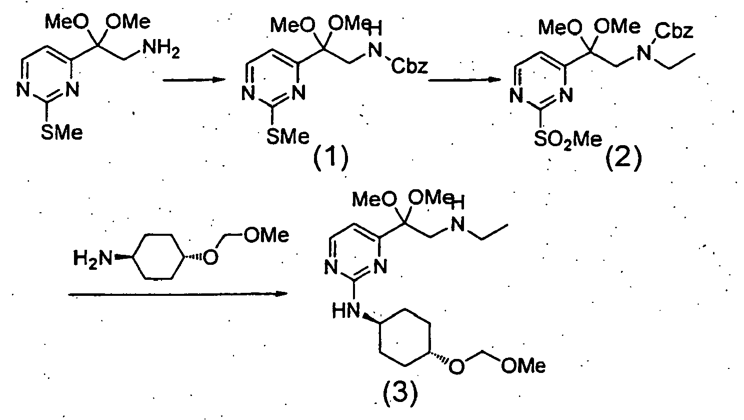
(1) In 30 ml of methylene chloride was dissolved 3.0 g of the compound of Reference example 5(3), 3.65 ml of triethylamine was added to the solution, and under ice-cooling, 3.35 g of benzyloxycarbonyl chloride was added dropwise to the mixture, and the mixture was stirred at room temperature overnight. The reaction mixture was washed with water and brine, and dried over anhydrous magnesium sulfate. After concentration under reduced pressure, the residue was purified by silica gel column chromatography to give 2.23 g of Compound (1) as colorless crystals.
   MS 364 ([M+H)⁺)
(2) In 17 ml of DMF was dissolved 4.2.g of Compound (1), and under ice-cooling, 528 mg of sodium hydride was added to the solution, and the mixture was stirred at room temperature for an hour. The mixture was again ice-cooled, 1.39 ml of ethyl iodide was added thereto, and the resulting mixture was stirred at room temperature for 30 minutes. Water was added to the reaction mixture and the mixture was extracted with ethyl acetate, the extract was washed with water and brine, and dried over anhydrous magnesium sulfate. After concentration under reduced pressure, the residue was dissolved in 50 ml of chloroform, 6.26 g of 3-chloroperoxybenzoic acid was added to the mixture at room temperature, and the resulting mixture was stirred at the same temperature for 30 minutes. To the reaction mixture was added 2.58 g of calcium hydroxide and after stirring the mixture, the insoluble matters were removed by filtration. The filtrate was concentrated under reduced pressure, and purified by silica gel column chromatography to give 4.55 g of Compound (2) as a colorless oily product.
   MS 423 ([M+H]⁺)
(3) In 30 ml of dioxane was dissolved 2.19 g of Compound (2), 1.65 g of trans-4-(Methoxymethoxy)cyclohexylamine and 1. 08 ml of 1,1'-diisopropylethylamine were added to the solution, and the mixture was stirred at 100°C for 14 hours. Water was added to the reaction mixture and the mixture was extracted with ethyl acetate, washed with brine and dried over anhydrous magnesium sulfate. After concentration under reduced pressure, the residue was purified by silica gel column chromatography to give 2.0 g of a brownish oily product. This product was dissolved in 40 ml of methanol, 1 g of 10% palladium-carbon was added thereto, and the mixture was subjected to catalytic reduction under hydrogen pressure (2.7 atm) for 2 hours. Palladium was removed-by filtration, and after concentration under reduced pressure, the residue was purified by NH silica gel column chromatography to give 1.04 g of Compound (3) as a brownish oily product.
   MS 369 [M+H]⁺)

### Experimental Example 1 (pharmacological test)

### Inhibition of lipopolysaccharide (LPS)-stimulated TNF-α production in mice in vivo

Tests were carried out to measure an inhibitory effects of the compounds of the present invention on LPS-stimulated TNF-α production in mice.

To Balb/cAnNCrj mice (6-8 weeks old, female, available from Japan Charlesriver, Co.) were administered test compounds (10 mg/kg, p.o.) dissolved in 0.5% methyl cellulose and 0.2% PEG-60 hydrogenated caster oil (HCO60, available from Nikko Chemicals, Co.). After 30 minutes, LPS (E. coli 0111:B4, available from Difco, with a final concentration of 1 mg/kg adjusted by phosphate buffered saline) was administered (0.4 ml/head, i.p.). 90 minutes later, blood was collected from abdominal vein of the mouse under diethyl ether anesthesia. The collected blood was subjected to centrifugation with 3000g to collect serum. TNF-α in the sera was measured by DuoSet mouse TNF-α ELISA kit (trade name, available from genzymeTECHNE).

As a result, the compounds of the present invention significantly reduced the production of TNF-α as shown in Table 52.

**Table 52**

| Examples | TNF-α inhibition rate |
|---|---|
| 182 | 64% |
| 202 | 57% |
| 239 | 69% |
| 296 | 52% |
| 300 | 57% |

### Industrial Applicability

According to the present invention, a novel 4-imidazolin-2-one compound having excellent p38MAP kinase inhibitory activity, which is useful as a medicine, can be provided.

## Claims

1. A compound having the formula [Ia] : 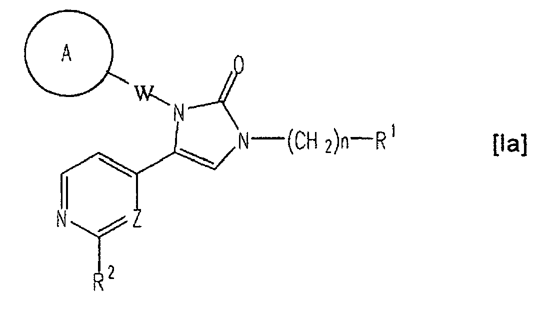 wherein
• ring A is benzene ring or thiophene ring, and the benzene ring and thiophene ring may be substituted by 1 to 3 substituent(s), which is(are) the same or different, and selected from the group consisting of
- a halogen atom;
- nitro;
- a C₁-C₆ alkyl which may be substituted by 1 to 3 group(s) selected from a halogen atom(s), hydroxy(s) and amino(s);
- a C₁-C₆ alkoxy which may be substituted by 1 to 3 group(s) selected from hydroxy(s) and amino(s);
- an amino which may be substituted by 1 or 2 group(s) selected from (a) a C₁-C₆ alkyl which may be substituted by 1 to 3 group(s) selected from the group consisting of C₁-C₆ allcoxy(s), amino(s) and carboxy(s), and (b) a C₂-C₇ alkanoyl;
- a carbamoyl which may be substituted by 1 or 2 C₁-C₆ alkyl(s); and
- cyano,
• W is a single bond, or a C₁-C₄ alkylene which may be substituted by 1 or 2 C₁-C₆ alkyl(s),
• n is 0, 1, 2, 3 or 4,
• R¹ is
- hydrogen atom;
- a C₁-C₆ alkyl which may be substituted by 1 to 3 group(s) selected from (a) a C₂-C₇ alkynyl, (b) cyano, (c) a C₁-C₆ alkoxy, (d) hydroxy, (e) amino which may be substituted with 1 or 2 substituents selected from the group consisting of a C₁-C₆ alkyl, a C₂-C₇ alkanoyl and a C₁-C₆ alkyl-sulfonyl, (f) carboxy, (g) a C₁-C₆ alkoxy-carbonyl, (h) carbamoyl which may be substituted with 1 or 2 C₁-C₆ alkyl(s), (i) phenyl and (j) naphthyl;
- a C₃-C₈ cycloalkyl which may be substituted by 1 to 3 group(s) selected from (a) hydroxy, (b) a C₁-C₆ alkoxy which may be substituted by 1 to 3 C₁-C₆ alkoxy(s), (c) amino which may be substituted by I or 2 group(s) selected from the group consisting of the following (1) to (5): (1) a C₁-C₆ alkyl, (2) a C₂-C₇ alkanoyl, (3) a C₁-C₆ alkoxy-carbonyl, (4) carbamoyl which may be substituted by 1 or 2 C₁-C₆ alkyl(s), and(5) a C₁-C₆ alkyl-sulfonyl, (d) carboxy, (e) a C₁-C₆ alkyl which may be substituted by a group selected from the group consisting of hydroxy, a C₁-C₆ alkoxy and amino, (f) a carbamoyl which may be substituted by C₁-C₆ alkyl(s);
- a phenyl which may be substituted by 1 to 3 group(s) selected from (a) a halogen atom, (b) nitro, (c) a C₁-C₆ alkyl which may be substituted by 1 to 3 group(s) selected from the group consisting of a halogen atom, hydroxy, amino, carboxy, and phenylsulfonyl, (d) a C₂-C₇ alkenyl, (e) cyano, (f) hydroxy, (g) a C₁-C₆ alkoxy which may be substituted by 1 to 3 group(s) selected from the group consisting of a halogen atom, carboxy, a C₁-C₆ alkoxy-carbonyl, carbamoyl, phenyl and morpholinylcarbonyl, (h) amino which may be substituted with 1 or 2 group(s) selected from the group consisting of the following (1) to (4) : (1) a C₁-C₆ alkyl, (2) a C₂-C₇ alkanoyl, (3) carbamoyl which may be substituted by 1 or 2 group(s) selected from the group consisting of a C₁-C₆ alkyl and a C₃-C₈ cycloalkyl, and (4) a C₁-C₆ alkyl-sulfonyl, (i) a C₂-C₇ alkanoyl, (j) carboxy, (k) a C₁-C₆alkoxy-carbonyl, (1) carbamoyl which may be substituted by 1 or 2 group(s) selected from the group consisting of the following (1) and (2): (1) a C₁-C₆ alkyl which may be substituted by 1 to 3 hydroxy(s) and (2) a C₃-C₈ cycloalkyl, (m) a C₁-C₆ alkyl-thio, (n) a C₁-C₆ alkyl-sulfinyl, (o) a C₁-C₆ alkyl-sulfonyl, (p) phenyl, (q) tetrazolyl, and (r) a carbonyl substituted by heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of a C₁-C₆ alkyl and a C₁-C₆ alkoxy-carbonyl; or
- a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from (a) a halogen atom, (b) nitro, (c) a C₁-C₆alkyl which may be substituted by a group selected from the group consisting of hydroxy, a C₁-C₆alkoxy, a carbamoyl which may be substituted by C₁-C₆ alkyl(s) and carboxy(s), (d) cyano, (e) hydroxy, (f) amino, (g) a C₂-C₇ alkanoyl, (h) carboxy, (i) a C₁-C₆ alkoxy-carbonyl, (j) carbamoyl which may be substituted by 1 or 2 C₁-C₆ alkyl(s), (k) a C₁-C₆ alkyl-sulfonyl, and (1) phenyl,
• Z is CH or N,
• R² is hydrogen atom, -NR³R⁴, -OR⁵, -COR⁶ or -CHR⁷R⁸,
where R³ to R⁸, each independently is
- hydrogen atom,
- a C₁-C₆ alkyl which may be substituted by 1 to 3 group(s) selected from (a) hydroxy, (b) a C₁-C₆alkoxy, (c) amino which may be substituted by I or 2 group(s) selected from the group consisting of a C₁-C₆ alkyl, a C₂-C₇ alkanoyl and a C₁-C₆ alkyl-sulfonyl, (d) a C₁-C₆ alkoxy-carbonyl, (e) a C₃-C₈ cycloalkyl which may be substituted by 1 to 3 group(s) selected from the group consisting of (1) hydroxy, (2) an amino which may be substituted by C₁-C₆ alkyl(s), (3) a C₂-C₇ alkanoyl-amino, (4) a C₁-C₆ alkyl-sulfonylamino, (5) a C₁-C₆ alkyl which may be substituted by a group selected from hydroxy, a C₁-C₆ alkoxy, amino and a carbamoyl which may be substituted by C₁-C₆ alkyl(s), (6) carboxy and (7) a carbamoyl which may be substituted by C₁-C₆ alkyl(s),(f) phenyl which may be substituted by 1 to 3 group(s) selected from the group consisting of the following (1) to (6): (1) a halogen atom, (2) a C₁-C₆ alkoxy, (3) amino which may be substituted by 1 or 2 group(s) selected from the group consisting of a C₁-C₆ alkyl and a C₁-C₆ alkoxy-carbonyl, (4) a C₁-C₆ alkoxy-carbonyl, (5) carbamoyl, and (6) morpholinylcarbonyl, and (g) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of the following (1) to (5): (1) a C₁-C₆ alkyl which may be substituted by 1 to 3 hydroxy(s), (2) hydroxy, (3) amino, (4) a C₁-C₆ alkoxy-carbonyl, and (5) carbamoyl;
- a C₂-C₇ alkenyl;
- a C₂-C₇ alkynyl;
- hydroxy;
- a C₁-C₆alkoxy;
- an amino which may be substituted by 1 or 2 group(s) selected from (a) a C₁-C₆ alkyl which may be substituted by 1 to 3 group(s) selected from the group consisting of (1) hydroxy, (2) a C₁-C₆ alkoxy and (3) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom, (b) a C₃-C₈ cycloalkyl which may be substituted by 1 to 3 hydroxy(s), and (c) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
- a C₂-C₇ alkanoyl which may be substituted by 1 to 3 group(s) selected from (a) hydroxy, (b) a C₁-C₆alkoxy, (c) amino which may be substituted by 1 or 2 group(s) selected from the group consisting of a C₁-C₆ alkyl and a C₂-C₇ alkanoyl, and (d) a C₁-C₆ alkoxy-carbonyl;
- a carbamoyl which may be substituted by 1 or 2 group(s) selected from (a) a C₁-C₆ alkyl, (b) a C₃-C₈ cycloalkyl, and (c) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
- a C₁-C₆ alkoxy-oxalyl;
- a C₁-C₆ alkyl-sulfonyl;
- a C₃-C₈ cycloalkyl which may be substituted by 1 to 3 group(s) selected from (a) a halogen atom, (b) a C₁-C₆ alkyl, which may be substituted by 1 to 3 group(s) selected from the group consisting of (1) hydroxy, (2) a C₁-C₆alkoxy, (3) amino and (4) a carbamoyl which may be substituted by a C₁-C₆ alkyl, (c) hydroxy, (d) a C₁-C₆ alkoxy, (e) amino which may be substituted by 1 or 2 group(s) selected from the group consisting of (1) a C₁-C₆ alkyl, (2) a C₂-C₇ alkanoyl, (3) a C₁-C₆ alkoxy-carbonyl and (4) a C₁-C₆alkyl-sulfonyl, (f) carboxy, (g) a C₂-C₇ alkanoyl-oxy, (h) a C₁-C₆ alkoxy-carbonyl, and (i) carbamoyl which may be substituted by 1 or 2 group(s) selected from the group consisting of (1) a C₁-C₆ alkyl, (2) a C₃-C₈ cycloalkyl and (3) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
- a phenyl which may be substituted by 1 to 3 group(s) selected from (a) a C₁-C₆alkyl, (b) hydroxy, (c) a C₁-C₆ alkoxy, (d) a halogen atom, and (e) amino which may be substituted by 1 or 2 C₁-C₆ alkyl(s);
- a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from (a) a C₁-C₆ alkyl which may be substituted by 1 to 3 group(s) selected from the group consisting of (1) phenyl, (2) hydroxy, (3) a C₁-C₆ alkoxy, (4) amino and (5) a carbamoyl which may be substituted by a C₁-C₆ alkyl, (b) carboxy, (c) a C₁-C₆ alkoxy-carbonyl, (d) a C₂-C₇ alkanoyl, (e) a C₁-C₆ alkyl-sulfonyl, and (f) oxo;
- a carbonyl substituted by a C₃-C₈ cycloalkyl which may be substituted by 1 to 3 group(s) selected from (a) hydroxy, (b) a C₁-C6 alkoxy, (c) amino which may be substituted by 1 or 2 group(s) selected from the group consisting of (1) a C₁-C₆ alkyl and (2) a C₂-C₇ alkanoyl, and (d) a C₁-C₆ alkoxy-carbonyl;
- a carbonyl substituted by phenyl, which may be substituted by 1 to 3 group(s) selected from (a) a halogen atom, (b) hydroxy, (c) a C₁-C₆ alkoxy, and (d) amino which may be substituted by 1 or 2 group(s) selected from the group consisting of a C₃-C₆ alkyl and a C₂-C₇ alkanoyl; or
- a carbonyl substituted by a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 groups selected from (a) a halogen atom, (b) a C₁-C₆ alkyl, (c) hydroxy, (d) amino which may be substituted by 1 or 2 C₁-C₆ alkyl(s), (e) a C₂-C₇ alkanoyl, and (f) oxo,
or a pharmaceutically acceptable salt thereof.

2. The compound according to Claim 1, wherein the ring A is a benzene ring which may be substituted by 1 to 3 substituent(s), which is(are) the same or different, and selected from the group consisting of
- a halogen atom;
- nitro;
- a C₁-C₆ alkyl which may be substituted by 1 to 3 group(s) selected from a halogen atom(s), hydroxy(s) and amino(s);
- a C₁-C₆ alkoxy which may be substituted by 1 to 3 group(s) selected from hydroxy(s) and amino(s);
- an amino which may be substituted by 1 or 2 group(s) selected from (a) a C₁-C₆ alkyl which may be substituted by 1 to 3 group(s) selected from the group consisting of C₁-C₆ alkoxy(s), amino(s) and carboxy(s), and (b) a C₂-C₇ alkanoyl, and
- cyano,
and W is a single bond,
or a pharmaceutically acceptable salt thereof.

3. The compound according to Claim I or 2, wherein n is 0 or 1, or a pharmaceutically acceptable salt thereof.

4. The compound according to any one of Claims 1 to 3, wherein
• n is 0 and R¹ is a C₁-C₆ alkyl which may be substituted by 1 to 3 group(s) selected from (a) a C₂-C₇ alkynyl, (b) cyano, (c) a C₁-C₆ alkoxy, (d) hydroxy, (e) amino which may be substituted with 1 or 2 substituents selected from the group consisting of a C₁-C₆ alkyl, a C₂-C₇ alkanoyl and a C₁-C₆ alkyl-sulfonyl, (f) carboxy, (g) a C₁-C₆ alkoxy-carbonyl, (h) carbamoyl which may be substituted with 1 or 2 C₁-C₆ alkyl(s), (i) phenyl and (j) naphthyl, or
• n is 1 and R¹ is
(A) a C₃-C₈ cycloalkyl which may be substituted by 1 to 3 group(s) selected from (a) hydroxy, (b) a C₁-C₆alkoxy which may be substituted by 1 to 3 C₁-C₆ alkoxy(s), (c) amino which may be substituted by 1 or 2 group(s) selected from the group consisting of the following (1) to (5): (1) a C₁-C₆ alkyl, (2) a C₂-C₇ alkanoyl, (3) a C₁-C₆ alkoxy-carbonyl, (4) carbamoyl which may be substituted by 1 or 2 C₁-C₆alkyl(s), and (5) a C₁-C₆ alkyl-sulfonyl, (d) carboxy, (e) a C₁-C₆ alkyl which may be substituted by a group selected from the group consisting of hydroxy, a C₁-C₆ alkoxy and amino, (f) a carbamoyl which may be substituted by C₁-C₆ alkyl(s), or
(B) a phenyl which may be substituted by 1 to 3 group(s) selected from (a) a halogen atom, (b) nitro, (c) a C₁-C₆ alkyl which may be substituted by 1 to 3 group(s) selected from the group consisting of a halogen atom, hydroxy, amino, carboxy, and phenylsulfonyl, (d) a C₂-C₇ alkenyl, (e) cyano, (f) hydroxy, (g) a C₁-C₆ alkoxy which may be substituted by 1 to 3 group(s) selected from the group consisting of a halogen atom, carboxy, a C₁-C₆ alkoxy-carbonyl, carbamoyl, phenyl and morpholinylcarbonyl, (h) amino which may be substituted with 1 or 2 group(s) selected from the group consisting of the following (1) to (4) : (1) a C₁-C₆ alkyl, (2) a C₂-C₇ alkanoyl, (3) carbamoyl which may be substituted by 1 or 2 group(s) selected from the group consisting of a C₁-C₆ alkyl and a C₃-C₈ cycloalkyl, and (4) a C₁-C₆ alkyl-sulfonyl, (i) a C₂-C₇ alkanoyl, (j) carboxy, (k) a C₁-C₆ alkoxy-carbonyl, (1) carbamoyl which may be substituted by 1 or 2 group(s) selected from the group consisting of the following (1) and (2): (1) a C₁-C₆ alkyl which may be substituted by 1 to 3 hydroxy(s) and (2) a C₃-C₈ cycloalkyl, (m) a C₁-C₆ alkyl-thio, (n) a C₁-C₆alkyl-sulfinyl, (o) a C₁-C₆ alkyl-sulfonyl, (p) phenyl, (q) tetrazolyl, and (r) a carbonyl substituted by heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of a C₁-C₆ alkyl and a C₁-C₆ alkoxy-carbonyl,
or a pharmaceutically acceptable salt thereof.

5. The compound according to any one of Claims 1 to 4, wherein R² is -NR³R⁴ or -OR⁵, or a pharmaceutically acceptable salt thereof.

6. The compound according to any one of Claims 1 to 4, wherein R² is -NHR⁴, and R⁴ is
- a C₁-C₆ alkyl which may be substituted by 1 to 3 group(s) selected from
(a) hydroxy,
(b) a C₁-C₆ alkoxy,
(c) amino which may be substituted by 1 or 2 group(s) selected from the group consisting of a C₁-C₆ alkyl, a C₂-C₇ alkanoyl and a C₁-C₆ alkyl-sulfonyl,
(d) a C₁-C₆ alkoxy-carbonyl,
(e) a C₃-C₈ cycloalkyl which may be substituted by 1 to 3 group(s) selected from the group consisting of (1) hydroxy, (2) an amino which may be substituted by C₁-C₆ alkyl(s), (3) a C₂-C₇ alkanoyl-amino, (4) a C₁-C₆ alkyl-sulfonylamino, (5) a C₁-C₆ alkyl which may be substituted by a group selected from hydroxy, a C₁-C₆ alkoxy, amino and a carbamoyl which may be substituted by C₁-C₆ alkyl(s), (6) carboxy and (7) a carbamoyl which may be substituted by C₁-C₆ alkyl(s),
(f) phenyl which may be substituted by 1 to 3 group(s) selected from the group consisting of the following (1) to (6): (1) a halogen atom, (2) a C₁-C₆ alkoxy, (3) amino which may be substituted by 1 or 2 group(s) selected from the group consisting of a C₁-C₆ alkyl and a C₁-C₆ alkoxy-carbonyl, (4) a C₁-C₆ alkoxy-carbonyl, (5) carbamoyl, and (6) morpholinylcarbonyl, and
(g) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of the following (1) to (5): (1) a C₁-C₆ alkyl which may be substituted by 1 to 3 hydroxy(s), (2) hydroxy, (3) amino, (4) a C₁-C₆ alkoxy-carbonyl, and(5) carbamoyl,;
- a C₂-C₇ alkenyl;
- a C₂-C₇ alkanoyl which may be substituted by 1 to 3 group(s) selected from (a) hydroxy, (b) a C₁-C₆ alkoxy, (c) amino which may be substituted by 1 or 2 group(s) selected from the group consisting of a C₁-C₆ alkyl and a C₂-C₇ alkanoyl, and (d) a C₁-C₆ alkoxy-carbonyl;
- a carbamoyl which may be substituted by 1 or 2 group(s) selected from (a) a C₁-C₆ alkyl, (b) a C₃-C₈ cycloalkyl, and (c) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
- a C₃-C₈ cycloalkyl which may be substituted by 1 to 3 group(s) selected from (a) a halogen atom, (b) a C₁-C₆ alkyl which may be substituted by 1 to 3 group(s) selected from the group consisting of (1) hydroxy, (2) a C₁-C₆ alkoxy, (3) amino and (4) a carbamoyl which may be substituted by a C₁-C₆ alkyl, (c) hydroxy, (d) a C₁-C₆ alkoxy, (e) amino which may be substituted by 1 or 2 group(s) selected from the group consisting of (1) a C₁-C₆alkyl, (2) a C₂-C₇ alkanoyl, (3) a C₁-C₆ alkoxy-carbonyl and (4) a C₁-C₆ alkyl-sulfonyl, (f) carboxy, (g) a C₂-C₇ alkanoyl-oxy, (h) a C₁-C₆ alkoxy-carbonyl, and (i) carbamoyl which may be substituted by 1 or 2 group(s) selected from the group consisting of (1) a C₁-C₆ alkyl, (2) a C₃-C₈ cycloalkyl and (3) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom;
- a phenyl which may be substituted by 1 to 3 group(s) selected from (a) a C₁-C₆ alkyl, (b) hydroxy, (c) a C₁-C₆ alkoxy, (d) a halogen atom, and (e) amino which may be substituted by 1 or 2 C₁-C₆ alkyl(s);
- a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from (a) a C₁-C₆ alkyl which may be substituted by 1 to 3 group(s) selected from the group consisting of (1) phenyl, (2) hydroxy, (3) a C₁-C₆ alkoxy, (4) amino and (5) a carbamoyl which may be substituted by a C₁-C₆ alkyl, (b) carboxy, (c) a C₁-C₆ alkoxy-carbonyl, (d) a C₂-C₇ alkanoyl, (e) a C₁-C₆ alkyl-sulfonyl, and (f) oxo;
- a carbonyl substituted by a C₃-C₈ cycloalkyl which may be substituted by 1 to 3 group(s) selected from (a) hydroxy, (b) a C₁-C₆ alkoxy, (c) amino which may be substituted by 1 or 2 group(s) selected from the group consisting of (1) a C₁-C₆ alkyl and (2) a C₂-C₇ alkanoyl, and (d) a C₁-C₆ alkoxy-carbonyl; or
- a carbonyl substituted by a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 selected from (a) a halogen atom, (b) a C₁-C₆ alkyl, (c) hydroxy, (d) amino which may be substituted by 1 or 2 C₁-C₆ alkyl(s), (e) a C₂-C₇ alkanoyl, and (f) oxo,
or a pharmaceutically acceptable salt thereof.

7. The compound according to Claim 1, wherein
- the ring A is a benzene ring which may be substituted by 1 or 2 substituent(s), which is(are) the same or different, and selected from the group consisting of a halogen atom, a C₁-C₆ alkyl, a C₁-C₆ alkoxy, an amino optionally substituted by C₁-C₆ alkyl(s) and cyano,
- W is a single bond,
- n is 0 or 1,
- R¹ is
(1) hydrogen atom,
(2) a C₁-C₆ alkyl optionally substituted by group(s) selected from the group consisting of phenyl, a C₁-C₆ alkoxy, a C₁-C₆alkyl-amino, a di C₁-C₆ alkyl-amino, a C₂-C₇ alkanoyl-amino, a C₁-C₆ alkyl-sulfonylamino, a carbamoyl optionally substituted by C₁-C₆ alkyl(s), hydroxy, carboxy and cyano,
(3) a C₃-C₈ cycloalkyl optionally substituted by group(s) selected from the group consisting of the following (i) to (v): (i) hydroxy, (ii) a C₁-C₆ alkoxy optionally substituted by C₁-C₆ alkoxy(s), (iii) an amino optionally substituted by group(s) selected from the group consisting of a C₁-C₆ alkyl, a C₂-C₇ alkanoyl and a C₁-C₆ alkyl-sulfonyl, (iv) a carbamoyl optionally substituted by C₁-C₆ alkyl(s), and (v) a C₁-C₆ alkyl optionally substituted by group(s) selected from the group consisting of hydroxy, a C₁-C₆ alkoxy and amino,
(4) a phenyl optionally substituted by group(s) selected from the group consisting of the following (i) to (vi): (i) a halogen atom, (ii) a C₁-C₆ alkyl optionally substituted by group(s) selected from the group consisting of a halogen atom, hydroxy and phenylsulfonyl, (iii) cyano, (iv) a C₁-C₆ alkoxy, (v) an amino optionally substituted by group(s) selected from the group consisting of a C₁C₆ alkyl and a C₁-C₆ alkyl-sulfonyl, (vi) a carbonyl substituted by a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, or
(5) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by group(s) selected from the group consisting of the following (i) to (iv): (i) a C₁-C₆ alkoxy-carbonyl, (ii) a C₁-C₆ alkyl optionally substituted by group(s) selected from the group consisting of hydroxy, a C₁-C₆ alkoxy and a carbamoyl optionally substituted by C₁-C₆ alkyl(s), (iii) a C₂-C₇ alkanoyl and (iv) a C₁-C₆ alkyl-sulfonyl,
- Z is CH or N,
- R² is hydrogen atom, -NR³R⁴, -OR⁵, -COR⁶ or -CHR⁷R⁸,
where R³ to R⁸ each independently is:
(1) hydrogen atom,
(2) a C₁-C₆ alkyl optionally substituted by group(s) selected from the group consisting of the following (i) to (vii):
(i) hydroxy,
(ii) a C₁-C₆ alkoxy,
(iii) an amino optionally substituted by group(s) selected from the group consisting of a C₁-C₆ alkyl, a C₂-C₇ alkanoyl and a C₁-C₆ alkyl-sulfonyl,
(iv) a C₁-C₆ alkoxy-carbonyl,
(v) a C₃-C₈ cycloalkyl optionally substituted by group(s) selected from the group consisting of the following a) to g):
a) hydroxy,
b) an amino optionally substituted by C₁-C₆ alkyl(s),
c) a C₂-C₇ alkanoyl-amino,
d) a C₁-C₆ alkyl-sulfonylamino,
e) a C₁-C₆ alkyl optionally substituted by group(s) selected from the group consisting of hydroxy, a C₁-C₆ alkoxy, amino, a carbamoyl optionally substituted by C₁-C₆ alkyl(s),
f) carboxy and
g) a carbamoyl optionally substituted by C₁-C₆ alkyl(s),
(vi) a phenyl optionally substituted by group(s) selected from the group consisting of a halogen atom, a C₁-C₆ alkoxy and morpholinylcarbonyl, and
(vii) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by C₁-C₆ alkyl(s),
(3) a C₂-C₇ alkenyl,
(4) a C₁-C₆ alkoxy,
(5) a C₂-C₇ alkanoyl optionally substituted by group(s) selected from the group consisting of the following (i) to (iv):
(i) hydroxy,
(ii) a C₁-C₆ alkoxy,
(iii) an amino optionally substituted by group(s) selected from the group consisting of a C₁-C₆ alkyl and a C₂-C₇ alkanoyl,
(iv) a C₁-C₆ alkoxy-carbonyl,
(6) a carbamoyl optionally substituted by C₁-C₆ alkyl(s),
(7) a C ₁-C₆ alkoxy-oxalyl,
(8) a C₃-C₈ cycloalkyl optionally substituted by group(s) selected from the group consisting of the following (i) to (vii):
(i) a halogen atom,
(ii) hydroxy,
(iii) a C₁-C₆ alkoxy,
(iv) an amino optionally substituted by group(s) selected from the group consisting of a C₁-C₆ alkyl, a C₂-C₇ alkanoyl, a C₁-C₆ alkoxy-carbonyl and a C₁-C₆ alkyl-sulfonyl,
(v) a C₁-C₆ alkyl optionally substituted by group(s) selected from the group consisting of hydroxy, a C₁-C₆ alkoxy, amino, a carbamoyl optionally substituted by C₁-C₆ alkyl(s),
(vi) a C₂-C₇ alkanoyl-oxy and
(vii) a carbamoyl optionally substituted by C₁-C₆ alkyl(s),
(9) a phenyl optionally substituted by group(s) selected from the group consisting of a halogen atom and a C₁-C₆ alkoxy,
(10) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by group(s) selected from the group consisting of the following (i) to (v):
(i) a C₁-C₆ alkyl optionally substituted by group(s) selected from the group consisting of phenyl, hydroxy, a C₁-C₆ alkoxy, amino and a carbamoyl optionally substituted by C₁-C₆ alkyl(s),
(ii) a C₁-C₆ alkoxy-carbonyl,
(iii) a C₂-C₇ alkanoyl,
(iv) a C₁-C₆ alkyl-sulfonyl,
(v) oxo
(11) a carbonyl substituted by a C₃-C₈ cycloalkyl optionally substituted by group(s) selected from the group consisting of hydroxy, amino and a C₂-C₇ alkanoyl-amino, or
(12) a carbonyl substituted by heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom,
or a pharmaceutically acceptable salt thereof.

8. A compound according to Claim 1 and having the formula [Ic]: 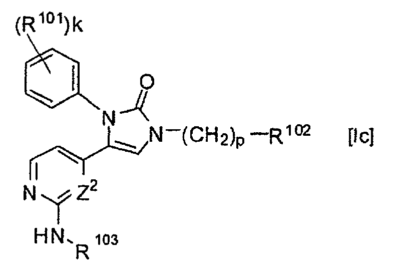 wherein each R¹⁰¹ is the same or different, and selected from the group consisting of
(1) a halogen atom;
(2) nitro;
(3) a C₁-C₆ alkyl which may be substituted by 1 to 3 group(s) selected from a halogen atom(s), hydroxy(s) and amino(s);
(4) an C₁-C₆ alkoxy which may be substituted by 1 to 3 group(s) selected from hydroxy(s) and amino(s);
(5) an amino which may be substituted by 1 or 2 group(s) selected from (a) a C₁-C₆ alkyl which may be substituted by 1 to 3 group(s) selected from the group consisting of C₁-C₆ alkoxy(s), amino(s) and carboxy(s), and (b) a C₂-C₇ alkanoyl; and
(6) cyano,
k is 0 to 3,
p is 0 or 1;
R¹⁰² is
(1) a C₁-C₆ alkyl which may be substituted by 1 to 3 group(s) selected from (a) a C₂-C₇ alkynyl, (b) cyano, (c) a C₁-C₆ alkoxy, (d) hydroxy, (e) amino which may be substituted with 1 or 2 substituents selected from the group consisting of a C₁-C₆ alkyl, a C₂-C₇ alkanoyl and a C₁-C₆ alkyl-sulfonyl, (f) carboxy, (g) a C₁-C₆ alkoxy-carbonyl, (h) carbamoyl which may be substituted with 1 or 2 C₁-C₆ alkyl(s), (i) phenyl and (j) naphthyl; or
(2) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from (a) a halogen atom, (b) nitro, (c) a C₁-C₆ alkyl which may be substituted by a group selected from the group consisting of hydroxy, a C₁-C₆ alkoxy, a carbamoyl which may be substituted by C₁-C₆ alkyl(s) and carboxy(s), (d) cyano, (e) hydroxy, (f) amino, (g) a C₂-C₇ alkanoyl, (h) carboxy, (i) a C₁-C₆ alkoxy-carbonyl, (j) carbamoyl which may be substituted by 1 or 2 C₁-C₆ alkyl(s), (k) a C₁-C₆ alkyl-sulfonyl,
and (1) phenyl,
Z² is CH or N, and
R¹⁰³ is
(1) a C₁-C₆ alkyl which may be substituted by 1 to 3 group(s) selected from
(a) hydroxy,
(b) a C₁-C₆ alkoxy,
(c) amino which may be substituted by 1 or 2 group(s) selected from the group consisting of a C₁-C₆ alkyl, a C₂-C₇ alkanoyl and a C₁-C₆ alkyl-sulfonyl,
(d) a C₁-C₆ alkoxy-carbonyl,
(e) a C₃-C₈ cycloalkyl which may be substituted by 1 to 3 group(s) selected from the group consisting of (i) hydroxy, (ii) an amino which may be substituted by C₁-C₆ alkyl(s), (iii) a C₂-C₇ alkanoyl-amino, (iv) a C₁-C₆ alkyl-sulfonylamino, (v) a C₁-C₆ alkyl which may be substituted by a group selected from hydroxy, a C₁-C₆ alkoxy, amino and a carbamoyl which may be substituted by C₁-C₆ alkyl(s), (vi) carboxy and (vii) a carbamoyl which may be substituted by C₁-C₆ alkyl(s),
(f) phenyl which may be substituted by 1 to 3 group(s) selected from the group consisting of the following (i) to (vi): (i) a halogen atom, (ii) a C₁-C₆ alkoxy, (iii) amino which may be substituted by 1 or 2 group(s) selected from the group consisting of a C₁-C₆ alkyl and a C₁-C₆ alkoxy-carbonyl, (iv) a C₁-C₆ alkoxy-carbonyl, (v) carbamoyl, and (vi) morpholinylcarbonyl, and
(g) a heterocyclic group, which is an unsaturated, or a partially or completely saturated monocyclic, bicyclic or tricyclic heterocyclic group containing 1 to 3 heteroatoms selected from nitrogen atom, oxygen atom and sulfur atom, and which may be substituted by 1 to 3 group(s) selected from the group consisting of the following (i) to (v): (i) a C₁-C₆ alkyl which may be substituted by 1 to 3 hydroxy(s), (ii) hydroxy, (iii) amino, (iv) a C₁-C₆ alkoxy-carbonyl, and (v) carbamoyl; or
(2) a C₃-C₈ cycloalkyl which may be substituted by 1 to 3 group(s) selected from
(a) a halogen atom,
(b) a C₁-C₆ alkyl which may be substituted by 1 to 3 group(s) selected from the group consisting of (i) hydroxy, (ii) a C₁-C₆ alkoxy, (iii) amino and (iv) a carbamoyl which may be substituted by a C₁-C₆ alkyl,
(c) hydroxy,
(d) a C₁-C₆alkoxy,
(e) amino which may be substituted by 1 or 2 group(s) selected from the group consisting of (i) a C₁-C₆ alkyl, (ii) a C₂-C₇ alkanoyl, (iii) a C₁-C₆ alkoxy-carbonyl and (iv) a C₁-C₆ alkyl-sulfonyl,
(f) carboxy,
(g) a C₂-C₇ alkanoyl-oxy,
(h) a C₁-C₆alkoxy-carbonyl, and
(i) carbamoyl which may be substituted by 1 or 2 group(s) selected from the group consisting of (i) a C₁-C₆ alkyl, (ii) a C₃-C₈ cycloalkyl and (iii) a heterocyclic group which is an unsaturated, or a partially or completely saturated monocyclic, bicyclic or tricyclic heterocyclic group containing 1 to 3 heteroatoms selected from nitrogen atom, oxygen atom and sulfur atom,
or a pharmaceutically acceptable salt thereof.

9. The compound according to Claim 8, wherein p is 0, or a pharmaceutically acceptable salt thereof.

10. The compound according to Claim 8 or 9, wherein R¹⁰¹ is a halogen atom, a C₁ - C₄ alkyl or a C₁ - C₄ alkoxy, or a pharmaceutically acceptable salt thereof.

11. The compound according to Claim 8 or 9, wherein R¹⁰² is
(1) 5- or 6-membered monocyclic heterocyclic group containing 1 to 3 heteroatoms selected from nitrogen atom, oxygen atom, and sulfur atom or
(2) a C₁-C₆ alkyl optionally substituted by 1 to 3 substituent(s), which may be the same or different from each other, and selected from the group consisting of cyano, a C₁-C₆ alkoxy, hydroxy, amino, carboxy, a carbamoyl optionally substituted by a C₁-C₆ alkyl, and phenyl,
or a pharmaceutically acceptable salt thereof.

12. The compound according to any one of Claims 8 to 11, wherein R¹⁰³ is
(1) a C₁-C₆ alkyl optionally substituted by 1 to 3 substituent(s) which may be the same or different from each other, and selected from (a) hydroxy, (b) a C₁-C₆ alkoxy, (c) amino, (d) a C₁-C₆ alkoxy-carbonyl, (e) a C₃-C₈ cycloalkyl, (f) phenyl and (g) a heterocyclic group, which is an unsaturated, or a partially or completely saturated monocyclic, bicyclic or tricyclic heterocyclic group containing 1 to 3 heteroatoms selected from nitrogen atom, oxygen atom, and sulfur atom, or
(2) a C₃-C₈ cycloalkyl optionally substituted by 1 to 3 substituent(s) which may be the same or different from each other, and selected from the group consisting of a halogen, a C₁-C₆ alkyl, hydroxy, a C₁-C₆ alkoxy, amino, carboxy, a C₂-C₇ alkanoyl-oxy, a C₁-C₆ alkoxy-carbonyl and carbamoyl,
or a pharmaceutically acceptable salt thereof.

13. A medicament comprising a compound of formula [Ia] or [Ic] as defined in any one of claims 1 to 12, or a pharmaceutically acceptable salt thereof.

14. A compound of formula [Ia] or [Ic] as defined in any one of claims 1 to 12, or a pharmaceutically acceptable salt thereof, for use in a method for treatment of the human or animal body by therapy.

15. A compound of formula [Ia] or [Ic] as defined in any one of claims 1 to 12, or a pharmaceutically acceptable salt thereof, for use in the prophylaxis or treatment of an inflammatory disease.

16. A compound according to claim 15 for a use as defined in claim 15, wherein the inflammatory disease is arthritis.

17. A compound of the formula: 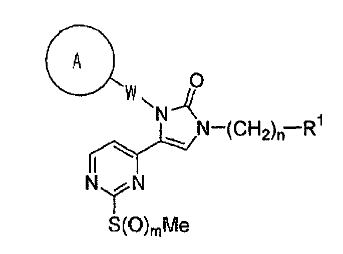 wherein m represents 1 or 2, and other symbols have the same meanings as defined in Claim 1.

## Patentansprüche

1. Verbindung mit der Formel [Ia]: 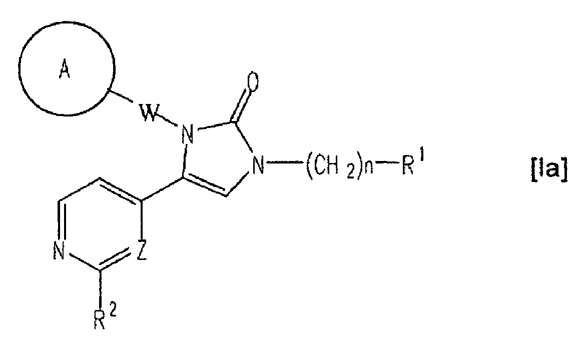 worin
• der Ring A für einen Benzolring oder einen Thiophenring steht und der Benzolring und der Thiophenring mit 1 bis 3 Substituenten substituiert sein können, die gleich oder verschieden sind und ausgewählt sind aus der Gruppe von
- einem Halogenatom;
- Nitro;
- einem C₁-C₆-Alkyl, das mit 1 bis 3 Gruppen substituiert sein kann, die aus Halogenatomen, Hydroxygruppen und Aminogruppen ausgewählt sind;
- einem C₁-C₆-Alkoxy, das mit 1 bis 3 Gruppen substituiert sein kann, die aus Hydroxygruppen und Aminogruppen ausgewählt sind;
- einer Aminogruppe, die mit 1 oder 2 Gruppen substituiert sein kann, die aus (a) einem C₁-C₆-Alkyl, das mit 1 bis 3 Gruppen substituiert sein kann, die aus C₁-C₆-Alkoxygruppen, Aminogruppen und Carboxygruppen ausgewählt sind, und (b) einem C₂-C₇-Alkanoyl ausgewählt sind;
- einem Carbamoyl, das mit 1 oder 2 C₁-C₆-Alkylgruppen substituiert sein kann; und
- Cyano,
• W für eine Einfachbindung oder ein C₁-C₄-Alkylen, das mit 1 oder 2 C₁-C₆-Alkylgruppen substituiert sein kann, steht,
• n für 0, 1, 2, 3 oder 4 steht,
• R¹ für
- ein Wasserstoffatom;
- ein C₁-C₆-Alkyl, das mit 1 bis 3 Gruppen substituiert sein kann, die aus (a) einem C₂-C₇-Alkinyl, (b) Cyano, (c) einem C₁-C₆-Alkoxy, (d) Hydroxy, (e) einer Aminogruppe, die mit 1 oder 2 Substituenten substituiert sein kann, die aus der Gruppe von einem C₁-C₆-Alkyl, einem C₂-C₇-Alkanoyl und einem C₁-C₆-Alkyl-sulfonyl ausgewählt sind, (f) Carboxy, (g) einem C₁-C₆-Alkoxy-carbonyl, (h) einem Carbamoyl, das mit 1 oder 2 C₁-C₆-Alkylgruppen substituiert sein kann, (i) Phenyl und (j) Naphthyl ausgewählt sind;
- ein C₃-C₈-Cycloalkyl, das mit 1 bis 3 Gruppen substituiert sein kann, die aus (a) Hydroxy, (b) einem C₁-C₆-Alkoxy, das mit 1 bis 3 C₁-C₆-Alkoxygruppen substituiert sein kann, (c) einer Aminogruppe, die mit 1 oder 2 Gruppen substituiert sein kann, die aus den im Folgenden angegebenen Gruppen (1) bis (5) ausgewählt sind: (1) ein C₁-C₆-Alkyl, (2) ein C₂-C₇-Alkanoyl, (3) ein C₁-C₆-Alkoxy-carbonyl, (4) ein Carbamoyl, das mit 1 oder 2 C₁-C₆-Alkygruppen substituiert sein kann, und (5) ein C₁-C₆-Alkyl-sulfonyl, (d) Carboxy, (e) einem C₁-C₆-Alkyl, das mit einer Gruppe substituiert sein kann, die aus der Gruppe von Hydroxy, einem C₁-C₆-Alkoxy und Amino ausgewählt ist, (f) einem Carbamoyl, das mit einer oder mehreren C₁-C₆-Alkylgruppen substituiert sein kann, ausgewählt sind;
- ein Phenyl, das mit 1 bis 3 Gruppen substituiert sein kann, die aus (a) einem Halogenatom, (b) Nitro, (c) einem C₁-C₆-Alkyl, das mit 1 bis 3 Gruppen substituiert sein kann, die aus der Gruppe von einem Halogenatom, Hydroxy, einem Amino, Carboxy und Phenylsulfonyl ausgewählt sind, (d) einem C₂-C₇-Alkenyl, (e) Cyano, (f) Hydroxy, (g) einem C₁-C₆-Alkoxy, das mit 1 bis 3 Gruppen substituiert sein kann, die aus der Gruppe von einem Halogenatom, Carboxy, einem C₁-C₆-Alkoxy-carbonyl, Carbamoyl, Phenyl und Morpholinylcarbonyl ausgewählt sind, (h) einer Aminogruppe, die mit 1 oder 2 Gruppen substituiert sein kann, die aus den im Folgenden angegebenen Gruppen (1) bis (4) ausgewählt sind: (1) ein C₁-C₆-Alkyl, (2) ein C₂-C₇-Alkanoyl, (3) ein Carbamoyl, das mit 1 oder 2 Gruppen substituiert sein kann, die aus der Gruppe von einem C₁-C₆-Alkyl und einem C₃-C₈-Cycloalkyl ausgewählt sind, und (4) ein C₁-C₆-Alkyl-sulfonyl, (i) einem C₂-C₇-Alkanoyl, (j) Carboxy, (k) einem C₁-C₆-Alkoxy-carbonyl, (l) einem Carbamoyl, das mit 1 oder 2 Gruppen substituiert sein kann, die aus den im Folgenden angegebenen Gruppen (1) und (2) ausgewählt sind: (1) ein C₁-C₆-Alkyl, das mit 1 bis 3 Hydroxygruppen substituiert sein kann, und (2) ein C₃-C₈-Cycloalkyl, (m) einem C₁-C₆-Alkyl-thio, (n) einem C₁-C₆-Alkyl-sulfinyl, (o) einem C₁-C₆-Alkyl-sulfonyl, (p) Phenyl, (q) Tetrazolyl und (r) einem Carbonyl, das mit einer heterocyclischen Gruppe substituiert ist, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus der Gruppe von einem C₁-C₆-Alkyl und einem C₁-C₆-Alkoxy-carbonyl ausgewählt sind, ausgewählt sind; oder
- eine heterocyclische Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus (a) einem Halogenatom, (b) Nitro, (c) einem C₁-C₆-Alkyl, das mit einer Gruppe substituiert sein kann, die aus der Gruppe von Hydroxy, einem C₁-C₆-Alkoxy, einem Carbamoyl, das mit einer oder mehreren C₁-C₆-Alkylgruppen und Carboxygruppen substituiert sein kann, ausgewählt ist, (d) Cyano, (e) Hydroxy, (f) Amino, (g) einem C₂-C₇-Alkanoyl, (h) Carboxy, (i) einem C₁-C₆-Alkoxycarbonyl, (j) einem Carbamomyl, das mit 1 oder 2 C₁-C₆-Alkylgruppen substituiert sein kann, (k) einem C₁-C₆-Alkyl-sulfonyl und (1) Phenyl ausgewählt sind, steht,
• Z für CH oder N steht,
• R² für ein Wasserstoffatom, -NR³R⁴, -OR⁵, -COR⁶ oder -CHR⁷R⁸ steht, wobei R³ bis R⁸ jeweils unabhängig voneinander für
- ein Wasserstoffatom;
- ein C₁-C₆-Alkyl, das mit 1 bis 3 Gruppen substituiert sein kann, die aus (a) Hydroxy, (b) einem C₁-C₆-Alkoxy, (c) einer Aminogruppe, die mit 1 oder 2 Gruppen substituiert sein kann, die aus der Gruppe von einem C₁-C₆-Alkyl, einem C₂-C₇-Alkanoyl und einem C₁-C₆-Alkyl-sulfonyl ausgewählt sind, (d) einem C₁-C₆-Alkoxy-carbonyl, (e) einem C₃-C₈-Cycloalkyl, das mit 1 bis 3 Gruppen substituiert sein kann, die aus der Gruppe von (1) Hydroxy, (2) einer Aminogruppe, die mit einer oder mehreren C₁-C₆-Alkylgruppen substituiert sein kann, (3) einem C₂-C₇-Alkanoyl-amino, (4) einem C₁-C₆-Alkyl-sulfonylamino, (5) einem C₁-C₆-Alkyl, das mit einer Gruppe substituiert sein kann, die aus Hydroxy, einem C₁-C₆-Alkoxy, Amino und einem Carbamoyl, das mit einer oder mehreren C₁-C₆-Alkylgruppen substituiert sein kann, ausgewählt ist, (6) Carboxy und (7) einem Carbamoyl, das mit einer oder mehreren C₁-C₆-Alkylgruppen substituiert sein kann, ausgewählt sind, (f) einem Phenyl, das mit 1 bis 3 Gruppen substituiert sein kann, die aus den im Folgenden angegebenen Gruppen (1) bis (6) ausgewählt sind: (1) ein Halogenatom, (2) ein C₁-C₆-Alkoxy, (3) eine Aminogruppe, die mit 1 oder 2 Gruppen substituiert sein kann, die aus der Gruppe von einem C₁-C₆-Alkyl und einem C₁-C₆-Alkoxy-carbonyl ausgewählt sind, (4) ein C₁-C₆-Alkoxy-carbonyl, (5) Carbamoyl und (6) Morpholinylcarbonyl, und (g) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus den im Folgenden angegebenen Gruppen (1) bis (5) ausgewählt sind: (1) ein C₁-C₆-Alkyl, das mit 1 bis 3 Hydroxygruppen substituiert sein kann, (2) Hydroxy, (3) Amino, (4) ein C₁-C₆-Alkoxy-carbonyl und (5) Carbamoyl, ausgewählt sind;
- ein C₂-C₇-Alkenyl;
- ein C₂-C₇-Alkinyl;
- Hydroxy;
- ein C₁-C₆-Alkoxy;
- eine Aminogruppe, die mit 1 oder 2 Gruppen substituiert sein kann, die aus (a) einem C₁-C₆-Alkyl, das mit 1 bis 3 Gruppen substituiert sein kann, die aus der Gruppe von (1) Hydroxy, (2) einem C₁-C₆-Alkoxy und (3) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind, (b) einem C₃-C₈-Cycloalkyl, das mit 1 bis 3 Hydroxygruppen substituiert sein kann und (c) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind;
- ein C₂-C₇-Alkanoyl, das mit 1 bis 3 Gruppen substituiert sein kann, die aus (a) Hydroxy, (b) einem C₁-C₆-Alkoxy, (c) einer Aminogruppe, die mit 1 oder 2 Gruppen substituiert sein kann, die aus der Gruppe von einem C₁-C₆-Alkyl und einem C₂-C₇-Alkanoyl ausgewählt sind, und (d) einem C₁-C₆-Alkoxy-carbonyl ausgewählt sind;
- ein Carbamoyl, das mit 1 oder 2 Gruppen substituiert sein kann, die aus (a) einem C₁-C₆-Alkyl, (b) einem C₃-C₈-Cycloalkyl und (c) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind;
- ein C₁-C₆-Alkoxy-oxalyl;
- ein C₁-C₆-Alkyl-sulfonyl;
- ein C₃-C₈-Cycloalkyl, das mit 1 bis 3 Gruppen substituiert sein kann, die aus (a) einem Halogenatom, (b) einem C₁-C₆-Alkyl, das mit 1 bis 3 Gruppen substituiert sein kann, die aus der Gruppe von (1) Hydroxy, (2) einem C₁-C₆-Alkoxy, (3) Amino und (4) einem Carbamoyl, das mit einem C₁-C₆-Alkyl substituiert sein kann, ausgewählt sind, (c) Hydroxy, (d) einem C₁-C₆-Alkoxy, (e) einer Aminogruppe, die mit 1 oder 2 Gruppen substituiert sein kann, die aus der Gruppe von (1) einem C₁-C₆-Alkyl, (2) einem C₂-C₇-Alkanoyl, (3) einem C₁-C₆-Alkoxy-carbonyl und (4) einem C₁-C₆-Alkyl-sulfonyl ausgewählt sind, (f) Carboxy, (g) einem C₂-C₇-Alkanoyl-oxy, (h) einem C₁-C₆-Alkoxy-carbonyl und (i) einem Carbamoyl, das mit 1 oder 2 Gruppen substituiert sein kann, die aus der Gruppe von (1) einem C₁-C₆-Alkyl, (2) einem C₃-C₈-Cycloalkyl und (3) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind;
- ein Phenyl, das mit 1 bis 3 Gruppen substituiert sein kann, die aus (a) einem C₁-C₆-Alkyl, (b) Hydroxy, (c) einem C₁-C₆-Alkoxy, (d) einem Halogenatom und (e) einer Aminogruppe, die mit 1 oder 2 C₁-C₆-Alkylgruppen substituiert sein kann, ausgewählt sind, ausgewählt sind;
- eine heterocyclische Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus (a) einem C₁-C₆-Alkyl, das mit 1 bis 3 Gruppen substituiert sein kann, die aus der Gruppe von (1) Phenyl, (2) Hydroxy, (3) einem C₁-C₆-Alkoxy, (4) Amino und (5) einem Carbamoyl, das mit einem C₁-C₆-Alkyl substituiert sein kann, ausgewählt sind, (b) Carboxy, (c) einem C₁-C₆-Alkoxycarbonyl, (d) einem C₂-C₇-Alkanoyl, (e) einem C₁-C₆-Alkyl-sulfonyl und (f) Oxo ausgewählt sind;
- ein Carbonyl, das mit einem C₃-C₈-Cycloalkyl substituiert ist, das mit 1 bis 3 Gruppen substituiert sein kann, die aus (a) Hydroxy, (b) einem C₁-C₆-Alkoxy, (c) einer Aminogruppe, die mit 1 oder 2 Gruppen substituiert sein kann, die aus der Gruppe von (1) einem C₁-C₆-Alkyl und (2) einem C₂-C₇-Alkanoyl ausgewählt sind, und (d) einem C₁-C₆-Alkoxy-carbonyl ausgewählt sind;
- ein Carbonyl, das mit einem Phenyl substituiert ist, wobei dieses mit 1 bis 3 Gruppen substituiert sein kann, die aus (a) einem Halogenatom, (b) Hydroxy, (c) einem C₁-C₆-Alkoxy und (d) einer Aminogruppe, die mit 1 oder 2 Gruppen substituiert sein kann, die aus der Gruppe von einem C₁-C₆-Alkyl und einem C₂-C₇-Alkanoyl ausgewählt sind, ausgewählt sind; oder
- ein Carbonyl, das mit einer heterocyclischen Gruppe substituiert ist, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus (a) einem Halogenatom, (b) einem C₁-C₆-Alkyl, (c) Hydroxy, (d) einer Aminogruppe, die mit 1 oder 2 C₁-C₆-Alkylgruppen substituiert sein kann, (e) einem C₂-C₇-Alkanoyl und (f) Oxo ausgewählt sind, oder ein pharmazeutisch akzeptables Salz hiervon.

2. Verbindung nach Anspruch 1, wobei der Ring A für einen Benzolring steht, der mit 1 bis 3 Substituenten substituiert sein kann, die gleich oder verschieden sind und ausgewählt sind aus der Gruppe von
- einem Halogenatom;
- Nitro;
- einem C₁-C₆-Alkyl, das mit 1 bis 3 Gruppen substituiert sein kann, die aus Halogenatomen, Hydroxygruppen und Aminogruppen ausgewählt sind;
- einem C₁-C₆-Alkoxy, das mit 1 bis 3 Gruppen substituiert sein kann, die aus Hydroxygruppen und Aminogruppen ausgewählt sind;
- einer Aminogruppe, die mit 1 oder 2 Gruppen substituiert sein kann, die aus (a) einem C₁-C₆-Alkyl, das mit 1 bis 3 Gruppen substituiert sein kann, die aus C₁-C₆-Alkoxygruppen, Aminogruppen und Carboxygruppen ausgewählt sind, und (b) einem C₂-C₇-Alkanoyl ausgewählt sind; und
- Cyano,
und W für eine Einfachbindung steht,
oder ein pharmazeutisch akzeptables Salz hiervon.

3. Verbindung nach Anspruch 1 oder 2, wobei n 0 oder 1 ist, oder ein pharmazeutisch akzeptables Salz hiervon.

4. Verbindung nach einem der Ansprüche 1 bis 3, wobei
• n 0 ist und R¹ für ein C₁-C₆-Alkyl steht, das mit 1 bis 3 Gruppen substituiert sein kann, die aus (a) einem C₂-C₇-Alkinyl, (b) Cyano, (c) einem C₁-C₆-Alkoxy, (d) Hydroxy, (e) einer Aminogruppe, die mit 1 oder 2 Substituenten substituiert sein kann, die aus der Gruppe von einem C₁-C₆-Alkyl, einem C₂-C₇-Alkanoyl und einem C₁-C₆-Alkyl-sulfonyl ausgewählt sind, (f) Carboxy, (g) einem C₁-C₆-Alkoxy-carbonyl, (h) einem Carbamoyl, das mit 1 oder 2 C₁-C₆-Alkylgruppen substituiert sein kann, (i) Phenyl und (j) Naphthyl ausgewählt sind, oder
• n 1 ist und R¹ für
(A) ein C₃-C₈-Cycloalkyl, das mit 1 bis 3 Gruppen substituiert sein kann, die aus (a) Hydroxy, (b) einem C₁-C₆-Alkoxy, das mit 1 bis 3 C₁-C₆-Alkoxygruppen substituiert sein kann, (c) einer Aminogruppe, die mit 1 oder 2 Gruppen substituiert sein kann, die aus den im Folgenden angegebenen Gruppen (1) bis (5) ausgewählt sind: (1) ein C₁-C₆-Alkyl, (2) ein C₂-C₇-Alkanoyl, (3) ein C₁-C₆-Alkoxy-carbonyl, (4) ein Carbamoyl, das mit 1 oder 2 C₁-C₆-Alkylgruppen substituiert sein kann, und (5) ein C₁-C₆-Alkylsulfonyl, (d) Carboxy, (e) einem C₁-C₆-Alkyl, das mit einer Gruppe substituiert sein kann, die aus der Gruppe von Hydroxy, einem C₁-C₆-Alkoxy und Amino ausgewählt ist, (f) einem Carbamoyl, das mit einer oder mehreren C₁-C₆-Alkylgruppen substituiert sein kann, oder
(B) ein Phenyl, das mit 1 bis 3 Gruppen substituiert sein kann, die aus (a) einem Halogenatom, (b) Nitro, (c) einem C₁-C₆-Alkyl, das mit 1 bis 3 Gruppen substituiert sein kann, die aus der Gruppe von einem Halogenatom, Hydroxy, Amino, Carboxy und Phenylsulfonyl ausgewählt sind, (d) einem C₂-C₇-Alkenyl, (e) Cyano, (f) Hydroxy, (g) einem C₁-C₆-Alkoxy, das mit 1 bis 3 Gruppen substituiert sein kann, die aus der Gruppe von einem Halogenatom, Carboxy, einem C₁-C₆-Alkoxy-carbonyl, Carbamoyl, Phenyl und Morpholinylcarbonyl ausgewählt sind, (h) einer Aminogruppe, die mit 1 oder 2 Gruppen substituiert sein kann, die aus den im Folgenden angegebenen Gruppen (1) bis (4) ausgewählt sind: (1) ein C₁-C₆-Alkyl, (2) ein C₂-C₇-Alkanoyl, (3) ein Carbamoyl, das mit 1 oder 2 Gruppen substituiert sein kann, die aus der Gruppe von einem C₁-C₆-Alkyl und einem C₃-C₈-Cycloalkyl ausgewählt sind, und (4) ein C₁-C₆-Alkylsulfonyl, (i) einem C₂-C₇-Alkanoyl, (j) Carboxy, (k) einem C₁-C₆-Alkoxy-carbonyl, (l) einem Carbamoyl, das mit 1 oder 2 Gruppen substituiert sein kann, die aus den im Folgenden angegebenen Gruppen (1) und (2) ausgewählt sind: (1) ein C₁-C₆-Alkyl, das mit 1 bis 3 Hydroxygruppen substituiert sein kann, und (2) ein C₃-C₈-Cycloalkyl, (m) einem C₁-C₆-Alkyl-thio, (n) einem C₁-C₆-Alkyl-sulfinyl, (o) einem C₁-C₆-Alkyl-sulfonyl, (p) Phenyl, (q) Tetrazolyl und (r) einem Carbonyl, das mit einer heterocyclischen Gruppe substituiert ist, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus der Gruppe von einem C₁-C₆-Alkyl und einem C₁-C₆-Alkoxy-carbonyl ausgewählt sind, ausgewählt sind, steht,
oder ein pharmazeutisch akzeptables Salz hiervon.

5. Verbindung nach einem der Ansprüche 1 bis 4, wobei R² für -NR³R⁴ oder -OR⁵ steht, oder ein pharmazeutisch akzeptables Salz hiervon.

6. Verbindung nach einem der Ansprüche 1 bis 4, wobei R² für -NHR⁴ steht und R⁴ für
- ein C₁-C₆-Alkyl, das mit 1 bis 3 Gruppen substituiert sein kann, die aus
(a) Hydroxy,
(b) einem C₁-C₆-Alkoxy,
(c) einer Aminogruppe, die mit 1 oder 2 Gruppen substituiert sein kann, die aus der Gruppe von einem C₁-C₆-Alkyl, einem C₂-C₇-Alkanoyl und einem C₁-C₆-Alkylsulfonyl ausgewählt sind,
(d) einem C₁-C₆-Alkoxy-carbonyl,
(e) einem C₃-C₈-Cycloalkyl, das mit 1 bis 3 Gruppen substituiert sein kann, die aus der Gruppe von (1) Hydroxy, (2) einer Aminogruppe, die mit einer oder mehreren C₁-C₆-Alkylgruppen substituiert sein kann, (3) einem C₂-C₇-Alkanoyl-amino, (4) einem C₁-C₆-Alkyl-sulfonylamino, (5) einem C₁-C₆-Alkyl, das mit einer Gruppe substituiert sein kann, die aus Hydroxy, einem C₁-C₆-Alkoxy, Amino und einem Carbamoyl, das mit einer oder mehreren C₁-C₆-Alkylgruppen substituiert sein kann, ausgewählt ist, (6) Carboxy und (7) einem Carbamoyl, das mit einer oder mehreren C₁-C₆-Alkylgruppen substituiert sein kann, ausgewählt sind,
(f) einem Phenyl, das mit 1 bis 3 Gruppen substituiert sein kann, die aus den im Folgenden angegebenen Gruppen (1) bis (6) ausgewählt sind: (1) ein Halogenatom, (2) ein C₁-C₆-Alkoxy, (3) eine Aminogruppe, die mit 1 oder 2 Gruppen substituiert sein kann, die aus der Gruppe von einem C₁-C₆-Alkyl und einem C₁-C₆-Alkoxy-carbonyl ausgewählt sind, (4) ein C₁-C₆-Alkoxy-carbonyl, (5) Carbamoyl und (6) Morpholinylcarbonyl, und
(g) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus den im Folgenden angegebenen Gruppen (1) bis (5) ausgewählt sind: (1) ein C₁-C₆-Alkyl, das mit 1 bis 3 Hydroxygruppen substituiert sein kann, (2) Hydroxy, (3) Amino, (4) ein C₁-C₆-Alkoxy-carbonyl und (5) Carbamoyl, ausgewählt sind;
- ein C₂-C₇-Alkenyl;
- ein C₂-C₇-Alkanoyl, das mit 1 bis 3 Gruppen substituiert sein kann, die aus (a) Hydroxy, (b) einem C₁-C₆-Alkoxy, (c) einer Aminogruppe, die mit 1 oder 2 Gruppen substituiert sein kann, die aus der Gruppe von einem C₁-C₆-Alkyl und einem C₂-C₇-Alkanoyl ausgewählt sind, und (d) einem C₁-C₆-Alkoxy-carbonyl ausgewählt sind;
- ein Carbamoyl, das mit 1 oder 2 Gruppen substituiert sein kann, die aus (a) einem C₁-C₆-Alkyl, (b) einem C₃-C₈-Cycloalkyl und (c) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind;
- ein C₃-C₈-Cycloalkyl, das mit 1 bis 3 Gruppen substituiert sein kann, die aus (a) einem Halogenatom, (b) einem C₁-C₆-Alkyl, das mit 1 bis 3 Gruppen substituiert sein kann, die aus der Gruppe von (1) Hydroxy, (2) einem C₁-C₆-Alkoxy, (3) Amino und (4) einem Carbamoyl, das mit einem C₁-C₆-Alkyl substituiert sein kann, ausgewählt sind, (c) Hydroxy, (d) einem C₁-C₆-Alkoxy, (e) einer Aminogruppe, die mit 1 oder 2 Gruppen substituiert sein kann, die aus der Gruppe von (1) einem C₁-C₆-Alkyl, (2) einem C₂-C₇-Alkanoyl, (3) einem C₁-C₆-Alkoxy-carbonyl und (4) einem C₁-C₆-Alkylsulfonyl ausgewählt sind, (f) Carboxy, (g) einem C₂-C₇-Alkanoyl-oxy, (h) einem C₁-C₆-Alkoxy-carbonyl und (i) einem Carbamoyl, das mit 1 oder 2 Gruppen substituiert sein kann, die aus der Gruppe von (1) einem C₁-C₆-Alkyl, (2) einem C₃-C₈-Cycloalkyl und (3) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind, ausgewählt sind;
- ein Phenyl, das mit 1 bis 3 Gruppen substituiert sein kann, die aus (a) einem C₁-C₆-Alkyl, (b) Hydroxy, (c) einem C₁-C₆-Alkoxy, (d) einem Halogenatom und (e) einer Aminogruppe, die mit 1 oder 2 C₁-C₆-Alkylgruppen substituiert sein kann, ausgewählt sind;
- eine heterocyclische Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus (a) einem C₁-C₆-Alkyl, das mit 1 bis 3 Gruppen substituiert sein kann, die aus der Gruppe von (1) Phenyl, (2) Hydroxy, (3) einem C₁-C₆-Alkoxy, (4) Amino und (5) einem Carbamoyl, das mit einem C₁-C₆-Alkyl substituiert sein kann, ausgewählt sind, (b) Carboxy, (c) einem C₁-C₆-Alkoxy-carbonyl, (d) einem C₂-C₇-Alkanoyl, (e) einem C₁-C₆-Alkyl-sulfonyl und (f) Oxo ausgewählt sind;
- ein Carbonyl, das mit einem C₃-C₈-Cycloalkyl substituiert ist, das mit 1 bis 3 Gruppen substituiert sein kann, die aus (a) Hydroxy, (b) einem C₁-C₆-Alkoxy, (c) einer Aminogruppe, die mit 1 oder 2 Gruppen substituiert sein kann, die aus der Gruppe von (1) einem C₁-C₆-Alkyl und (2) einem C₂-C₇-Alkanoyl ausgewählt sind, und (d) einem C₁-C₆-Alkoxy-carbonyl ausgewählt sind; oder
- ein Carbonyl, das mit einer heterocyclischen Gruppe substituiert ist, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus (a) einem Halogenatom, (b) einem C₁-C₆-Alkyl, (c) Hydroxy, (d) einer Aminogruppe, die mit 1 oder 2 C₁-C₆-Alkylgruppen substituiert sein kann, (e) einem C₂-C₇-Alkanoyl und (f) Oxo ausgewählt sind, steht,
oder ein pharmazeutisch akzeptables Salz hiervon.

7. Verbindung nach Anspruch 1, wobei
- der Ring A für einen Benzolring steht, der mit 1 oder 2 Substituenten substituiert sein kann, wobei diese gleich oder verschieden sind und ausgewählt sind aus der Gruppe von einem Halogenatom, einem C₁-C₆-Alkyl, einem C₁-C₆-Alkoxy, einer Aminogruppe, die optional mit einer oder mehreren C₁-C₆-Alkylgruppen substituiert ist, und Cyano ausgewählt sind,
- W für eine Einfachbindung steht,
- n 0 oder 1 ist,
- R¹ für
(1) ein Wasserstoffatom;
(2) ein C₁-C₆-Alkyl, das optional mit einer oder mehreren Gruppen substituiert ist, die aus der Gruppe von einem Phenyl, einem C₁-C₆-Alkoxy, einem C₁-C₆-Alkyl-amino, einem Di-C₁-C₆-alkyl-amino, einem C₂-C₇-Alkanoyl-amino, einem C₁-C₆-Alkyl-sulfonylamino, einem Carbamoyl, das optional mit einer oder mehreren C₁-C₆-Alkylgruppen, Hydroxy, Carboxy und Cyano substituiert ist, ausgewählt sind,
(3) ein C₃-C₈-Cycloalkyl, das optional mit einer oder mehreren Gruppen substituiert ist, die aus den im Folgenden angegebenen Gruppen (i) bis (v) ausgewählt sind: (i) Hydroxy, (ii) ein C₁-C₆-Alkoxy, das optional mit einer oder mehreren C₁-C₆-Alkoxygruppen substituiert ist, (iii) eine Aminogruppe, die optional mit einer oder mehreren Gruppen substituiert ist, die aus der Gruppe von einem C₁-C₆-Alkyl, einem C₂-C₇-Alkanoyl und einem C₁-C₆-Alkyl-sulfonyl ausgewählt sind, (iv) ein Carbamoyl, das optional mit einer oder mehreren C₁-C₆-Alkylgruppen substituiert ist, und (v) ein C₁-C₆-Alkyl, das optional mit einer oder mehreren Gruppen substituiert ist, die aus der Gruppe von Hydroxy, einem C₁-C₆-Alkoxy und Amino ausgewählt sind,
(4) ein Phenyl, das optional mit einer oder mehreren Gruppen substituiert ist, die aus den im Folgenden angegebenen Gruppen (i) bis (vi) ausgewählt sind: (i) ein Halogenatom, (ii) ein C₁-C₆-Alkyl, das optional mit einer oder mehreren Gruppen substituiert ist, die aus der Gruppe von einem Halogenatom, Hydroxy und Phenylsulfonyl ausgewählt sind, (iii) Cyano, (iv) ein C₁-C₆-Alkoxy, (v) eine Aminogruppe, die optional mit einer oder mehreren Gruppen substituiert ist, die aus der Gruppe von einem C₁-C₆-Alkyl und einem C₁-C₆-Alkylsulfonyl ausgewählt sind, (vi) ein Carbonyl, das mit einer heterocyclischen Gruppe substituiert ist, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, oder
(5) eine heterocyclische Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit einer oder mehreren Gruppen substituiert sein kann, die aus den im Folgenden angegebenen Gruppen (i) bis (iv) ausgewählt sind: (i) ein C₁-C₆-Alkoxy-carbonyl, (ii) ein C₁-C₆-Alkyl, das optional mit einer oder mehreren Gruppen substituiert ist, die aus der Gruppe von Hydroxy, einem C₁-C₆-Alkoxy und einem Carbamoyl, das optional mit einer oder mehreren C₁-C₆-Alkylgruppen substituiert ist, ausgewählt sind, (iii) ein C₂-C₇-Alkanoyl und (iv) ein C₁-C₆-Alkyl-sulfonyl, steht,
- Z für CH oder N steht,
- R² für ein Wasserstoffatom, -NR³R⁴, -OR⁵, -COR⁶ oder -CHR⁷R⁸ steht, wobei R³ bis R⁸ jeweils unabhängig voneinander für:
(1) ein Wasserstoffatom,
(2) ein C₁-C₆-Alkyl, das optional mit einer oder mehreren Gruppen substituiert ist, die aus den im Folgenden angegebenen Gruppen (i) bis (vii) ausgewählt sind:
(i) Hydroxy,
(ii) ein C₁-C₆-Alkoxy,
(iii) eine Aminogruppe, die optional mit einer oder mehreren Gruppen substituiert ist, die aus der Gruppe von einem C₁-C₆-Alkyl, einem C₂-C₇-Alkanoyl und einem C₁-C₆-Alkyl-sulfonyl ausgewählt sind,
(iv) ein C₁-C₆-Alkoxy-carbonyl,
(v) ein C₃-C₈-Cycloalkyl, das optional mit einer oder mehreren Gruppen substituiert ist, die aus den im Folgenden angegebenen Gruppen (a) bis (g) ausgewählt sind:
(a) Hydroxy,
(b) eine Aminogruppe, die optional mit einer oder mehreren C₁-C₆-Alkylgruppen substituiert ist,
(c) ein C₂-C₇-Alkanoyl-amino,
(d) ein C₁-C₆-Alkyl-sulfonylamino,
(e) ein C₁-C₆-Alkyl, das optional mit einer oder mehreren Gruppen substituiert ist, die aus der Gruppe von Hydroxy, einem C₁-C₆-Alkoxy, einem Amino, einem Carbamoyl, das optional mit einer oder mehreren C₁-C₆-Alkylgruppen substituiert ist,
(f) Carboxy und
(g) ein Carbamoyl, das optional mit einer oder mehreren C₁-C₆-Alkylgruppen substituiert ist,
(vi) ein Phenyl, das optional mit einer oder mehreren Gruppen substituiert ist, die aus der Gruppe von einem Halogenatom, einem C₁-C₆-Alkoxy und Morpholinylcarbonyl ausgewählt sind, und
(vii) eine heterocyclische Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit einer oder mehreren C₁-C₆-Alkylgruppen substituiert sein kann,
(3) ein C₂-C₇-Alkenyl,
(4) ein C₁-C₆-Alkoxy,
(5) ein C₂-C₇-Alkanoyl, das optional mit einer oder mehreren Gruppen substituiert ist, die aus den im Folgenenden angegebenen Gruppen (i) bis (iv) ausgewählt sind:
(i) Hydroxy,
(ii) ein C₁-C₆-Alkoxy,
(iii) eine Aminogruppe, die optional mit einer oder mehreren Gruppen substituiert ist, die aus der Gruppe von einem C₁-C₆-Alkyl und einem C₂-C₇-Alkanoyl ausgewählt sind,
(iv) ein C₁-C₆-Alkoxy-carbonyl,
(6) ein Carbamoyl, das optional mit einer oder mehreren C₁-C₆-Alkylgruppen substituiert ist,
(7) ein C₁-C₆-Alkoxy-oxalyl,
(8) ein C₃-C₈-Cycloalkyl, das optional mit einer oder mehreren Gruppen substituiert ist, die aus den im Folgenden angegebenen Gruppen (i) bis (vii) ausgewählt sind:
(i) ein Halogenatom,
(ii) Hydroxy,
(iii) ein C₁-C₆-Alkoxy,
(iv) eine Aminogruppe, die optional mit einer oder mehreren Gruppen substituiert ist, die aus der Gruppe von einem C₁-C₆-Alkyl, einem C₂-C₇-Alkanoyl, einem C₁-C₆-Alkoxy-carbonyl und C₁-C₆-Alkyl-sulfonyl ausgewählt sind,
(v) ein C₁-C₆-Alkyl, das optional mit einer oder mehreren Gruppen substituiert ist, die aus der Gruppe von Hydroxy, einem C₁-C₆-Alkoxy, Amino, einem Carbamoyl, das optional mit einer oder mehreren C₁-C₆-Alkylgruppen substituiert ist,
(vi) ein C₂-C₇-Alkanoyl-oxy und
(vii) ein Carbamoyl, das optional mit einer oder mehreren C₁-C₆-Alkylgruppen substituiert ist,
(9) ein Phenyl, das optional mit einer oder mehreren Gruppen substituiert ist, die aus der Gruppe von einem Halogenatom und einem C₁-C₆-Alkoxy ausgewählt sind,
(10) eine heterocyclische Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit einer oder mehreren Gruppen substituiert sein kann, die aus den im Folgenden angegebenen Gruppen (i) bis (v) ausgewählt sind:
(i) ein C₁-C₆-Alkyl, das optional mit einer oder mehreren Gruppen substituiert ist, die aus der Gruppe von Phenyl, Hydroxy, einem C₁-C₆-Alkoxy, Amino und einem Carbamoyl, das optional mit einer oder mehreren C₁-C₆-Alkylgruppen substituiert ist, ausgewählt sind,
(ii) ein C₁-C₆-Alkoxy-carbonyl,
(iii) ein C₂-C₇-Alkanoyl,
(iv) ein C₁-C₆-Alkyl-sulfonyl,
(v) Oxo,
(11) ein Carbonyl, das mit einem C₃-C₈-Cycloalkyl substituiert ist, das optional mit einer oder mehreren Gruppen substituiert ist, die aus der Gruppe von Hydroxy, Amino und einem C₂-C₇-Alkanoyl-amino ausgewählt sind, oder
(12) ein Carbonyl, das mit einer heterocyclischen Gruppe substituiert ist, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, stehen,
oder ein pharmazeutisch akzeptables Salz hiervon.

8. Verbindung nach Anspruch 1 mit der Formel [Ic]: 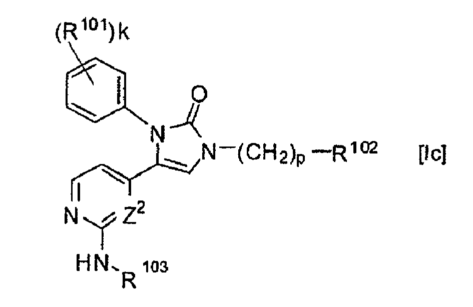 worin
die einzelnen Reste R¹⁰¹ gleich oder verschieden sind und aus gewählt sind aus der Gruppe von
(1) einem Halogenatom;
(2) Nitro;
(3) einem C₁-C₆-Alkyl, das mit 1 bis 3 Gruppen substituiert sein kann, die aus Halogenatomen, Hydroxygruppen und Aminogruppen ausgewählt sind;
(4) einem C₁-C₆-Alkoxy, das mit 1 bis 3 Gruppen substituiert sein kann, die aus Hydroxygruppen und Aminogruppen ausgewählt sind;
(5) einer Aminogruppe, die mit 1 oder 2 Gruppen substituiert sein kann, die aus (a) einem C₁-C₆-Alkyl, das mit 1 bis 3 Gruppen substituiert sein kann, die aus C₁-C₆-Alkoxygruppen, Aminogruppen und Carboxygruppen ausgewählt sind, und (b) einem C₂-C₇-Alkanoyl ausgewählt sind; und
(6) Cyano,
k für 0 bis 3 steht,
p 0 oder 1 ist;
R¹⁰² für
(1) ein C₁-C₆-Alkyl, das mit 1 bis 3 Gruppen substituiert sein kann, die aus (a) einem C₂-C₇-Alkinyl, (b) Cyano, (c) einem C₁-C₆-Alkoxy, (d) Hydroxy, (e) einer Aminogruppe, die mit 1 oder 2 Substituenten substituiert sein kann, die aus der Gruppe von einem C₁-C₆-Alkyl, einem C₂-C₇-Alkanoyl und einem C₁-C₆-Alkyl-sulfonyl ausgewählt sind, (f) Carboxy, (g) einem C₁-C₆-Alkoxy-carbonyl, (h) einem Carbamoyl, das mit 1 oder 2 C₁-C₆-Alkylgruppen substituiert sein kann, (i) Phenyl und (j) Naphthyl ausgewählt sind; oder
(2) eine heterocyclische Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus (a) einem Halogenatom, (b) Nitro, (c) einem C₁-C₆-Alkyl, das mit einer Gruppe substituiert sein kann, die aus der Gruppe von Hydroxy, einem C₁-C₆-Alkoxy, einem Carbamoyl, das mit einer oder mehreren C₁-C₆-Alkylgruppen und Carboxygruppen substituiert sein kann, ausgewählt ist, (d) Cyano, (e) Hydroxy, (f) Amino, (g) einem C₂-C₇-Alkanoyl, (h) Carboxy, (i) einem C₁-C₆-Alkoxy-carbonyl, (j) einem Carbamoyl, das mit 1 oder 2 C₁-C₆-Alkylgruppen substituiert sein kann, (k) einem C₁-C₆-Alkyl-sulfonyl und (1) Phenyl ausgewählt sind, steht,
2² für CH oder N steht und
R¹⁰³ für
(1) ein C₁-C₆-Alkyl, das mit 1 bis 3 Gruppen substituiert sein kann, die ausgewählt sind aus
(a) Hydroxy,
(b) einem C₁-C₆-Alkoxy,
(c) einer Aminogruppe, die mit 1 oder 2 Gruppen substituiert sein kann, die aus der Gruppe von einem C₁-C₆-Alkyl, einem C₂-C₇-Alkanoyl und einem C₁-C₆-Alkylsulfonyl ausgewählt sind,
(d) einem C₁-C₆-Alkoxy-carbonyl,
(e) einem C₃-C₈-Cycloalkyl, das mit 1 bis 3 Gruppen substituiert sein kann, die aus der Gruppe von (i) Hydroxy, (ii) einer Aminogruppe, die mit einer oder mehreren C₁-C₆-Alkylgruppen substituiert sein kann, (iii) einem C₂-C₇-Alkanoyl-amino, (iv) einem C₁-C₆-Alkyl-sulfonylamino, (v) einem C₁-C₆-Alkyl, das mit einer Gruppe substituiert sein kann, die aus Hydroxy, einem C₁-C₆-Alkoxy, Amino und einem Carbamoyl, das mit einer oder mehreren C₁-C₆-Alkylgruppen substituiert sein kann, ausgewählt ist, (vi) Carboxy und (vii) einem Carbamoyl, das mit einer oder mehreren C₁-C₆-Alkylgruppen substituiert sein kann, ausgewählt sind,
(f) Phenyl, das mit 1 bis 3 Gruppen substituiert sein kann, die aus den im Folgenden angegebenen Gruppen (i) bis (vi) ausgewählt sind: (i) ein Halogenatom, (ii) ein C₁-C₆-Alkoxy, (iii) eine Aminogruppe, die mit 1 oder 2 Gruppen substituiert sein kann, die aus der Gruppe von einem C₁-C₆-Alkyl und einem C₁-C₆-Alkoxycarbonyl ausgewählt sind, (iv) ein C₁-C₆-Alkoxycarbonyl, (v) Carbamoyl und (vi) Morpholinylcarbonyl und
(g) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, und wobei diese mit 1 bis 3 Gruppen substituiert sein kann, die aus den im Folgenden angegebenen Gruppen (i) bis (v) ausgewählt sind: (i) ein C₁-C₆-Alkyl, das mit 1 bis 3 Hydroxygruppen substituiert sein kann, (ii) Hydroxy, (iii) Amino, (iv) ein C₁-C₆-Alkoxy-carbonyl und (v) Carbamoyl; oder
(2) ein C₃-C₈-Cycloalkyl, das mit 1 bis 3 Gruppen substituiert sein kann, die ausgewählt sind aus
(a) einem Halogenatom,
(b) einem C₁-C₆-Alkyl, das mit 1 bis 3 Gruppen substituiert sein kann, die aus der Gruppe von (i) Hydroxy, (ii) einem C₁-C₆-Alkoxy, (iii) Amino und (iv) einem Carbamoyl, das mit einem C₁-C₆-Alkyl substituiert sein kann, ausgewählt sind,
(c) Hydroxy,
(d) einem C₁-C₆-Alkoxy,
(e) einer Aminogruppe, die mit 1 oder 2 Gruppen substituiert sein kann, die aus der Gruppe von (i) einem C₁-C₆-Alkyl, (ii) einem C₂-C₇-Alkanoyl, (iii) einem C₁-C₆-Alkoxy-carbonyl und (iv) einem C₁-C₆-Alkylsulfonyl ausgewählt sind,
(f) Carboxy,
(g) einem C₂-C₇-Alkanoyl-oxy,
(h) einem C₁-C₆-Alkoxy-carbonyl und
(i) einem Carbamoyl, das mit 1 oder 2 Gruppen substituiert sein kann, die aus der Gruppe von (i) einem C₁-C₆-Alkyl, (ii) einem C₃-C₈-Cycloalkyl und (iii) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind, steht,
oder ein pharmazeutisch akzeptables Salz hiervon.

9. Verbindung nach Anspruch 8, wobei p 0 ist, oder ein pharmazeutisch akzeptables Salz hiervon.

10. Verbindung nach Anspruch 8 oder 9, wobei R¹⁰¹ für ein Halogenatom, ein C₁-C₄-Alkyl oder ein C₁-C₄-Alkoxy steht, oder ein pharmazeutisch akzeptables Salz hiervon.

11. Verbindung nach Anspruch 8 oder 9, wobei R¹⁰² für
(1) eine 5- oder 6-gliedrige monocyclische heterocyclische Gruppe steht, die 1 bis 3 Heteroatome enthält, die aus einem Stickstoffatom, einem Sauerstoffatom und einem Schwefelatom ausgewählt sind, oder
(2) ein C₁-C₆-Alkyl, das optional mit 1 bis 3 Substituenten substituiert ist, die gleich oder voneinander verschieden sein können und aus der Gruppe von Cyano, einem C₁-C₆-Alkoxy, Hydroxy, Amino, Carboxy, einem Carbamoyl, das optional mit einem C₁-C₆-Alkyl substituiert ist, und Phenyl ausgewählt sind, steht, oder ein pharmazeutisch akzeptables Salz hiervon.

12. Verbindung nach einem der Ansprüche 8 bis 11, wobei R¹⁰³ für
(1) ein C₁-C₆-Alkyl, das optional mit 1 bis 3 Substituenten substituiert ist, die gleich oder voneinander verschieden sein können und aus (a) Hydroxy, (b) einem C₁-C₆-Alkoxy, (c) Amino, (d) einem C₁-C₆-Alkoxy-carbonyl, (e) einem C₃-C₈-Cycloalkyl, (f) Phenyl und (g) einer heterocyclischen Gruppe, wobei diese eine ungesättigte oder eine partiell oder vollständig gesättigte monocyclische, bicyclische oder tricyclische heterocyclische Gruppe ist, die 1 bis 3 Heteroatome enthält, die aus einem Stickstoffatom, einem Sauerstoffatom und einem Schwefelatom ausgewählt sind, ausgewählt sind, oder
(2) ein C₃-C₈-Cycloalkyl, das optional mit 1 bis 3 Substituenten substituiert ist, die gleich oder voneinander verschieden sein können und aus der Gruppe von einem Halogen, einem C₁-C₆-Alkyl, Hydroxy, einem C₁-C₆-Alkoxy, Amino, Carboxy, einem C₂-C₇-Alkanoyl-oxy, einem C₁-C₆-Alkoxy-carbonyl und Carbamoyl ausgewählt sind, steht,
oder ein pharmazeutisch akzeptables Salz hiervon.

13. Medikament, das eine Verbindung der Formel [Ia] oder [Ic] gemäß den Angaben in einem der Ansprüche 1 bis 12 oder ein pharmazeutisch akzeptables Salz derselben umfasst.

14. Verbindung der Formel [Ia] oder [Ic] gemäß den Angaben in einem der Ansprüche 1 bis 12 oder ein pharmazeutisch akzeptables Salz derselben zur Verwendung bei einem Verfahren zur Behandlung des Körpers von Menschen oder Tieren durch eine Therapie.

15. Verbindung der Formel [Ia] oder [Ic] gemäß den Angaben in einem der Ansprüche 1 bis 12 oder ein pharmazeutisch akzeptables Salz derselben zur Verwendung bei der Prophylaxe oder Behandlung einer entzündlichen Erkrankung.

16. Verbindung nach Anspruch 15 zur Verwendung gemäß den Angaben in Anspruch 15, wobei es sich bei der entzündlichen Erkrankung um Arthritis handelt.

17. Verbindung der Formel: 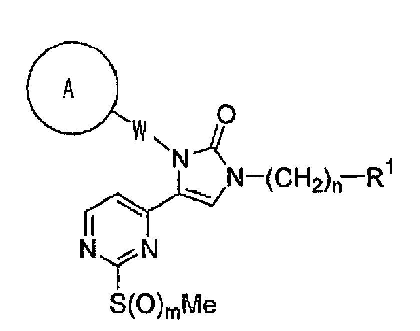 worin m für 1 oder 2 steht und die anderen Symbole die in Anspruch 1 angegebenen Bedeutungen aufweisen.

## Revendications

1. Composé de formule [Ia] : 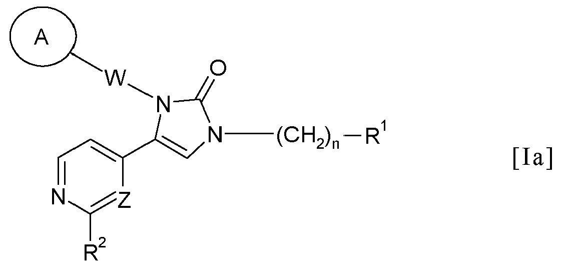 dans laquelle
• le cycle A est un cycle de type benzène ou thiophène, lequel cycle de type benzène ou thiophène peut porter de 1 à 3 substituant(s), identiques ou différents et choisi(s) dans l'ensemble constitué par les suivants :
- les atomes d'halogène,
- le groupe nitro,
- les groupes alkyle en C₁₋₆, éventuellement porteurs de 1 à 3 substituant(s) choisi(s) parmi les atomes d'halogène et les groupes hydroxyle et amino,
- les groupes alcoxy en C₁₋₆, éventuellement porteurs de 1 à 3 substituant(s) choisi(s) parmi les groupes hydroxyle et amino,
- le groupe amino, éventuellement porteur de 1 ou 2 substituant(s) choisi(s) parmi
a) les groupes alkyle en C₁₋₆, éventuellement porteurs de 1 à 3 substituant(s) choisi(s) parmi les groupes alcoxy en C₁₋₆, amino et carboxyle,
b) et les groupes alcanoyle en C₂₋₇,
- le groupe carbamyle, éventuellement porteur de 1 ou 2 substituant(s) alkyle en C₁₋₆,
- et le groupe cyano ;
• W représente une liaison simple, ou un groupe alcanediyle en C₁₋₄, éventuellement porteur de 1 ou 2 substituant(s) alkyle en C₁₋₆ ;
• l'indice n vaut 0, 1, 2, 3 ou 4 ;
• R¹ représente :
- un atome d'hydrogène,
- un groupe alkyle en C₁₋₆, éventuellement porteur de 1 à 3 substituant(s) choisi(s) parmi
a) les groupes alcynyle en C₂₋₇,
b) le groupe cyano,
c) les groupes alcoxy en C₁₋₆,
d) le groupe hydroxyle,
e) le groupe amino, éventuellement porteur de 1 ou 2 substituant(s) choisi(s) parmi les groupes alkyle en C₁₋₆, alcanoyle en C₂₋₇ et (alkyle en C₁₋₆)-sulfonyle,
f) le groupe carboxyle,
g) les groupes (alcoxy en C₁₋₆)-carbonyle,
h) le groupe carbamyle, éventuellement porteur de 1 ou 2 substituant(s) alkyle en C₁₋₆,
i) le groupe phényle,
j) et le groupe naphtyle,
- un groupe cycloalkyle en C₃₋₈, éventuellement porteur de 1 à 3 substituant(s) choisi(s) parmi
a) le groupe hydroxyle,
b) les groupes alcoxy en C₁₋₆, éventuellement porteurs de 1 à 3 substituant(s) alcoxy en C₁₋₆,
c) le groupe amino, éventuellement porteur de 1 ou 2 substituant(s) choisi(s) parmi les substituants (1) à (5) suivants :
1) les groupes alkyle en C₁₋₆,
2) les groupes alcanoyle en C₂₋₇,
3) les groupes (alcoxy en C₁₋₆)-carbonyle,
4) le groupe carbamyle, éventuellement porteur de 1 ou 2 substituant(s) alkyle en C₁₋₆,
5) et les groupes (alkyle en C₁₋₆)-sulfonyle,
d) le groupe carboxyle,
e) les groupes alkyle en C₁₋₆, éventuellement porteurs d'un substituant choisi parmi les groupes hydroxyle, alcoxy en C₁₋₆ et amino,
f) et le groupe carbamyle, éventuellement porteur d'un ou de substituant(s) alkyle en C₁-₆,
- un groupe phényle, éventuellement porteur de 1 à 3 substituant(s) choisi(s) parmi
a) les atomes d'halogène,
b) le groupe nitro,
c) les groupes alkyle en C₁₋₆, éventuellement porteurs de 1 à 3 substituant(s) choisi(s) parmi les atomes d'halogène et les groupes hydroxyle, amino, carboxyle et phénylsulfonyle,
d) les groupes alcényle en C₂₋₇,
e) le groupe cyano,
f) le groupe hydroxyle,
g) les groupes alcoxy en C₁₋₆, éventuellement porteurs de 1 à 3 substituant(s) choisi(s) parmi les atomes d'halogène et les groupes carboxyle, (alcoxy en C₁₋₆)-carbonyle, carbamyle, phényle et morpholinyl-carbonyle,
h) le groupe amino, éventuellement porteur de 1 ou 2 substituant(s) choisi(s) parmi les substituants (1) à (4) suivants :
1) les groupes alkyle en C₁₋₆,
2) les groupes alcanoyle en C₂₋₇,
3) le groupe carbamyle, éventuellement porteur de 1 ou 2 substituant(s) choisi(s) parmi les groupes alkyle en C₁₋₆ et cycloalkyle en C₃₋₈,
4) et les groupes (alkyle en C₁₋₆)-sulfonyle,
i) les groupes alcanoyle en C₂₋₇,
j) le groupe carboxyle,
k) les groupes (alcoxy en C₁₋₆)-carbonyle,
l) le groupe carbamyle, éventuellement porteur de 1 ou 2 substituant(s) choisi(s) parmi les substituants (1) et (2) suivants :
1) les groupes alkyle en C₁₋₆, éventuellement porteurs de 1 à 3 substituant(s) hydroxy,
2) et les groupes cycloalkyle en C₃₋₈,
m) les groupes alkylthio en C₁₋₆,
n) les groupes (alkyle en C₁₋₆)-sulfinyle,
o) les groupes (alkyle en C₁₋₆)-sulfonyle,
p) le groupe phényle,
q) le groupe tétrazolyle,
r) et un groupe carbonyle portant comme substituant un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi les groupes alkyle en C₁₋₆ et les groupes (alcoxy en C₁₋₆)-carbonyle,
- ou un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi
a) les atomes d'halogène,
b) le groupe nitro,
c) les groupes alkyle en C₁₋₆, éventuellement porteurs d'un substituant choisi parmi les groupes hydroxyle, alcoxy en C₁₋₆ et carbamyle éventuellement porteur d'un ou de substituant(s) alkyle en C₁₋₆ ou carboxy,
d) le groupe cyano,
e) le groupe hydroxyle,
f) le groupe amino,
g) les groupes alcanoyle en C₂₋₇,
h) le groupe carboxyle,
i) les groupes (alcoxy en C₁₋₆)-carbonyle,
j) le groupe carbamyle, éventuellement porteur de 1 ou 2 substituant(s) alkyle en C₁₋₆,
k) les groupes (alkyle en C₁₋₆)-sulfonyle,
l) et le groupe phényle ;
• Z représente un chaînon symbolisé par CH ou N ;
• et R² représente un atome d'hydrogène ou un groupe symbolisé par
- NR³R⁴, -OR⁵, -COR⁶ ou -CHR⁷R⁸, étant entendu que les symboles R³ à R⁸ représentent chacun, indépendamment,
- un atome d'hydrogène,
- un groupe alkyle en C₁₋₆, éventuellement porteur de 1 à 3 substituant(s) choisi(s) parmi
a) le groupe hydroxyle,
b) les groupes alcoxy en C₁₋₆,
c) le groupe amino, éventuellement porteur de 1 ou 2 substituant(s) choisi(s) parmi les groupes alkyle en C₁₋₆, alcanoyle en C₂₋₇ et (alkyle en C₁₋₆)-sulfonyle,
d) les groupes (alcoxy en C₁₋₆)-carbonyle,
e) les groupes cycloalkyle en C₃₋₈, éventuellement porteurs de 1 à 3 substituant(s) choisi(s) parmi
1) le groupe hydroxyle,
2) le groupe amino, éventuellement porteur d'un ou de substituant(s) alkyle en C₁₋₆,
3) les groupes (alcanoyle en C₂₋₇)-amino,
4) les groupes (alkyle en C₁₋₆)-sulfonylamino,
5) les groupes alkyle en C₁₋₆, éventuellement porteurs d'un substituant choisi parmi les groupes hydroxyle, alcoxy en C₁₋₆, amino et carbamyle éventuellement porteur d'un ou de substituant(s) alkyle en C₁₋₆,
6) le groupe carboxyle,
7) et le groupe carbamyle, éventuellement porteur d'un ou de substituant(s) alkyle en C₁₋₆,
f) le groupe phényle, éventuellement porteur de 1 à 3 substituant(s) choisi(s) parmi les substituants (1) à (6) suivants :
1) les atomes d'halogène,
2) les groupes alcoxy en C₁₋₆,
3) le groupe amino, éventuellement porteur de 1 ou 2 substituant(s) choisi(s) parmi les groupes alkyle en C₁₋₆ et (alcoxy en C₁₋₆)-carbonyle,
4) les groupes (alcoxy en C₁₋₆)-carbonyle,
5) le groupe carbamyle,
6) et le groupe morpholinyl-carbonyle,
g) et un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi les substituants (1) à (5) suivants :
1) les groupes alkyle en C₁₋₆ éventuellement porteurs de 1 à 3 substituant(s) hydroxy,
2) le groupe hydroxyle,
3) le groupe amino,
4) les groupes (alcoxy en C₁₋₆)-carbonyle,
5) et le groupe carbamyle,
- un groupe alcényle en C₂₋₇,
- un groupe alcynyle en C₂₋₇,
- un groupe hydroxyle,
- un groupe alcoxy en C₁₋₆,
- un groupe amino, éventuellement porteur de 1 ou 2 substituant(s) choisi(s) parmi
a) les groupes alkyle en C₁₋₆, éventuellement porteurs de 1 à 3 substituant(s) choisi(s) parmi
1) le groupe hydroxyle,
2) les groupes alcoxy en C₁₋₆,
3) et les groupes hétérocycliques qui sont des groupes hétérocycliques monocycliques, bicycliques ou tricycliques, insaturés ou partiellement ou totalement saturés, et comportant 1 à 3 hétéroatome(s) choisi(s) parmi les atomes d'azote, d'oxygène et de soufre,
b) les groupes cycloalkyle en C₃₋₈, éventuellement porteurs de 1 à 3 substituant(s) hydroxy,
c) et les groupes hétérocycliques qui sont des groupes hétérocycliques monocycliques, bicycliques ou tricycliques, insaturés ou partiellement ou totalement saturés, et comportant 1 à 3 hétéroatome(s) choisi(s) parmi les atomes d'azote, d'oxygène et de soufre,
- un groupe alcanoyle en C₂₋₇, éventuellement porteur de 1 à 3 substituant(s) choisi(s) parmi
a) le groupe hydroxy,
b) les groupes alcoxy en C₁₋₆,
c) le groupe amino, éventuellement porteur de 1 ou 2 substituant(s) choisi(s) parmi les groupes alkyle en C₁₋₆ et les groupes alcanoyle en C₂₋₇,
d) et les groupes (alcoxy en C₁₋₆)-carbonyle,
- un groupe carbamyle, éventuellement porteur de 1 ou 2 substituant(s) choisi(s) parmi
a) les groupes alkyle en C₁₋₆,
b) les groupes cycloalkyle en C₃₋₈,
c) et les groupes hétérocycliques qui sont des groupes hétérocycliques monocycliques, bicycliques ou tricycliques, insaturés ou partiellement ou totalement saturés, et comportant 1 à 3 hétéroatome(s) choisi(s) parmi les atomes d'azote, d'oxygène et de soufre,
- un groupe (alcoxy en C₁₋₆)-oxalyle,
- un groupe (alkyle en C₁₋₆)-sulfonyle,
- un groupe cycloalkyle en C₃₋₈, éventuellement porteur de 1 à 3 substituant(s) choisi(s) parmi
a) les atomes d'halogène,
b) les groupes alkyle en C₁₋₆, éventuellement porteurs de 1 à 3 substituant(s) choisi(s) parmi
1) le groupe hydroxyle,
2) les groupes alcoxy en C₁₋₆,
3) le groupe amino,
4) et le groupe carbamyle, éventuellement porteur d'un substituant alkyle en C₁₋₆,
c) le groupe hydroxyle,
d) les groupes alcoxy en C₁₋₆,
e) le groupe amino, éventuellement porteur de 1 ou 2 substituant(s) choisi(s) parmi
1) les groupes alkyle en C₁₋₆,
2) les groupes alcanoyle en C₂₋₇,
3) les groupes (alcoxy en C₁₋₆)-carbonyle,
4) et les groupes (alkyle en C₁₋₆)-sulfonyle,
f) le groupe carboxyle,
g) les groupes (alcanoyle en C₂₋₇)-oxy,
h) les groupes (alcoxy en C₁₋₆)-carbonyle,
i) et le groupe carbamyle, éventuellement porteur de 1 ou 2 substituant(s) choisi(s) parmi
1) les groupes alkyle en C₁₋₆,
2) les groupes cycloalkyle en C₃₋₈,
3) et les groupes hétérocycliques qui sont des groupes hétérocycliques monocycliques, bicycliques ou tricycliques, insaturés ou partiellement ou totalement saturés, et comportant 1 à 3 hétéroatome(s) choisi(s) parmi les atomes d'azote, d'oxygène et de soufre,
- un groupe phényle, éventuellement porteur de 1 à 3 substituant(s) choisi(s) parmi
a) les groupes alkyle en C₁₋₆,
b) le groupe hydroxyle,
c) les groupes alcoxy en C₁₋₆,
d) les atomes d'halogène,
e) et le groupe amino, éventuellement porteur de 1 ou 2 substituant(s) alkyle en C₁₋₆,
- un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi
a) les groupes alkyle en C₁₋₆, éventuellement porteurs de 1 à 3 substituant(s) choisi(s) parmi
1) le groupe phényle,
2) le groupe hydroxyle,
3) les groupes alcoxy en C₁₋₆,
4) le groupe amino,
5) et le groupe carbamyle, éventuellement porteur d'un substituant alkyle en C₁₋₆,
b) le groupe carboxyle,
c) les groupes (alcoxy en C₁₋₆)-carbonyle,
d) les groupes alcanoyle en C₂₋₇,
e) les groupes (alkyle en C₁₋₆)-sulfonyle,
f) et le substituant oxo,
- un groupe carbonyle porteur d'un substituant cycloalkyle en C₃₋₈ éventuellement porteur de 1 à 3 substituant(s) choisi(s) parmi
a) le groupe hydroxyle,
b) les groupes alcoxy en C₁₋₆,
c) le groupe amino, éventuellement porteur de 1 ou 2 substituant(s) choisi(s) parmi
1) les groupes alkyle en C₁₋₆,
2) et les groupes alcanoyle en C₂₋₇,
d) et les groupes (alcoxy en C₁₋₆)-carbonyle,
- un groupe carbonyle porteur d'un substituant phényle éventuellement porteur de 1 à 3 substituant(s) choisi(s) parmi
a) les atomes d'halogène,
b) le groupe hydroxyle,
c) les groupes alcoxy en C₁₋₆,
d) et le groupe amino, éventuellement porteur de 1 ou 2 substituant(s) choisi(s) parmi
1) les groupes alkyle en C₁₋₆,
2) et les groupes alcanoyle en C₂₋₇,
- ou un groupe carbonyle porteur d'un substituant hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et qui porte éventuellement 1 à 3 substituant(s) choisi(s) parmi
a) les atomes d'halogène,
b) les groupes alkyle en C₁₋₆,
c) le groupe hydroxyle,
d) le groupe amino, éventuellement porteur de 1 ou 2 substituant(s) alkyle en C₁₋₆,
e) les groupes alcanoyle en C₂₋₇,
f) et le substituant oxo ;
ou sel pharmacologiquement admissible d'un tel composé.

2. Composé conforme à la revendication 1, dans lequel le cycle A est un cycle de type benzène qui peut porter de 1 à 3 substituant(s), identiques ou différents et choisi(s) dans l'ensemble constitué par les suivants :
- les atomes d'halogène,
- le groupe nitro,
- les groupes alkyle en C₁₋₆, éventuellement porteurs de 1 à 3 substituant(s) choisi(s) parmi les atomes d'halogène et les groupes hydroxyle et amino,
- les groupes alcoxy en C₁₋₆, éventuellement porteurs de 1 à 3 substituant(s) choisi(s) parmi les groupes hydroxyle et amino,
- le groupe amino, éventuellement porteur de 1 ou 2 substituant(s) choisi(s) parmi
a) les groupes alkyle en C₁₋₆, éventuellement porteurs de 1 à 3 substituant(s) choisi(s) parmi les groupes alcoxy en C₁₋₆, amino et carboxyle,
b) et les groupes alcanoyle en C₂₋₇,
- et le groupe cyano,
et W représente une liaison simple,
ou sel pharmacologiquement admissible d'un tel composé.

3. Composé conforme à la revendication 1 ou 2, dans lequel l'indice n vaut 0 ou 1, ou sel pharmacologiquement admissible d'un tel composé.

4. Composé conforme à l'une des revendications 1 à 3, dans lequel
• l'indice n vaut 0 et R¹ représente un groupe alkyle en C₁₋₆, éventuellement porteur de 1 à 3 substituant(s) choisi(s) parmi
a) les groupes alcynyle en C₂₋₇,
b) le groupe cyano,
c) les groupes alcoxy en C₁₋₆,
d) le groupe hydroxyle,
e) le groupe amino, éventuellement porteur de 1 ou 2 substituant(s) choisi(s) parmi les groupes alkyle en C₁₋₆, alcanoyle en C₂₋₇ et (alkyle en C₁₋₆)-sulfonyle,
f) le groupe carboxyle,
g) les groupes (alcoxy en C₁₋₆)-carbonyle,
h) le groupe carbamyle, éventuellement porteur de 1 ou 2 substituant(s) alkyle en C₁₋₆,
i) le groupe phényle,
j) et le groupe naphtyle ;
• ou l'indice n vaut 1 et R¹ représente :
A) un groupe cycloalkyle en C₃₋₈, éventuellement porteur de 1 à 3 substituant(s) choisi(s) parmi
a) le groupe hydroxyle,
b) les groupes alcoxy en C₁₋₆, éventuellement porteurs de 1 à 3 substituant(s) alcoxy en C₁₋₆,
c) le groupe amino, éventuellement porteur de 1 ou 2 substituant(s) choisi(s) parmi les substituants (1) à (5) suivants :
1) les groupes alkyle en C₁₋₆,
2) les groupes alcanoyle en C₂₋₇,
3) les groupes (alcoxy en C₁₋₆)-carbonyle,
4) le groupe carbamyle, éventuellement porteur de 1 ou 2 substituant(s) alkyle en C₁₋₆,
5) et les groupes (alkyle en C₁₋₆)-sulfonyle,
d) le groupe carboxyle,
e) les groupes alkyle en C₁₋₆, éventuellement porteurs d'un substituant choisi parmi les groupes hydroxyle, alcoxy en C₁₋₆ et amino,
f) et le groupe carbamyle, éventuellement porteur d'un ou de substituant(s) alkyle en C₁₋₆,
B) ou un groupe phényle, éventuellement porteur de 1 à 3 substituant(s) choisi(s) parmi
a) les atomes d'halogène,
b) le groupe nitro,
c) les groupes alkyle en C₁₋₆, éventuellement porteurs de 1 à 3 substituant(s) choisi(s) parmi les atomes d'halogène et les groupes hydroxyle, amino, carboxyle et phénylsulfonyle,
d) les groupes alcényle en C₂₋₇,
e) le groupe cyano,
f) le groupe hydroxyle,
g) les groupes alcoxy en C₁₋₆, éventuellement porteurs de 1 à 3 substituant(s) choisi(s) parmi les atomes d'halogène et les groupes carboxyle, (alcoxy en C₁₋₆)-carbonyle, carbamyle, phényle et morpholinyl-carbonyle,
h) le groupe amino, éventuellement porteur de 1 ou 2 substituant(s) choisi(s) parmi les substituants (1) à (4) suivants :
1) les groupes alkyle en C₁₋₆,
2) les groupes alcanoyle en C₂₋₇,
3) le groupe carbamyle, éventuellement porteur de 1 ou 2 substituant(s) choisi(s) parmi les groupes alkyle en C₁₋₆ et cycloalkyle en C₃₋₈,
4) et les groupes (alkyle en C₁₋₆)-sulfonyle,
i) les groupes alcanoyle en C₂₋₇,
j) le groupe carboxyle,
k) les groupes (alcoxy en C₁₋₆)-carbonyle,
l) le groupe carbamyle, éventuellement porteur de 1 ou 2 substituant(s) choisi(s) parmi les substituants (1) et (2) suivants :
1) les groupes alkyle en C₁₋₆, éventuellement porteurs de 1 à 3 substituant(s) hydroxy,
2) et les groupes cycloalkyle en C₃₋₈,
m) les groupes alkylthio en C₁₋₆,
n) les groupes (alkyle en C₁₋₆)-sulfinyle,
o) les groupes (alkyle en C₁₋₆)-sulfonyle,
p) le groupe phényle,
q) le groupe tétrazolyle,
r) et un groupe carbonyle portant comme substituant un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi les groupes alkyle en C₁₋₆ et les groupes (alcoxy en C₁₋₆)-carbonyle ;
ou sel pharmacologiquement admissible d'un tel composé.

5. Composé conforme à l'une des revendications 1 à 4, dans lequel R² représente un groupe symbolisé par -NR³R⁴ ou -OR⁵, ou sel pharmacologiquement admissible d'un tel composé.

6. Composé conforme à l'une des revendications 1 à 4, dans lequel R² représente un groupe symbolisé par -NHR⁴ et R⁴ représente
- un groupe alkyle en C₁₋₆, éventuellement porteur de 1 à 3 substituant(s) choisi(s) parmi
a) le groupe hydroxyle,
b) les groupes alcoxy en C₁₋₆,
c) le groupe amino, éventuellement porteur de 1 ou 2 substituant(s) choisi(s) parmi les groupes alkyle en C₁₋₆, alcanoyle en C₂₋₇ et (alkyle en C₁₋₆)-sulfonyle
d) les groupes (alcoxy en C₁₋₆)-carbonyle,
e) les groupes cycloalkyle en C₃₋₈, éventuellement porteurs de 1 à 3 substituant(s) choisi(s) parmi
1) le groupe hydroxyle,
2) le groupe amino, éventuellement porteur de substituant(s) alkyle en C₁₋₆,
3) les groupes (alcanoyle en C₂₋₇)-amino,
4) les groupes (alkyle en C₁₋₆)-sulfonylamino,
5) les groupes alkyle en C₁₋₆, éventuellement porteurs d'un substituant choisi parmi les groupes hydroxyle, alcoxy en C₁₋₆, amino et carbamyle éventuellement porteur d'un ou de substituant(s) alkyle en C₁₋₆,
6) le groupe carboxyle,
7) et le groupe carbamyle, éventuellement porteur d'un ou de substituant(s) alkyle en C₁₋₆,
f) le groupe phényle, éventuellement porteur de 1 à 3 substituant(s) choisi(s) parmi les substituants (1) à (6) suivants :
1) les atomes d'halogène,
2) les groupes alcoxy en C₁₋₆,
3) le groupe amino, éventuellement porteur de 1 ou 2 substituant(s) choisi(s) parmi les groupes alkyle en C₁₋₆ et (alcoxy en C₁₋₆)-carbonyle,
4) les groupes (alcoxy en C₁₋₆)-carbonyle,
5) le groupe carbamyle,
6) et le groupe morpholinyl-carbonyle,
g) et un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi les substituants (1) à (5) suivants :
1) les groupes alkyle en C₁₋₆ éventuellement porteurs de 1 à 3 substituant(s) hydroxy,
2) le groupe hydroxyle,
3) le groupe amino,
4) les groupes (alcoxy en C₁₋₆)-carbonyle,
5) et le groupe carbamyle,
- un groupe alcényle en C₂₋₇,
- un groupe alcanoyle en C₂₋₇, éventuellement porteur de 1 à 3 substituant(s) choisi(s) parmi
a) le groupe hydroxy,
b) les groupes alcoxy en C₁₋₆,
c) le groupe amino, éventuellement porteur de 1 ou 2 substituant(s) choisi(s) parmi les groupes alkyle en C₁₋₆ et alcanoyle en C₂₋₇,
d) et les groupes (alcoxy en C₁₋₆)-carbonyle,
- un groupe carbamyle, éventuellement porteur de 1 ou 2 substituant(s) choisi(s) parmi
a) les groupes alkyle en C₁₋₆,
b) les groupes cycloalkyle en C₃₋₈,
c) et les groupes hétérocycliques qui sont des groupes hétérocycliques monocycliques, bicycliques ou tricycliques, insaturés ou partiellement ou totalement saturés, et comportant 1 à 3 hétéroatome(s) choisi(s) parmi les atomes d'azote, d'oxygène et de soufre,
- un groupe cycloalkyle en C₃₋₈, éventuellement porteur de 1 à 3 substituant(s) choisi(s) parmi
a) les atomes d'halogène,
b) les groupes alkyle en C₁₋₆, éventuellement porteurs de 1 à 3 substituant(s) choisi(s) parmi
1) le groupe hydroxyle,
2) les groupes alcoxy en C₁₋₆,
3) le groupe amino,
4) et le groupe carbamyle, éventuellement porteur d'un substituant alkyle en C₁₋₆,
c) le groupe hydroxyle,
d) les groupes alcoxy en C₁₋₆,
e) le groupe amino, éventuellement porteur de 1 ou 2 substituant(s) choisi(s) parmi
1) les groupes alkyle en C₁₋₆,
2) les groupes alcanoyle en C₂₋₇,
3) les groupes (alcoxy en C₁₋₆)-carbonyle,
4) et les groupes (alkyle en C₁₋₆)-sulfonyle,
f) le groupe carboxyle,
g) les groupes (alcanoyle en C₂₋₇)-oxy,
h) les groupes (alcoxy en C₁₋₆)-carbonyle,
i) et le groupe carbamyle, éventuellement porteur de 1 ou 2 substituant(s) choisi(s) parmi
1) les groupes alkyle en C₁₋₆,
2) les groupes cycloalkyle en C₃₋₈,
3) et les groupes hétérocycliques qui sont des groupes hétérocycliques monocycliques, bicycliques ou tricycliques, insaturés ou partiellement ou totalement saturés, et comportant 1 à 3 hétéroatome(s) choisi(s) parmi les atomes d'azote, d'oxygène et de soufre,
- un groupe phényle, éventuellement porteur de 1 à 3 substituant(s) choisi(s) parmi
a) les groupes alkyle en C₁₋₆,
b) le groupe hydroxyle,
c) les groupes alcoxy en C₁₋₆,
d) les atomes d'halogène,
e) et le groupe amino, éventuellement porteur de 1 ou 2 substituant(s) alkyle en C₁₋₆,
- un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi
a) les groupes alkyle en C₁₋₆, éventuellement porteurs de 1 à 3 substituant(s) choisi(s) parmi
1) le groupe phényle,
2) le groupe hydroxyle,
3) les groupes alcoxy en C₁₋₆,
4) le groupe amino,
5) et le groupe carbamyle, éventuellement porteur d'un substituant alkyle en C₁₋₆,
b) le groupe carboxyle,
c) les groupes (alcoxy en C₁₋₆)-carbonyle,
d) les groupes alcanoyle en C₂₋₇,
e) les groupes (alkyle en C₁₋₆)-sulfonyle,
f) et le substituant oxo,
- un groupe carbonyle porteur d'un substituant cycloalkyle en C₃₋₈ éventuellement porteur de 1 à 3 substituant(s) choisi(s) parmi
a) le groupe hydroxyle,
b) les groupes alcoxy en C₁₋₆,
c) le groupe amino, éventuellement porteur de 1 ou 2 substituant(s) choisi(s) parmi
1) les groupes alkyle en C₁₋₆,
2) et les groupes alcanoyle en C₂₋₇,
d) et les groupes (alcoxy en C₁₋₆)-carbonyle,
- ou un groupe carbonyle porteur d'un substituant hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi
a) les atomes d'halogène,
b) les groupes alkyle en C₁₋₆,
c) le groupe hydroxyle,
d) le groupe amino, éventuellement porteur de 1 ou 2 substituant(s) alkyle en C₁₋₆,
e) les groupes alcanoyle en C₂₋₇,
f) et le substituant oxo ;
ou sel pharmacologiquement admissible d'un tel composé.

7. Composé conforme à la revendication 1, dans lequel
- le cycle A est un cycle benzénique, qui peut porter 1 ou 2 substituant(s), identiques ou différents et choisi(s) parmi les atomes d'halogène et les groupes alkyle en C₁₋₆, alcoxy en C₁₋₆, cyano et amino éventuellement porteur d'un ou de substituant(s) alkyle en C₁₋₆ ;
- W représente une liaison simple ;
- l'indice n vaut 0 ou 1 ;
- R¹ représente :
1) un atome d'hydrogène,
2) un groupe alkyle en C₁₋₆, en option porteur d'un ou de substituant(s) choisi(s) parmi les groupes phényle, alcoxy en C₁₋₆, (alkyle en C₁₋₆)-amino, di(alkyle en C₁₋₆)-amino, (alcanoyle en C₂₋₇)-amino, (alkyle en C₁₋₆)-sulfonyl-amino, carbamyle éventuellement porteur d'un ou de substituant(s) alkyle en C₁₋₆, hydroxyle, carboxyle et cyano,
3) un groupe cycloalkyle en C₃₋₈, en option porteur d'un ou de substituant(s) choisi(s) parmi les substituants (i) à (v) suivants :
i) le groupe hydroxyle,
ii) les groupes alcoxy en C₁₋₆, en option porteurs d'un ou de substituant(s) alcoxy en C₁₋₆,
iii) le groupe amino, en option porteur d'un ou de substituant(s) choisi(s) parmi les groupes alkyle en C₁₋₆, alcanoyle en C₂₋₇ et (alkyle en C₁₋₆)-sulfonyle,
iv) le groupe carbamyle, en option porteur d'un ou de substituant(s) alkyle en C₁₋₆,
v) et les groupes alkyle en C₁₋₆, en option porteurs d'un ou de substituant(s) choisi(s) parmi les groupes hydroxyle, alcoxy en C₁₋₆ et amino,
4) un groupe phényle, en option porteur d'un ou de substituant(s) choisi(s) parmi les substituants (i) à (vi) suivants :
i) les atomes d'halogène,
ii) les groupes alkyle en C₁₋₆, en option porteurs d'un ou de substituant(s) choisi(s) parmi les atomes d'halogène et les groupes hydroxyle et phénylsulfonyle,
iii) le groupe cyano,
iv) les groupes alcoxy en C₁₋₆,
v) le groupe amino, en option porteur d'un ou de substituant(s) choisi(s) parmi les groupes alkyle en C₁₋₆ et (alkyle en C₁₋₆)-sulfonyle,
vi) et un groupe carbonyle portant comme substituant un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre,
5) ou un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur d'un ou de substituant(s) choisi(s) parmi les substituants (i) à (iv) suivants :
i) les groupes (alcoxy en C₁₋₆)-carbonyle,
ii) les groupes alkyle en C₁₋₆, en option porteurs d'un ou de substituant(s) choisi(s) parmi les groupes hydroxyle, alcoxy en C₁₋₆ et carbamyle porteur, en option, d'un ou de substituant(s) alkyle en C₁₋₆,
iii) les groupes alcanoyle en C₂₋₇,
iv) et les groupes (alkyle en C₁₋₆)-sulfonyle ;
- Z représente un chaînon symbolisé par CH ou N ;
- et R² représente un atome d'hydrogène ou un groupe symbolisé par -NR³R⁴, -OR⁵, -COR⁶ ou -CHR⁷R⁸, étant entendu que les symboles R³ à R⁸ représentent chacun, indépendamment,
1) un atome d'hydrogène,
2) un groupe alkyle en C₁₋₆, en option porteur d'un ou de substituant(s) choisi(s) parmi les substituants (i) à (vii) suivants :
i) le groupe hydroxyle,
ii) les groupes alcoxy en C₁₋₆,
iii) le groupe amino, en option porteur d'un ou de substituant(s) choisi(s) parmi les groupes alkyle en C₁₋₆, alcanoyle en C₂₋₇ et (alkyle en C₁₋₆)-sulfonyle
iv) les groupes (alcoxy en C₁₋₆)-carbonyle,
v) les groupes cycloalkyle en C₃₋₈, en option porteurs d'un ou de substituant(s) choisi(s) parmi les substituants (a) à (g) suivants :
a) le groupe hydroxyle,
b) le groupe amino, en option porteur d'un ou de substituant(s) alkyle en C₁₋₆,
c) les groupes (alcanoyle en C₂₋₇)-amino,
d) les groupes (alkyle en C₁₋₆)-sulfonylamino,
e) les groupes alkyle en C₁₋₆, en option porteurs d'un ou de substituant(s) choisi(s) parmi les groupes hydroxyle, alcoxy en C₁₋₆, amino et carbamyle porteur, en option, d'un ou de substituant(s) alkyle en C₁₋₆,
f) le groupe carboxyle,
g) et le groupe carbamyle, en option porteur d'un ou de substituant(s) alkyle en C₁₋₆,
vi) le groupe phényle, en option porteur d'un ou de substituant(s) choisi(s) parmi les atomes d'halogène, les groupes alcoxy en C₁₋₆ et le groupe morpholinyl-carbonyle,
vii) et un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur d'un ou de substituant(s) alkyle en C₁₋₆,
3) un groupe alcényle en C₂₋₇,
4) un groupe alcoxy en C₁₋₆,
5) un groupe alcanoyle en C₂₋₇, en option porteur d'un ou de substituant(s) choisi(s) parmi les substituants (i) à (iv) suivants :
i) le groupe hydroxyle,
ii) les groupes alcoxy en C₁₋₆,
iii) le groupe amino, en option porteur d'un ou de substituant(s) choisi(s) parmi les groupes alkyle en C₁₋₆ et alcanoyle en C₂₋₇,
iv) et les groupes (alcoxy en C₁₋₆)-carbonyle,
6) un groupe carbamyle, en option porteur d'un ou de substituant(s) alkyle en C₁₋₆,
7) un groupe (alcoxy en C₁₋₆)-oxalyle,
8) un groupe cycloalkyle en C₃₋₈, en option porteur d'un ou de substituant(s) choisi(s) parmi les substituants (i) à (vii) suivants :
i) les atomes d'halogène,
ii) le groupe hydroxyle,
iii) les groupes alcoxy en C₁₋₆,
iv) le groupe amino, en option porteur d'un ou de substituant(s) choisi(s) parmi les groupes alkyle en C₁₋₆, alcanoyle en C₂₋₇, (alcoxy en C₁₋₆)-carbonyle et (alkyle en C₁₋₆)-sulfonyle,
v) les groupes alkyle en C₁₋₆, en option porteurs d'un ou de substituant(s) choisi(s) parmi les groupes hydroxyle, alcoxy en C₁₋₆, amino et carbamyle porteur, en option, d'un ou de substituant(s) alkyle en C₁₋₆,
vi) les groupes (alcanoyle en C₂₋₇)-oxy,
vii) et le groupe carbamyle, en option porteur d'un ou de substituant(s) alkyle en C₁₋₆,
9) un groupe phényle, en option porteur d'un ou de substituant(s) choisi(s) parmi les atomes d'halogène et les groupes alcoxy en C₁₋₆,
10) un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi les substituants (i) à (v) suivants :
i) les groupes alkyle en C₁₋₆, en option porteurs d'un ou de substituant(s) choisi(s) parmi les groupes phényle, hydroxyle, alcoxy en C₁₋₆, amino et carbamyle porteur, en option, d'un ou de substituant(s) alkyle en C₁₋₆,
ii) les groupes (alcoxy en C₁₋₆)-carbonyle,
iii) les groupes alcanoyle en C₂₋₇,
iv) les groupes (alkyle en C₁₋₆)-sulfonyle,
v) et le substituant oxo,
11) un groupe carbonyle porteur d'un substituant cycloalkyle en C₃₋₈ en option porteur d'un ou de substituant(s) choisi(s) parmi les groupes hydroxyle, amino et (alcanoyle en C₂₋₇)-amino,
12) ou un groupe carbonyle porteur d'un substituant hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre ;
ou sel pharmacologiquement admissible d'un tel composé.

8. Composé conforme à la revendication 1 et de formule [Ic] : 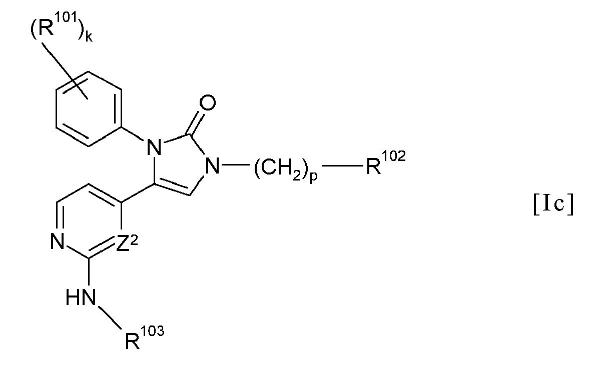 dans laquelle
- les symboles R¹⁰¹ représentent des entités identiques ou différentes, choisies chacune parmi les suivantes :
1) les atomes d'halogène,
2) le groupe nitro,
3) les groupes alkyle en C₁₋₆, éventuellement porteurs de 1 à 3 substituant(s) choisi(s) parmi les atomes d'halogène et les groupes hydroxyle et amino,
4) les groupes alcoxy en C₁₋₆, éventuellement porteurs de 1 à 3 substituant(s) choisi(s) parmi les groupes hydroxyle et amino,
5) le groupe amino, éventuellement porteur de 1 ou 2 substituant(s) choisi(s) parmi
a) les groupes alkyle en C₁₋₆, éventuellement porteurs de 1 à 3 substituant(s) choisi(s) parmi les groupes alcoxy en C₁₋₆, amino et carboxyle,
b) et les groupes alcanoyle en C₂₋₇,
6) et le groupe cyano ;
- l'indice k vaut de 0 à 3 ;
- l'indice p vaut 0 ou 1 ;
- R¹⁰² représente :
1) un groupe alkyle en C₁₋₆, éventuellement porteur de 1 à 3 substituant(s) choisi(s) parmi
a) les groupes alcynyle en C₂₋₇,
b) le groupe cyano,
c) les groupes alcoxy en C₁₋₆,
d) le groupe hydroxyle,
e) le groupe amino, éventuellement porteur de 1 ou 2 substituant(s) choisi(s) parmi les groupes alkyle en C₁₋₆, alcanoyle en C₂₋₇ et (alkyle en C₁₋₆)-sulfonyle,
f) le groupe carboxyle,
g) les groupes (alcoxy en C₁₋₆)-carbonyle,
h) le groupe carbamyle, éventuellement porteur de 1 ou 2 substituant(s) alkyle en C₁₋₆,
i) le groupe phényle,
j) et le groupe naphtyle,
2) ou un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi
a) les atomes d'halogène,
b) le groupe nitro,
c) les groupes alkyle en C₁₋₆, éventuellement porteurs d'un substituant choisi parmi les groupes hydroxyle, alcoxy en C₁₋₆ et carbamyle éventuellement porteur d'un ou de susbtituant(s) alkyle en C₁₋₆ ou carboxy,
d) le groupe cyano,
e) le groupe hydroxyle,
f) le groupe amino,
g) les groupes alcanoyle en C₂₋₇,
h) le groupe carboxyle,
i) les groupes (alcoxy en C₁₋₆)-carbonyle,
j) le groupe carbamyle, éventuellement porteur de 1 ou 2 substituant(s) alkyle en C₁₋₆,
k) les groupes (alkyle en C₁₋₆)-sulfonyle,
l) et le groupe phényle ;
- Z² représente un chaînon symbolisé par CH ou N ;
- et R¹⁰³ représente
1) un groupe alkyle en C₁₋₆, éventuellement porteur de 1 à 3 substituant(s) choisi(s) parmi
a) le groupe hydroxyle,
b) les groupes alcoxy en C₁₋₆,
c) le groupe amino, éventuellement porteur de 1 ou 2 substituant(s) choisi(s) parmi les groupes alkyle en C₁₋₆, alcanoyle en C₂₋₇ et (alkyle en C₁₋₆)-sulfonyle,
d) les groupes (alcoxy en C₁₋₆)-carbonyle,
e) les groupes cycloalkyle en C₃₋₈, éventuellement porteurs de 1 à 3 substituant(s) choisi(s) parmi
i) le groupe hydroxyle,
ii) le groupe amino, éventuellement porteur de substituant(s) alkyle en C₁₋₆,
iii) les groupes (alcanoyle en C₂₋₇)-amino,
iv) les groupes (alkyle en C₁₋₆)-sulfonylamino,
v) les groupes alkyle en C₁₋₆, éventuellement porteurs d'un substituant choisi parmi les groupes hydroxyle, alcoxy en C₁₋₆, amino et carbamyle éventuellement porteur d'un ou de substituant(s) alkyle en C₁₋₆,
vi) le groupe carboxyle,
vii) et le groupe carbamyle, éventuellement porteur d'un ou de substituant(s) alkyle en C₁₋₆,
f) le groupe phényle, éventuellement porteur de 1 à 3 substituant(s) choisi(s) parmi les substituants (i) à (vi) suivants :
i) les atomes d'halogène,
ii) les groupes alcoxy en C₁₋₆,
iii) le groupe amino, éventuellement porteur de 1 ou 2 substituant(s) choisi(s) parmi les groupes alkyle en C₁₋₆ et (alcoxy en C₁₋₆)-carbonyle,
iv) les groupes (alcoxy en C₁₋₆)-carbonyle,
v) le groupe carbamyle,
vi) et le groupe morpholinyl-carbonyle,
g) et un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre, et éventuellement porteur de 1 à 3 substituant(s) choisi(s) parmi les substituants (i) à (v) suivants :
i) les groupes alkyle en C₁₋₆ éventuellement porteurs de 1 à 3 substituant(s) hydroxy,
ii) le groupe hydroxyle,
iii) le groupe amino,
iv) les groupes (alcoxy en C₁₋₆)-carbonyle,
v) et le groupe carbamyle,
2) ou un groupe cycloalkyle en C₃₋₈, éventuellement porteur de 1 à 3 substituant(s) choisi(s) parmi
a) les atomes d'halogène,
b) les groupes alkyle en C₁₋₆, éventuellement porteurs de 1 à 3 substituant(s) choisi(s) parmi
i) le groupe hydroxyle,
ii) les groupes alcoxy en C₁₋₆,
iii) le groupe amino,
iv) et le groupe carbamyle, éventuellement porteur d'un substituant alkyle en C₁₋₆,
c) le groupe hydroxyle,
d) les groupes alcoxy en C₁₋₆,
e) le groupe amino, éventuellement porteur de 1 ou 2 substituant(s) choisi(s) parmi
i) les groupes alkyle en C₁₋₆,
ii) les groupes alcanoyle en C₂₋₇,
iii) les groupes (alcoxy en C₁₋₆)-carbonyle,
iv) et les groupes (alkyle en C₁₋₆)-sulfonyle,
f) le groupe carboxyle,
g) les groupes (alcanoyle en C₂₋₇)-oxy,
h) les groupes (alcoxy en C₁₋₆)-carbonyle,
i) et le groupe carbamyle, éventuellement porteur de 1 ou 2 substituant(s) choisi(s) parmi
i) les groupes alkyle en C₁₋₆,
ii) les groupes cycloalkyle en C₃₋₈,
iii) et les groupes hétérocycliques qui sont des groupes hétérocycliques monocycliques, bicycliques ou tricycliques, insaturés ou partiellement ou totalement saturés, et comportant 1 à 3 hétéroatome(s) choisi(s) parmi les atomes d'azote, d'oxygène et de soufre,
ou sel pharmacologiquement admissible d'un tel composé.

9. Composé conforme à la revendication 8, dans lequel l'indice p vaut 0, ou sel pharmacologiquement admissible d'un tel composé.

10. Composé conforme à la revendication 8 ou 9, dans lequel R¹⁰¹ représente un atome d'halogène ou un groupe alkyle en C₁₋₄ ou alcoxy en C₁₋₄, ou sel pharmacologiquement admissible d'un tel composé.

11. Composé conforme à la revendication 8 ou 9, dans lequel R¹⁰² représente
1) un groupe hétérocyclique monocyclique à 5 ou 6 chaînons, comportant 1 à 3 hétéroatome(s) choisi(s) parmi les atomes d'azote, d'oxygène et de soufre,
2) ou un groupe alkyle en C₁₋₆, en option porteur de 1 à 3 substituant(s) qui peuvent être identiques ou différents les uns des autres, choisi(s) parmi les groupes cyano, alcoxy en C₁₋₆, hydroxyle, amino, carboxyle, phényle et carbamyle porteur, en option, d'un substituant alkyle en C₁₋₆,
ou sel pharmacologiquement admissible d'un tel composé.

12. Composé conforme à la revendication 8 ou 9, dans lequel R¹⁰³ représente
1) un groupe alkyle en C₁₋₆, en option porteur de 1 à 3 substituant(s) qui peuvent être identiques ou différents les uns des autres, choisi(s) parmi
a) le groupe hydroxyle,
b) les groupes alcoxy en C₁₋₆,
c) le groupe amino,
d) les groupes (alcoxy en C₁₋₆)-carbonyle,
e) les groupes cycloalkyle en C₃₋₈,
f) le groupe phényle,
g) et un groupe hétérocyclique qui est un groupe hétérocyclique monocyclique, bicyclique ou tricyclique, insaturé ou partiellement ou totalement saturé, comportant 1 à 3 hétéroatome(s) choisi(s) parmi les atomes d'azote, d'oxygène et de soufre,
2) ou un groupe cycloalkyle en C₃₋₈, en option porteur de 1 à 3 substituant(s) qui peuvent être identiques ou différents les uns des autres, choisi(s) parmi les atomes d'halogène et les groupes alkyle en C₁₋₆, hydroxyle, alcoxy en C₁₋₆, amino, carboxyle, (alcanoyle en C₂₋₇)-oxy, (alcoxy en C₁₋₆)-carbonyle et carbamyle,
ou sel pharmacologiquement admissible d'un tel composé.

13. Médicament comprenant un composé de formule [Ia] ou [Ic], conforme à l'une des revendications 1 à 12, ou un sel pharmacologiquement admissible d'un tel composé.

14. Composé de formule [Ia] ou [Ic], conforme à l'une des revendications 1 à 12, ou sel pharmacologiquement admissible d'un tel composé, pour utilisation dans un procédé de traitement thérapeutique du corps d'un humain ou d'un animal.

15. Composé de formule [Ia] ou [Ic], conforme à l'une des revendications 1 à 12, ou sel pharmacologiquement admissible d'un tel composé, pour utilisation dans la prophylaxie ou le traitement d'une maladie inflammatoire

16. Composé conforme à la revendication 15, pour utilisation définie dans la revendication 15, la maladie inflammatoire étant une arthrite.

17. Composé de formule : 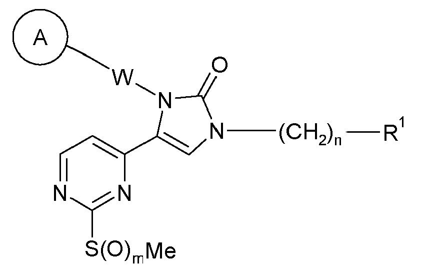 dans laquelle l'indice m vaut 1 ou 2, et les autres symboles ont les significations indiquées dans la revendication 1.
